# EUROPEAN PATENT APPLICATION

(11) **EP 1 661 572 A1**
(43) Date of publication of application: **31.05.2006**
(21) Application number: 04771706.1
(22) Date of filing: 16.08.2004
(51) Int. Cl.: A61K 31/5415, A61K 31/435, A61P 11/04, A61P 11/08, A61P 29/00, A61P 43/00

(54) **NOVEL USE OF CANNABINOID RECEPTOR AGONIST**

(30) Priority: 18.08.2003 JP 2003294114
(71) Applicant: SHIONOGI & CO., LTD., Osaka-shi, Osaka 541-0045 (JP)
(72) Inventor: ARIMURA, Akinori, c/o Shionogi & Co., Ltd, Toyonaka-shi, Osaka 561-0825 (JP)
(74) Representative: Vossius & Partner
(86) International application number: PCT/JP2004/011745
(87) International publication number: WO 2005/016351

(57) **Abstract**

An inhibitor for an inflammatory cell infiltration in the respiratory tract, an inhibitor for hyperirritability in the respiratory tract, a muciparous inhibitor, or a bronchodilator which contains as an active ingredient a compound represented by the formula (I) or (II): 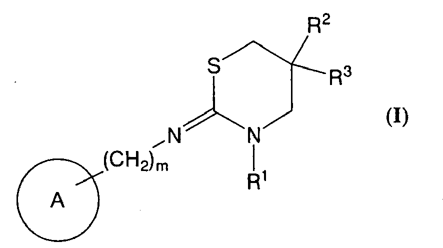 wherein R¹ is the group represented by the formula: -C(=Z)-W-R⁴ wherein Z is an oxygen atom or the like; W is an oxygen atom or the like; R⁴ is optionally substituted alkyl or the like;
R² and R³ are independently optionally substituted alkyl or the like; or
R² and R³ are taken together to form optionally substituted alkylene which may contain a heteroatom(s);
m is an integer of 0 to 2;
A is optionally substituted aryl or optionally substituted heteroaryl; 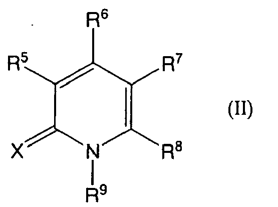 wherein R⁵ is the group represented by the formula: -Y¹-Y²-Y³-R^{a} wherein Y¹ and Y³ are each independently a bond or the like; Y² is -C(=O)-NR^{b}- or the like; R^{a} is optionally substituted alkyl, or the like; R^{b} is a hydrogen atom or the like;
R⁶ is a hydrogen atom or the like;
R⁷ and R⁸ are each independently optionally substituted alkyl or the like; or
R⁷ and R⁸ are taken together with the adjacent carbon atoms to form a 5 to 8 membered ring which may contain a heteroatom(s) and /or an unsaturated bond (s);
R⁹ is optionally substituted alkyl which may contain a heteroatom(s) and /or an unsaturated bond(s), or the like;
X is a oxygen atom or the like.

## Description

### Techinical Field

The present invention relates to an inhibitor for inflammatory cell infiltration in the respiratory tract, an inhibitor for hyperirritability in the respiratory tract, a muciparous inhibitor, or a bronchodilator which contains a compound having a cannabinoid receptor agonistic acitivity as an active ingredient.

### Background Art

In Patent 1 and Non-Patent 1, it is described that (R)-methanandamide which is a cannabinoid receptor modulator and a cannabinoid receptor agonist, exhibits an inhibitory activity for hyperirritability in the respiratory tract. Furthermore, in Non-Patent 1, 2, 3, 4, and 5, it is described that cannabinoid, anandamide, nabilone, and CP55,940, which are cannabinoid receptor agonists exhibit an inhibitory activity for constriction of bronchial plain muscle. However, an inhibitory activity for inflammatory cell infiltration in the respiratory tract and a muciparous inhibitory activity are not described in the literatures. In Patent 2, it is described that a cannabinoid receptor agonist exhibits preventing effect and/or treating effect for asthma. Furthermore, in Patent 3, it is described that a cannabinoid receptor agonist exhibits treating effect for espiratory illness.

As a cannabinoid receptor agonist, are disclosed quinoline derivatives in Patent 4 and Patent 5, thiazine derivatives in Patent 6 and Patent 7, pyridone derivatives in Patent 8 and the like.
Patent 1: WO03/061699
Patent 2: WO02/10135
Patent 3: WO04/000807
Patent 4: WO99/02499
Patent 5: WO00/40562
Patent 6: WO01/19807
Patent 7: WO02/072562
Patent 8: WO02/053543
Non-Patent 1: British Journal of Pharmacology, 2001, 134(4), 771-776
Non-Patent 2: Journal of Cannabis Therapeutics, 2002, 2(1), 59-71
Non-Patent 3: Marihuana and Medicine, New York, 1999, Mar. 20-21, 1998
Non-Patent 4: Pharmacol. Marihuna, 1976, 1, 269-276
Non-Patent 5: American Review of Respivatory Disease

### Disclosure of Invention

The object of the present invention is to provide an inhibitor for inflammatory cell infiltration in the respiratory tract, an inhibitor for hyperirritability in the respiratory tract, a muciparous inhibitor, or a bronchodilator which contains as an active ingredient a compound having a cannabinoid receptor agonistic acitivity.

The inventors of the present invention have found that the cannabinoid receptor agonist as shown below exhibits strong effect as an inhibitor for inflammatory cell infiltration in the respiratory tract, an inhibitor for hyperirritability in the respiratory tract, a muciparous inhibitor, or a bronchodilator.

The present invention relates to 1) an inhibitor for inflammatory cell infiltration in the respiratory tract, an inhibitor for hyperirritability in the respiratory tract, a muciparous inhibitor, or a bronchodilator which contains as an active ingredient a compound represented by the formula (I): 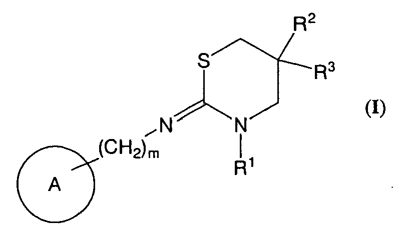 wherein R¹ is the group represented by the formula: -C(=Z)-W-R⁴ wherein Z is a oxygen atom or a sulfur atom; W is a oxygen atom or a sulfur atom; R⁴ is optionally substituted alkyl, optionally substituted alkenyl, or optionally substituted alkynyl;
R² and R³ are independently optionally substituted alkyl or optionally substituted cycloalkyl; or
R² and R³ are taken together to form alkylene which may contain an optionally substituted heteroatom(s);
m is an integer of 0 to 2;
A is optionally substituted aryl or optionally substituted heteroaryl,
2) An inhibitor for inflammatory cell infiltration in the respiratory tract, an inghibitor for hyperirritability in the respiratory tract, a muciparous inhibitor, or a bronchodilator according to 1) wherein R¹ is the group represented by the formula: -C(=Z)-W-R⁴ wherein Z is a oxygen atom or a sulfur atom; W is a sulfur atom; R⁴ is optionally substituted alkyl or alkenyl; R² and R³ are independently alkyl; or R² and R³ taken together may form optionally substituted alkylene; m is 0; A is aryl optionally substituted with one or two substitutent(s) selected from the group consisting of alkyl, haloalkyl, hydroxy, alkoxy, haloalkoxy, alkylthio, and haloalkylthio,
3) An inhibitor for inflammatory cell infiltration in the respiratory tract, an inhibitor for hyperirritability in the respiratory tract, a muciparous inhibitor, or a bronchodilator which contains as an active ingredient a compound represented by the formula (II): 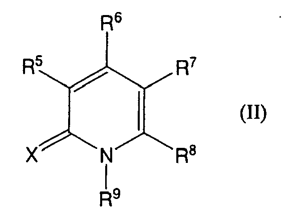 wherein R⁵ is the group represented by the formula: -Y¹-Y²-Y³-R^{a} wherein Y¹ and Y³ are each independently a bond or optionally substituted alkylene; Y² is a bond, -O- -O-SO₂-, -NR^{b}-, -NR^{b}-C(=O)-, -NR^{b}-SO₂-, -NR^{b}-C(=O)-O-, -NR^{b}-C(=O)-NR^{b}-, -NR^{b}-C(=S)-NR^{b}-, -S-, -C(=O)-O-, or -C(=O)-NR^{b}-; R^{a} is optionally substituted alkyl, optionally substituted alkenyl, optionally substituted alkylnyl, an optionally substituted carbocyclic group, an optionally substituted heterocyclic group, or acyl; R^{b} is each independently a hydrogen atom, optionally substituted alkyl, or acyl;
   R⁶ is a hydrogen atom, optionally substituted alkyl, optionally substituted alkenyl, optionally substituted alkylnyl, a halogen atom, or alkoxy;
   R⁷ and R⁸ are each independently a hydrogen atom, optionally substituted alkyl, optionally substituted alkenyl, optionally substituted alkylnyl, a halogen atom, optionally substituted phenyl, or optionally substituted carbamoyl; or
   R⁷ and R⁸ are taken together with the adjacent carbon atoms to form a 5 to 8 membered ring which may contain a heteroatom(s) and /or an unsaturated bond(s);
   R⁹ is a hydrogen atom, optionally substituted alkyl which may contain a heteroatom(s) and /or an unsaturated bond(s), or the group represented by the formula -Y⁶-R^{e} wherein Y⁶ is a bond, optionally substituted alkylene, alkenylene, alkylnylene, -O-, -S-, -SO-, or -SO₂-; R^{e} is an optionally substituted carbocyclic group or an optionally substituted heterocyclic group;
   X is a oxygen atom or a sulfur atom,
4) An inhibitor for inflammatory cell infiltration in the respiratory tract, an inhibitor for hyperirritability in the respiratory tract, a muciparous inhibitor, or a bronchodilator according to 3) wherein R⁵ is the group represented by the formula: -Y¹-Y²-Y³-R^{a} wherein Y¹ is a bond; Y² is -C(=O)-NH-; Y³ is a bond or optionally substituted alkylene; R^{a} is an optionally substituted carbocyclic group; R⁶ is a hydrogen atom; R⁷ is alkyl, a halogen atom, or optionally substituted phenyl; R⁸ is a hydrogen atom or alkyl; or R⁷ and R⁸ are taken together with the adjacent carbon atoms to form a 8 membered ring which may contain an unsaturated bond(s); R⁹ is optionally substituted C3 or more alkyl which may contain a heteroatom(s) and /or an unsaturated bond(s), or the group represented by the formula -Y⁶-R^{e} wherein Y⁶ is a bond or optionally substituted alkylene; R^{e} is an optionally substituted carbocyclic group,
5) Use of a compounds represented by the formula (I) in 1) or (II) in 3) for preparation of a pharmaceutical composition for preventing and/or treating an inflammatory cell infiltration in the respiratory tract, a hyperirritability in the respiratory tract, a muciparous, or a bronchoconstrictive action,
6) A method for preventing and/or treating a mammal, including a human, to alleviate the pathological effects of an inflammatory cell infiltration in the respiratory tract, a hyperirritability in the respiratory tract, a muciparous, or a bronchoconstrictive action wherein the method comprises administration to said mammal of a compound represented by the formula (I) in 1) or (II) in 3) in a pharmaceutically effective amount.

In the present specification, "cannabinoid" is a general term including about 30 compounds having the fundamental skeleton represented by the formula (III) wherein is two isoprene groups bonds with 5-pentylresorcinol which is included in an amulet at 2-position, cyclization derivatives thereof, oxidation derivatives thereof, and a transformation derivatives thereof. Examples are the following Δ⁹⁻tetrahydrocannabinol and the like.

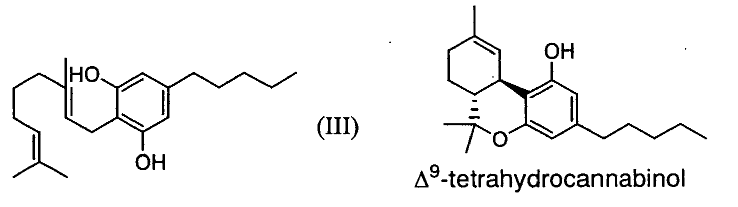

The meaning of each term are shown as follows. Each term is used to express the same meaning employed alone or in combination with other terms in the specification.

In the present specification, the term "halogen atom" means fluorine atom, chlorine atom, bromine atom, and iodine atom.

The term "alkyl" includes a straight- or branched chain C1-C10 alkyl. Examples are methyl, ethyl, n-propyl, isopropyl, n-butyl, isobutyl, sec-butyl, t-butyl, npentyl, isopentyl, neo-pentyl, n-hexyl, n-heptyl, n-octyl, n-nonyl, n-decyl, and the like. Especially, preferable is a straight- or branched chain C1-C4 alkyl, for example, preferable are methyl, ethyl, n-propyl, isopropyl, n-butyl, isobutyl, sec-butyl, or t-buty.

The term "alkenyl" includes a straight- or branched chain C2-C8 alkenyl which is the above-mentioned "alkyl" substituted with one or more double bond. Examples are viny, 1-propenyl, allyl, isopropenyl, 1-buteneyl, 2-buteneyl, 3-buteneyl, 3-pentenyl, 1,3-butadienyl, 3-methyl-2-butenyl, and tke like. Especially, preferable is a straight- or branched chain C2-C4 alkenyl, for example, preferable are allyl, isopropenyl, or 3-buteneyl.

The term "alkynyl" includes a straight- or branched chain C2-C8 alkynyl which is the above-mentioned "alkyl" substituted with one or more triple bond. Examples are ethynyl, propargyl, and tke like. Especially, preferable is a straight- or branched chain C2-C4 alkynyl, for example, preferable is propargyl.

The term "haloalkyl" means the above-mentioned "alkyl" substituted with one or more halogen atom(s). Example are chloromethyl, dichloromethyl, difluoromethyl, trifluoromethyl, chloroethyl (e.g. 2-chloroethyl), dichloroethyl (e.g., 1,2-dichloroethyl, 2,2-chloroethyl), chloropropyl (e.g., 2- chloropropyl, 3-chloropropyl), and the like. Preferable is haloC1-C3 alkyl.

The term "alkylene" includes straight- or branched chain C1-C10 alkylene. Examples are methylene, ethylene, trimethylene, tetramethylene, pentamethylene, hexamethylene, heptamethylene, 1-methylethylene, 1-ethylethylene, 1-dimethylethylene, 1,2-dimethylethylene, 1,1-diethylethylene, 1,2-diethylethylene, 1-ethyl-2-methylethylene, 1-methyltrimethylene, 2-methyltrimethylene, 1,1-dimethyltrimethylene, 1,2-dimethyltrimethylene, 2,2-dimethyltrimethylene, 1-ethyltrimethylene, 2-ethyltrimethylene, 1,1-diethyltrimethylene, 1,2-diethyltrimethylene, 2,2-diethyltrimethylene, 2-ethyl-2-methyltrimethylene, 2,2-di-n-propyltrimethylene, 1-methyltetramethylene, 2-methyltetramethylene, 1,1-dimethyltetramethylene, 1,2-dimethyltetramethylene, 2,2-dimethyltetramethylene, 3,3-dimethylpentamethylene, and the like. Especially, preferable is a straight- or branched chain C1-C6 alkylene, for example, preferable are methylene, ethylene, trimethylene, tetramethylene, pentamethylene, or hexamethylene.

Alkylene (e.g., methylene, ethylene, trimethylene, tetramethylene, pentamethylene), cycloalkyl (e.g., cyclopropyl, cyclo, trimethylene, tetramethylene, pentamethylene), alkoxy (e.g., methoxy, ethoxy), alkylthio (e.g., methylthio, ethylthio), alkylamino (e.g., methylamino, ethylamino, dimethylamino), acylamino (e.g., acetylamino), aryl (e.g., phenyl), aryloxy (e.g., phenoxy), halogen (e.g., fluoro, chloro, bromo, iodo), hydroxy, amino, nitro, alkylsulfonyl (e.g., methanesulfonyl, ethanesulfonyl), arylsulfonyl (e.g., benzensulfonyl), cyano, hydroxyamino, carboxy, alkoxycarbonyl (e.g., methoxycarbonyl, ethoxycarbonyl), acyl (e.g., acetyl, benzoyl), aralkyl (e.g., benzyl), mercapto, hydrazino, amidino, guanidino or the like is exemplified as the substituents of "optionally substituted alkylene". One to four of these substituents may substitute at any position.

Furthermore, alkylene substituted with alkylene includes alkylene substituted atom with alkylene via a spiro (e.g., 2,2-ethylenetrimethylene, 2,2-trimethylenetrimethylene, 2,2-tetramethylenetrimethylene, 2,2-pentamethylenetrimethylene), and alkylene substituted with alkylene at different position (e.g., 1,2-tetramethyleneethylene, 1,2-ethylenetrimethylene). For example, preferable are 2,2-ethylenetrimethylene, 2,2-trimethylenetrimethylene, 2,2-tetramethylenetrimethylene, and 2,2-pentamethylenetrimethylene. Especially, preferable are 2,2-ethylenetrimethylene, 2,2-tetramethylenetrimethylene, and 2,2-pentamethylenetrimethylene.

The term "alkylene may contain a heteroatom(s)" includes straight- and branched chain C2-C10 alkylene which may contain optionally substituted one to three heteroatom(s). Examples are ethylene, trimethylene, tetramethylene, pentamethylene, methylenedioxy, ethylenedioxy, ethyleneoxyethylene, and the like, Especially, preferable is straight- C3-C5 alkylene may contain one heteroatom. Tetramethylene, pentamethylene, ethyleneoxyethylene, ethyleneaminoethylene, and ethylenethioethylene are exemplified.

The term "alkenylene" includes straight- or branched chain C2-C12 alkenylene which is the above-mentioned "alkylene" having one or more double bond(s). Examples are vinylene, propenylene, and butenylene. Preferable is straight- chain C2-C6 alkenylene. For example, vinylene, propenylene, butenylene, pentenylene, hexenylene, butadienylene, or the like.

The term "alkynylene" includes straight- or branched chain C2-C12 alkynylene which is the above-mentioned "alkylene" having one or more triple bond(s).

The term "a carbocyclic group" includes a cyclic group consisting of a carbon atom and a hydrogen atom. Further, "a carbocyclic group"may be a saturated ring or an unsaturated ring. Examples are the blow-mentioned "aryl", the blow-mentioned "cycloalkyl", the blow-mentioned "cycloalkenyl", and the like. Preferable is the group derived from a C3-C14 ring.

The term "cycloalkyl" includes C3-C10 saturated carbocyclic group. Examples are cyclopropyl, cyclobutyl, cyclopentyl, cyclohexyl, cycloheptyl, cyclooctyl, and the like. Preferable is C3-C6 cycloalkyl, and examples are cyclopropyl, cyclobutyl, cyclopentyl, or cyclohexyl.

The term "cycloalkenyl" includes C3-C12 cycloalkenyl which is the above-mentioned "cycloalkyl" having one or more double bond(s). Examples are cyclopropenyl (e.g., 1-cyclopropenyl), cyclobutenyl (e.g., 1-cyclobutenyl), cyclopentenyl (e.g., 1-cyclopenten-1-yl, 2-cyclopenten-1-yl, 3-cyclopenten-1-yl), cyclohexenyl (e.g., 1-cyclohexen-1-yl, 2-cyclohexen-1-yl, 3-cyclohexen-1-yl), cycloheptenyl (e.g., 1-cycloheptenyl), cyclooctenyl (1-cyclooctenyl), and the like. Especially, preferable are 1-cyclohexen-1-yl, 2-cyclohexen-1-yl, and 3-cyclohexen-1-yl.

The term "aryl" includes a C6-C14 aryl, and examples are phenyl, naphthyl, anthryl, phenanthryl, and the like. Especially, preferable are phenyl and naphthyl.

The term "aralkyl" includes the above-mentioned "alkyl" substituted with the above-mentioned "aryl". Examples are benzyl, phenylethyl (e.g., 1-phenylethyl, 2-phenylethyl), phenylpropyl (e.g., 1-phenylpropyl, 2-phenylpropyl, 3-phenylpropyl), naphthylmethyl (e.g., 1-naphthylmethyl, 2-naphthylmethyl), and the like. Especially, preferable are benzyl and naphthylmethyl.

The term "heteroaryl" includes a C1-C9 heteroaryl having one to four nitrogen atom(s), oxygen atom(s) and/or sulfur atom(s). Examples are furyl (e.g., 2-furyl, 3-furyl), thienyl (e.g., 2-thienyl, 3-thienyl), pyrrolyl (e.g., 1-pyrrolyl, 2-pyrrolyl, 3-pyrrolyl), imidazolyl (e.g., 1-imidazolyl, 2-imidazolyl, 4-imidazolyl), pyrazolyl (e.g., 1-pyrazolyl, 3-pyrazolyl, 4-pyrazolyl), triazolyl (e.g., 1,2,4-triazol-1-yl, 1,2,4-triazol-3-yl, 1,2,4-triazol-4-yl), tetrazolyl (e.g., 1-tetrazolyl, 2-tetrazolyl, 5-tetrazolyl), oxazolyl (e.g., 2-oxazolyl, 4-oxazolyl, 5-oxazolyl), isoxazolyl (e.g., 3-isoxazolyl, 4-isoxazolyl, 5-isoxazolyl), thiazolyl (e.g., 2-thiazolyl, 4-thiazolyl, 5-thiazolyl), thiadiazolyl, isothiazolyl (e.g., 3-isothiazolyl, 4-isothiazolyl, 5-isothiazolyl), pyridyl (e.g., 2-pyridyl, 3-pyridyl, 4-pyridyl), pyridazinyl (e.g., 3-pyridazinyl, 4-pyridazinyl), pyrimidinyl (e.g., 2-pyrimidinyl, 4-pyrimidinyl, 5-pyrimidinyl), furazanyl (e.g., 3-furazanyl), pyrazinyl (e.g., 2-pyrazinyl), oxadiazolyl (e.g., 1,3,4-oxadiazol-2-yl), benzofuryl (e.g., 2-benzo[b]furyl, 3-benzo[b]furyl, 4-benzo[b]furyl, 5-benzo[b]furyl, 6-benzo[b]furyl, 7-benzo[b]furyl), benzothienyl (e.g., 2-benzo[b]thienyl, 3-benzo[b]thienyl, 4-benzo[b]thienyl, 5-benzo[b]thienyl, 6-benzo[b]thienyl, 7-benzo[b]thienyl), benzimidazolyl (e.g., 1-benzimidazolyl, 2-benzimidazolyl, 4-benzimidazolyl, 5-benzimidazolyl), dibenzofuryl, benzoxazolyl, quinoxalinyl (e.g., 2-quinoxalinyl, 5-quinoxalinyl, 6-quinoxalinyl), cinnolinyl (e.g., 3-cinnolinyl, 4-cinnolinyl, 5-cinnolinyl, 6- cinnolinyl, 7-cinnolinyl, 8-cinnolinyl), quinazolinyl (e.g., 2-quinazolinyl, 4-quinazolinyl, 5-quinazolinyl, 6-quinazolinyl, 7-quinazolinyl, 8-quinazolinyl), quinolyl (e.g., 2-quinolyl, 3-quinolyl, 4-quinolyl, 5-quinolyl, 6-quinolyl, 7-quinolyl, 8-quinolyl), phthalazinyl (e.g., 1-phthalazinyl, 5-phthalazinyl, 6-phthalazinyl), isoquinolyl (e.g., 1-isoquinolyl, 3-isoquinolyl, 4-isoquinolyl, 5-isoquinolyl, 6-isoquinolyl, 7-isoquinolyl, 8-isoquinolyl), puryl, pteridinyl (e.g., 2-pteridinyl, 4-pteridinyl, 6-pteridinyl, 7-pteridinyl), carbazolyl, phenanthridinyl, acridinyl (e.g., 1-acridinyl, 2-acridinyl, 3-acridinyl, 4-acridinyl, 9-acridinyl), indolyl (e.g., 1-indolyl, 2-indolyl, 3-indolyl; 4-indolyl, 5-indolyl, 6-indolyl, 7-indolyl), isoindolyl, phenazinyl (e.g., 1-phenazinyl, 2-phenazinyl) or phenothiadinyl (e.g., 1-phenothiadinyl, 2-phenothiadinyl, 3- phenothiadinyl, 4-phenothiadinyl), and the like.

The term "a heterocyclic group" includes the group derived from a C1-C14 mono cyclic ring having one to four nitrogen atom(s), oxygen atom(s) and/or sulfur atom(s) and the group derived from a condensed ring which are combined two to three c rings. For example, "a heterocyclic group" includes the above-mentioned "heteroaryl" and the below-mentioned "non-heteroaryl".

The term "non-heteraryl" includes a C1-C9 non-aromatic ring having one to four nitrogen atom(s), oxygen atom(s) and/or sulfur atom(s). Examples are 1-pyrrolinyl, 2-pyrrolinyl, 3-pyrrolinyl, pyrrolidino, 2-pyrrolidinyl, 3-pyrrolidinyl, 1-imidazolinyl, 2-imidazolinyl, 4-imidazolinyl, 1-imidazolidinyl, 2-imidazolidinyl, 4-imidazolidinyl, 1-pyrazolinyl, 3-pyrazolinyl, 4-pyrazolinyl, 1-pyrazolidinyl, 3-pyrazolidinyl, 4-pyrazolidinyl, piperidino, 2-piperidyl, 3-piperidyl, 4-piperidyl, piperazino, 2-piperazinyl, 2-morpholinyl, 3-morpholinyl, morpholino, tetrahydropyranyl, and the like. Especially, preferable are morpholino, pyrrolidino, piperidino and piperazino.

The alkyl part of "alkoxy" is defined as the above "alkyl". Methoxy, ethoxy, n-propoxy, isopropoxy, n-butoxy, isobutoxy, sec-butoxy, t-butoxy, n-pentyloxy, n-hexyloxy, n-heptyloxy, n-octyloxy, and the like are exemplified as "alkoxy". Preferable are methoxy, ethoxy, n-propoxy, isopropoxy, n-butoxy, i-butoxy, sec-butoxy and t-butoxy.

The alkenyl part of "alkenyloxy" is defined as the above "alkenyl". Vinyloxy, 1-propenyloxy, 2-propenyloxy, 1-butenyloxy, 2-butenyloxy, 3-butenyloxy, 1,3-butadienyloxy, 3-methyl-2-butenyloxy, and the like are exemplified as "alkenyloxy". Preferred is 2-propenyloxy and 1-butenyloxy.

The term "haloalkoxy" means the above "alkoxy" substituted with one or more halogen. Examples are dichloromethoxy, difluoromethoxy, trifluoromethoxy, trifluoroethoxy (2,2,2-trifluoroethoxy), and the like. Especially, preferable are difluoromethoxy and trifluoromethoxy.

The term "aryloxy" includes an oxygen atom substituted with the above "aryl". Examples are phenoxy, naphthoxy (e.g., 1-naphthoxy, 2-naphthoxy), anthryloxy (e.g., 1-anthryloxy, 2-anthryloxy), phenanthryl (e.g., 1-phenanthryl, 2-phenanthryl) and the like. Especially, preferable are phenoxy and naphthoxy.

The term "alkoxyalkoxy" includes the above-mentioned "alkoxy" substituted with the above-mentioned "alkoxy". Examples are methoxymethoxy, ethoxymethoxy, n-propoxymethoxy, isopropoxymethoxy, 1-methoxyethoxy, 2-methoxyethoxy, and the like. Especially, preferable are 1-methoxyethoxy, 2-methoxyethoxy.

The term "alkylthioalkoxy" includes the above-mentioned "alkoxy" substituted with the below-mentioned "alkylthio". Examples are methylthiomethoxy, ethylthiomethoxy, n-propylthiomethoxy, isopropylthiomethoxy, 1-methylthioethoxy, 2-methylthioethoxy, and the like. Especially, preferable are 1-methylthioethoxy and 2-methylthioethoxy.

The alkyl part of "alkylthio" is defined as the above-mentioned "alkyl". Examples are methylthio, ethylthio, n-propylthio, isopropylthio, n-butylthio, isobutylthio, sec-butylthio, t-butylthio, n-pentylthio, n-hexylthio and the like. Especially, preferable is C1-C4 straight- or branched chain alkylthio. For example, methylthio, ethylthio, n-propylthio, i-propylthio, n-butylthio, i-butylthio, sec-butylthio, and t-butylthio are exemplified.

The term "haloalkylthio" means the above "alkylthio" substituted with one or more halogen. Examples are dichloromethylthio, difluoromethylthio, trifluoromethylthio, trifluoroethylthio (2,2,2-trifluoroethylthio) and the like. Preferable are difluoromethylthio and trifluoromethylthio.

Non-substituted amino, alkylamino (e.g., methylamino, ethylamino, n-propylamino, i-propylamino, dimethylamino, diethylamino, ethylmethylamino, propylmethylamino), acylamino (e.g., acetylamino, formylamino, propionylamino, benzoylamino), acylalkylamino (e.g., N-acethylmethylamino), aralkylamino (e.g., benzylamino, 1-phenylethylamino, 2-phenylethylamino, 1-phenylpropylamino, 2-phenylpropylamino, 3-phenylpropylamino, 1-naphthylmethylamino, 2-naphthylmethylamino, dibenzylamino), alkylsulfonylamino (e.g., methanesulfonylamino, ethanesulfonylamino), alkenyloxysulfonylamino (e.g., vinyloxysulfonylamino, allyloxysulfonylamino), alkoxycarbonylamino (e.g., methoxycaronylamino, ethoxycaronylamino, t-butoxycaronylamino), alkenylamono (e.g., vinylamino, allylamino), arylcarbonylamino (e.g., benzoylamino), and heteroarylcarbonylamino (e.g., pyridinecarboylamino) are exemplified as "optionally substituted amino".

The term "aralkylamino" means amino substituted with one or two the above-mentioned "aralkyl". Examples are benzylamino, phenylethylamino (e.g., 1-phenylethylamino, 2-phenylethylainino), phenylpropylamino (e.g., 1-phenylpropylamino, 2-phenylpropylamino, 3-phenylpropylamino), naphthylamino (e.g., 1-naphthylamin, 2-naphthylamin), dibenzylamino, and the like.

The term "acyl" means carbonyl substituted with the group except for a hydrogen atom. Examples are alkylcarbonyl (e.g., acetyl, propionyl, butyryl, isobutyryl, valeryl, isovaleryl, pivaloryl, hexanoyl, octanoyl, lauroyl), alkenylcarbonyl (e.g., acryloyl, methacryloyl), cycloalkylcarbonyl (e.g., cyclopropanecarbonyl, cyclobutanecarbonyl, cyclopentanecarbonyl, cyclohexanecarbonyl), arylcarbonyl (e.g., benzoyl, naphthoyl), and heteroarylcarbonyl (e.g., pyridinecarbonyl). These groups may be substuituted with alkyl, a halogen atom, or the like. Toluoyl which is an example of arylcarbonyl substituted with alkyl and trifluoroacetyl which is an example of alkylcarbonyl substituted with halogen atom are exemplified.

The term "alkoxycarbonyl" means carbonyl substituted with the above-mentioned "alkoxy". Examples are methoxycarbonyl, ethoxycarbonyl, n-propoxycarbonyl, i-propoxycarbonyl, n-butoxycarbonyl, i-butoxycarbonyl, sec-butoxycarbonyl, tert-butoxycarbonyl, n-pentyloxycarbonyl, n-hexyloxycarbonyl, n-heptyloxycarbonyl, n-hexyloxycarbonyl, n-heptyloxycarbonyl, n-octyloxycarbonyl, and the like. Preferable are methoxycarbonyl, ethoxycarbonyl and the like.

Alkyl (e.g., methyl, ethyl, n-propyl, i-propyl), acyl (e.g., formyl, acetyl, propionyl, benzoyl) and the like are exemplified as the substituents of "optionally substituted carbamoyl". The nitrogen atom of a carbamoyl group may be mono- or di- substituted with these substituents. Preferable are carbmoyl, N-methyl carbmoyl, N-ethyl carbmoyl, and the like as "optionally substituted carbamoyl".

The alkyl part of "alkylsulfonyl" is defined as the above-mentioned "alkyl". Methanesulfonyl, ethanesulfonyl and the like are exemplified as "alkylsulfonyl".

When "optionally substituted aralkyloxy", "optionally substituted aralkylthio", "optionally substituted aralkylamino", "optionally substituted phenyl", "optionally substituted aryl", "optionally substituted heteroaryl", "optionally substituted heteroaryl", "an optionally substituted heterocyclic group", "optionally substituted alkyl", "optionally substituted alkenyl", "optionally substituted alkynyl", "optionally substituted alkoxyalkyl", "optionally substituted cycloalkyl", "an optionally substituted carbocyclic group", "alkylene which may contain optionally substituted a heteroatom(s)", or "optionally substituted alkyl which may contain optionally substituted a heteroatom(s) and/or an unsubstituted bond(s)" has substituent(s), each one to four of these substituents may substitute at any position.

Hydroxy, carboxy, halogen atom (fluorine atom, chlorine atom, bromine atom, iodine atom), haloalkyl (e.g., CF₃, CH₂CF₃, CH₂CCl₃), haloalkoxy, alkyl (e.g., methyl, ethyl, isopropyl, tert-butyl), alkenyl (e.g., vinyl), formyl, acyl (e.g., acetyl, propionyl, butyryl, pivoloyl, benzoyl, pyridinecarbonyl, cyclopentanecarbonyl, cyclohexanecarbonyl), alkynyl (e.g., ethynyl), cycloalkyl (e.g., cyclopropyl, cyclobutyl, cyclopentyl, cyclohexyl), alkoxy (e.g., methoxy, ethoxy, propoxy, butoxy), alkoxycarbonyl (e.g., methoxycarbonyl, ethoxycarbonyl, tert-butoxycarbonyl), nitro, nitroso, oxo, optionally substituted amino (e.g., amino, alkylamino (e.g., methylamino, ethylamino, dimethylamino), formylamino, acylamino (e.g., acetylamino, benzoylamino), aralkylamino (e.g., benzylamino, tritylamino), hydroxyamino, alkylsulfonylamino, alkenyloxycarbonylamino, alkoxycarbonylamino, alkenylamino, arylcarbonylamino, heteroarylcarbonylamino), azido, aryl (e.g., phenyl), aryloxy (e.g., phenoxy), aralkyl (e.g., benzyl, phenethyl, phenylpropyl), alkylenedioxy (e.g., methylenedioxy), alkylene (e.g., methylene, ethylene, trimethylene, teteramethylene, pentamethylene), alkenylene (e.g., propenylene, butenylene, butadienylen), cyano, isocyano, isocyanato, thiocyanato, isothiocyanato, mercapto, alkylthio (e.g., methylthio, ethylthio), alkylsulfonyl (e.g., omethanesulfonyl, ethanesulfonyl), arylsuslfonyl (e.g., benzensulfonyl), optionally substituted carbamoyl, sulfamoyl, formyloxy, haloformyl, oxalo, thioformyl, thiocarboxy, dithiocarboxy, thiocarbamoyl, sulfino, sulfo, sulfoamino, hydrazino, ureido, amidino, guanidino, alkylsulfonyloxy, trialkylsilyl, haloalkylcarbonyloxy, formyloxy, acylthio, thioxo, alkoxyalkoxy, alkylthioalkoxy, and the like are exemplified as their substituents.

Preferable are oxo, hydroxy, alkenylene (e.g., propenylene, butenylene, butadienylene), acyl (e.g., acetyl, propionyl, pivaloyl, benzoyl, pyridinecarbonyl, cyclopentanecarbonyl, cyclohexanecarbonyl), aralkyl (e.g., benzyl), alkylene (e.g., methylene, ethylene, trimethylene, tetramethlene, pentamethylene), and the like as the substituents of "5-8 menbered ring which may contain a heteroatom(s) and/or an unsaturated bond(s)"

Substsituents groups (Ia) to (Im) are shown as preferable substituent(s) groups for R¹ to R³, m, and A of the compound represented by general formula (I).
R¹: (Ia) -C(=S)-S-R⁴ or -C(=O)-S-R⁴ wherein R⁴ is optionally substituted alkyl or optionally substituted alkenyl, (Ib) -C(=S)-S-R⁴ or -C(=O)-S-R⁴ wherein R⁴ is optionally substituted alkyl, (Ic) -C(=S)-S-R⁴ wherein R⁴ is optionally substituted alkyl.
R²: (Id) optionally substituted alkyl, (Ie) alkyl.
R³: (If) optionally substituted alkyl, (Ig) alkyl.
m: (Ih) 0.
A: (Ii) optionally substituted aryl or optionally substituted heteroaryl, (Ii) optionally substituted aryl, (Ik) optionally substituted heteroaryl.

Or, R² and R³ are taken together to form (Il) alkylene which may contain optionally substituted alkylene, (Im) alkylene.

Examples of preferable group of the compound represented by general formula (I) contains [R¹, R², R³, m, A]=[Ia, Id, If, Ih, Ii], [Ia, Id, If, Ih, Ij], [Ia, Id, If, Ih, Ik], [Ia, Id, Ig, Ih, Ii], [Ia, Id, Ig, Ih, Ij], [Ia, Id, Ig, Ih, Ik], [Ia, Ie, If, Ih, Ii], [Ia, Ie, If, Ih, Ij], [Ia, Ie, If, Ih, Ik], [Ia, Ie, Ig, Ih, Ii], [Ia, Ie, Ig, Ih, Ij], [Ia, Ie, Ig, Ih, Ik], [Ib, Id, If, Ih, Ii], [Ib, Id, If, Ih, Ij], [Ib, Id, If, Ih, Ik], [Ib, Id, Ig, Ih, Ii], [Ib, Id, Ig, Ih, Ij], [Ib, Id, Ig, Ih, Ik], [Ib, Ie, If, Ih, Ii], [Ib, Ie, If, Ih, Ij], [Ib, Ie, If, Ih, Ik], [Ib, Ie, Ig, Ih, Ii], [Ib, Ie, Ig, Ih, Ij], [Ib, Ie, Ig, Ih, Ik], [Ic, Id, If, Ih, Ii], [Ic, Id, If, Ih, Ij], [Ic, Id, If, Ih, Ik], [Ic, Id, Ig, Ih, Ii], [Ic, Id, Ig, Ih, Ij], [Ic, Id, Ig, Ih, Ik], [Ic, Ie, If, Ih, Ii], [Ic, Ie, If, Ih, Ij], [Ic, Ie, If, Ih, Ik], [Ic, Ie, Ig, Ih, Ii], [Ic, Ie, Ig, Ih, Ij], [Ic, Ie, Ig, Ih, Ik], or [R¹, R²-R³, m, A]=[Ia, Il, Ih, Ii], [Ia, Il, Ih, Ij], [Ia, Il, Ih, Ik], [Ia, Im, Ih, Ii], [Ia, Im, Ih, Ij], [Ia, Im, Ih, Ik], [Ib, Il, Ih, Ii], [Ib, Il, Ih, Ij], [Ib, Il, Ih, Ik], [Ib, Im, Ih, Ii], [Ib, Im, Ih, Ij], [Ib, Im, Ih, Ik], [Ic, Il, Ih, Ii], [Ic, Il, Ih, Ij], [Ic, Il, Ih, Ik], [Ic, Im, Ih, Ii], [Ic, Im, Ih, Ij], [Ic, Im, Ih, Ik].

Substituents groups (IIa) to (IIm) are shown as preferable substituent(s) groups for R⁵ to R⁹, and X of the compound represented by general formula (II).

R⁵: (IIa) -C(=O)-NH-Y³-R^{a} wherein Y³ is a bond or optionally substituted alkylene, R^{a} is optionally substituted alkyl, an optionally substituted carbocyclic group, or acyl, (IIb) -C(=O)-NH-Y³-R^{a} wherein Y³ is a bond or optionally substituted alkylene, R^{a} is an optionally substituted carbocyclic group, or acyl, (IIc) -C(=O)-NH-Y³-R^{a} wherein Y³ is a bond or optionally substituted alkylene, R^{a} is an optionally substituted carbocyclic group.

R⁶: (IId) a hydrogen atom.

R⁷: (IIe) a hydrogen atom or optionally substituted alkyl, (IIf) optionally substituted alkyl.

R⁸: (IIg) a hydrogen atom or optionally substituted alkyl, (IIh) optionally substituted alkyl.

R⁹: (IIi) optionally substituted alkyl or -Y⁶-R^{e} wherein Y⁶ is optionally substituted alkylene, R^{e} is an optinally substituted carbocyclic group, (IIj) optionally substituted alkyl.

X: (IIk) an oxygen atom.

Or, R⁷ and R⁸ are taken together with the adjacent carbon atom to form (Il) optionally substituted 5-8 membered ring, (Im) optionally substituted 8 membered ring, .

Examples of preferable group of the compound represented by general formula (II) contains [R⁵, R⁶, R⁷, R⁸, R⁹, X]=[IIa, IId, IIe, IIg, IIi, IIk], [IIa, IId, IIe, IIg, IIj, IIk], [IIa, IId, IIe, IIh, IIi, IIk], [IIa, IId, IIe, IIh, IIj, IIk], [IIa, IId, IIf, IIg, IIi, IIk], [IIa, IId, IIf, IIg, IIj, IIk], [IIa, IId, IIf, IIh, IIi, IIk], [IIa, IId, IIf, IIh, IIj, IIk], [IIb, IId, IIe, IIg, IIi, IIk], [IIb, IId, IIe, IIg, IIj, IIk], [IIb, IId, IIe, IIh, IIi, IIk], [IIb, IId, IIe, IIh, IIj, IIk], [IIb, IId, IIf, IIg, IIi, IIk], [IIb, IId, IIf, IIg, IIj, IIk], [IIb, IId, IIf, IIh, IIi, IIk], [IIb, IId, IIf, IIh, IIj, IIk], or [R⁵, R⁶, R⁷-R⁸, R⁹, X]=[IIb, IId, III, IIi, IIk], [IIc, IId, IIe, IIg, IIi, IIk], [IIc, IId, IIe, IIg, IIj, IIk], [IIc, IId, IIe, IIh, IIi, IIk], [IIc, IId, IIe, IIh, IIj, IIk], [IIc, IId, IIf, IIg, IIi, IIk], [IIc, IId, IIf, IIg, IIj, IIk], [IIc, IId, IIf, IIh, IIi, IIk], [IIc, IId, IIf, IIh, IIj, IIk].

The term "solvate" means solvates of compounds of the present invention or the pharmaceutical acceptable salts thereof. Examples are monosolvate, disolvate, monohydrate, dihydrate, and the like are exemplified as "solvate".

The compounds described in WO 01/19807 or WO 02/072562 are exemplified as the compounds represented by the formula (I). Preferable are the compounds described in the following Tables.

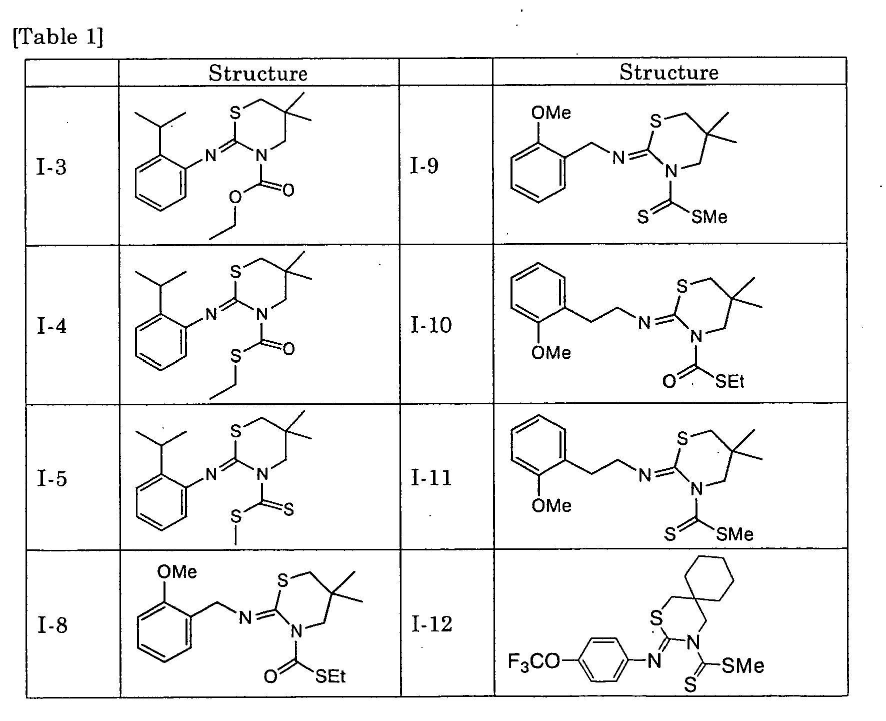

**[Table 2]**

| | | | | | | | | |
|---|---|---|---|---|---|---|---|---|
| 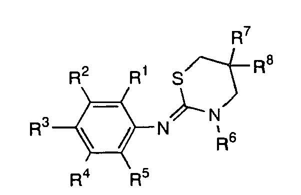 | | | | | | | | |

| | R¹ | R² | R³ | R⁴ | R⁵ | R⁶ | R⁷ | R⁸ |
|---|---|---|---|---|---|---|---|---|
| I-16 | H | H | H | H | H | COSEt | Me | Me |
| I-17 | F | H | H | H | H | COSEt | Me | Me |
| I-18 | Cl | H | H | H | H | COSEt | Me | Me |
| I-19 | Me | H | H | H | H | COSEt | Me | Me |
| I-20 | Et | H | H | H | H | COSEt | Me | Me |
| I-21 | Pr | H | H | H | H | COSEt | Me | Me |
| I-22 | Bu | H | H | H | H | COSEt | Me | Me |
| I-23 | Bu^{*s*} | H | H | H | H | COSEt | Me | Me |
| I-24 | Bu^{*t*} | H | H | H | H | COSEt | Me | Me |
| I-25 | Ph | H | H | H | H | COSEt | Me | Me |
| I-26 | CF₃ | H | H | H | H | COSEt | Me | Me |
| I-27 | OMe | H | H | H | H | COSEt | Me | Me |
| I-28 | OEt | H | H | H | H | COSEt | Me | Me |
| I-29 | OPr^{*i*} | H | H | H | H | COSEt | Me | Me |
| I-30 | SMe | H | H | H | H | COSEt | Me | Me |
| I-31 | SEt | H | H | H | H | COSEt | Me | Me |
| I-32 | SPr^{*i*} | H | H | H | H | COSEt | Me | Me |
| I-33 | NMe₂ | H | H | H | H | COSEt | Me | Me |
| I-34 | H | Pr^{*i*} | H | H | H | COSEt | Me | Me |
| I-35 | H | H | Cl | H | H | COSEt | Me | Me |
| I-36 | H | H | Pr^{*i*} | H | H | COSEt | Me | Me |
| I-37 | H | H | NO₂ | H | H | COSEt | Me | Me |
| I-38 | Me | Me | H | H | H | COSEt | Me | Me |
| I-39 | Me | H | Me | H | H | COSEt | Me | Me |
| I-40 | Me | H | H | Me | H | COSEt | Me | Me |
| I-41 | Me | H | H | H | Me | COSEt | Me | Me |
| I-42 | H | Me | Me | H | H | COSEt | Me | Me |
| I-43 | H | Me | H | Me | H | COSEt | Me | Me |
| I-44 | Me | H | Cl | H | H | COSEt | Me | Me |
| I-45 | Cl | H | Me | H | H | COSEt | Me | Me |
| I-46 | Pr^{*i*} | H | NO₂ | H | H | COSEt | Me | Me |

**[Table 3]**

| | | | | | | | | |
|---|---|---|---|---|---|---|---|---|
| 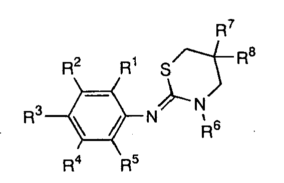 | | | | | | | | |

| | R¹ | R² | R³ | R⁴ | R⁵ | R⁶ | R⁷ | R⁸ |
|---|---|---|---|---|---|---|---|---|
| I-47 | Pr^{*i*} | H | H | H | NO₂ | COSEt | Me | Me |
| I-48 | NO₂ | H | NO₂ | H | H | COSEt | Me | Me |
| I-49 | Pr | H | H | H | H | COSMe | Me | Me |
| I-50 | Pr^{*i*} | H | H | H | H | COSMe | Me | Me |
| I-51 | Bu^{*s*} | H | H | H | H | COSMe | Me | Me |
| I-52 | H | Pr^{*i*} | H | H | H | COSMe | Me | Me |
| I-53 | H | OMe | OMe | H | H | COSMe | Me | Me |
| I-54 | H | -OCH₂O- | | H | H | COSMe | Me | Me |
| I-55 | H | OMe | OMe | OMe | H | COSMe | Me | Me |
| I-56 | Et | H | H | H | H | CSSMe | Me | Me |
| I-57 | Bu^{*s*} | H | H | H | H | CSSMe | Me | Me |
| I-58 | CH₂OMe | H | H | H | H | CSSMe | Me | Me |
| I-59 | CH(Me)O Me | H | H | H | H | CSSMe | Me | Me |
| I-60 | OMe | H | H | H | H | CSSMe | Me | Me |
| I-61 | OEt | H | H | H | H | CSSMe | Me | Me |
| I-62 | SMe | H | H | H | H | CSSMe | Me | Me |
| I-63 | SEt | H | H | H | H | CSSMe | Me | Me |
| I-64 | SPr^{*i*} | H | H | H | H | CSSMe | Me | Me |
| I-65 | SOMe | H | H | H | H | CSSMe | Me | Me |
| I-66 | SO₂Me | H | H | H | H | CSSMe | Me | Me |
| I-67 | SOEt | H | H | H | H | CSSMe | Me | Me |
| I-68 | NMe₂ | H | H | H | H | CSSMe | Me | Me |
| I-69 | H | Pr^{*i*} | H | H | H | CSSMe | Me | Me |
| I-70 | H | H | Cl | H | H | CSSMe | Me | Me |
| I-71 | Me | H | Me | H | H | CSSMe | Me | Me |
| I-72 | Me | H | H | Me | H | CSSMe | Me | Me |
| I-73 | Me | H | H | H | Me | CSSMe | Me | Me |
| I-74 | H | Me | Me | H | H | CSSMe | Me | Me |
| I-75 | H | Me | H | Me | H | CSSMe | Me | Me |
| I-76 | OMe | OMe | H | H | H | CSSMe | Me | Me |
| I-77 | H | OMe | OMe | H | H | CSSMe | Me | Me |
| I-78 | OMe | H | H | OMe | H | CSSMe | Me | Me |

**[Table 4]**

| | | | | | | | | |
|---|---|---|---|---|---|---|---|---|
| 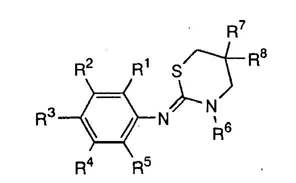 | | | | | | | | |

| | R¹ | R² | R³ | R⁴ | R⁵ | R⁶ | R⁷ | R⁸ |
|---|---|---|---|---|---|---|---|---|
| I-79 | OMe | H | OMe | | H | CSSMe | Me | Me |
| I-80 | H | -OCH₂O- | | H | H | CSSMe | Me | Me |
| I-81 | Pr^{*i*} | H | NO₂ | H | H | CSSMe | Me | Me |
| I-82 | Pr^{*i*} | H | H | H | NO₂ | CSSMe | Me | Me |
| I-83 | H | OMe | OMe | OMe | H | CSSMe | Me | Me |
| I-84 | Pr^{*i*} | H | H | H | H | CSSEt | Me | Me |
| I-85 | Bu^{*s*} | H | H | H | H | CSSEt | Me | Me |
| I-86 | OEt | H | H | H | H | CSSEt | Me | Me |
| I-87 | SMe | H | H | H | H | CSSEt | Me | Me |
| I-88 | H | Pr^{*i*} | H | H | H | CSSEt | Me | Me |
| I-118 | H | OEt | OEt | H | H | CSSMe | Me | Me |
| I-119 | OMe | H | Me | H | H | CSSMe | Me | Me |
| I-120 | OMe | H | H | Me | H | CSSMe | Me | Me |
| I-121 | H | OMe | Me | H | H | CSSMe | Me | Me |
| I-122 | Me | Me | H | H | H | CSSMe | Me | Me |
| I-123 | N(Me)Ac | H | H | H | H | CSSMe | Me | Me |

**[Table 5]**

| | | | | | | | |
|---|---|---|---|---|---|---|---|
| 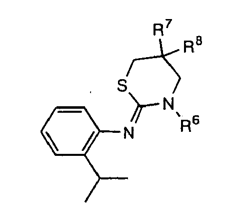 | | | | | | | |

| | R⁶ | R⁷ | R⁸ | | R⁶ | R⁷ | R⁸ |
|---|---|---|---|---|---|---|---|
| I-90 | COOMe | Me | Me | I-98 | CSSPr | Me | Me |
| I-91 | COOPr | Me | Me | I-99 | CSSPr^{*i*} | Me | Me |
| I-96 | CSOEt | Me | Me | I-100 | CSSBn | Me | Me |

**[Table 6]**

| | | | | | | | |
|---|---|---|---|---|---|---|---|
| 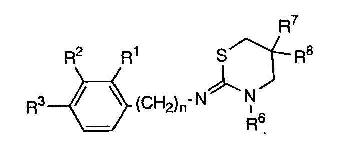 | | | | | | | |

| | R¹ | R² | R³ | n | R⁶ | R⁷ | R⁸ |
|---|---|---|---|---|---|---|---|
| I-101 | H | H | Cl | 1 | COSEt | Me | Me |
| I-102 | H | H | Cl | 1 | CSSMe | Me | Me |
| I-103 | Cl | H | Cl | 2 | COSEt | Me | Me |
| I-104 | Cl | H | Cl | 2 | CSSMe | Me | Me |

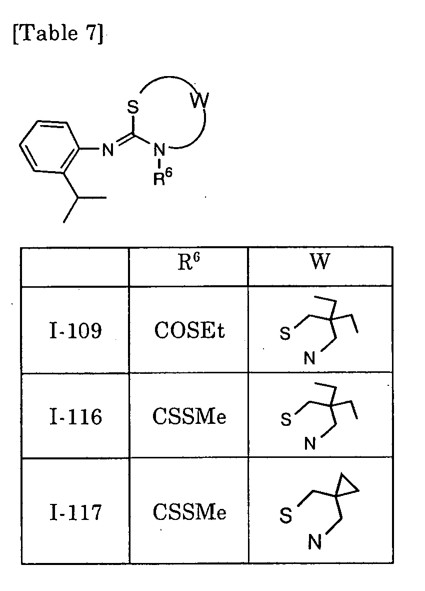

**[Table 8]**

| | | | | | | | | |
|---|---|---|---|---|---|---|---|---|
| 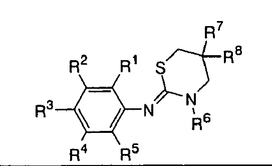 | | | | | | | | |

| | R¹ | R² | R³ | R⁴ | R⁵ | R⁶ | R⁷ | R⁸ |
|---|---|---|---|---|---|---|---|---|
| I-124 | H | H | OEt | H | H | CSSMe | Me | Me |
| I-125 | H | OEt | H | H | H | CSSMe | Me | Me |
| I-126 | H | H | OMe | H | H | CSSMe | Me | Me |
| I-127 | H | OMe | H | H | H | CSSMe | Me | Me |
| I-128 | H | OEt | OMe | H | H | CSSMe | Me | Me |
| I-129 | H | OPr | OMe | H | H | CSSMe | Me | Me |
| I-130 | H | OEt | OEt | H | H | CSSMe | Me | Me |
| I-131 | H | H | OPr | H | H | CSSMe | Me | Me |
| I-132 | H | OPr | H | H | H | CSSMe | Me | Me |
| I-133 | H | H | OBu | H | H | CSSMe | Me | Me |
| I-134 | H | OBu | H | H | H | CSSMe | Me | Me |
| I-135 | H | OMe | OEt | H | H | CSSMe | Me | Me |
| I-136 | H | OMe | OPr | H | H | CSSMe | Me | Me |
| I-137 | H | OBu | OMe | H | H | CSSMe | Me | Me |
| I-138 | H | H | OPr^{*i*} | H | H | CSSMe | Me | Me |
| I-139 | H | OPr^{*i*} | H | H | H | CSSMe | Me | Me |
| I-140 | H | H | H | H | H | CSSMe | Me | Me |
| I-141 | F | H | H | H | H | CSSMe | Me | Me |
| I-142 | Cl | H | H | H | H | CSSMe | Me | Me |
| I-143 | H | Cl | H | H | H | CSSMe | Me | Me |
| I-144 | Me | H | H | H | H | CSSMe | Me | Me |
| I-145 | H | Me | H | H | H | CSSMe | Me | Me |
| I-146 | H | H | Me | H | H | CSSMe | Me | Me |
| I-147 | H | Bu | H | H | H | CSSMe | Me | Me |
| I-148 | H | H | Bu | H | H | CSSMe | Me | Me |
| I-149 | Bu^{*t*} | H | H | H | H | CSSMe | Me | Me |
| I-150 | H | H | Et | H | H | CSSMe | Me | Me |
| I-151 | H | Et | H | H | H | CSSMe | Me | Me |
| I-152 | H | H | F | H | H | CSSMe | Me | Me |
| I-153 | H | F | H | H | H | CSSMe | Me | Me |
| I-154 | H | H | Pr^{*i*} | H | H | CSSMe | Me | Me |
| I-155 | H | H | Morpholino | H | H | CSSMe | Me | Me |
| I-156 | H | Ac | H | H | H | CSSMe | Me | Me |
| I-157 | H | H | Br | H | H | CSSMe | Me | Me |
| I-158 | H | Br | H | H | H | CSSMe | Me | Me |
| I-159 | Br | H | H | H | H | CSSMe | Me | Me |
| I-160 | H | C(Me)=N OMe | H | H | H | CSSMe | Me | Me |
| I-161 | H | H | Ac | H | H | CSSMe | Me | Me |
| I-162 | H | H | C(Me)= NOMe | H | H | CSSMe | Me | Me |
| I-163 | OPr^{*i*} | H | H | H | H | CSSMe | Me | Me |
| I-164 | Pr | H | H | H | H | CSSMe | Me | Me |
| I-165 | CF₃ | H | H | H | H | CSSMe | Me | Me |

**[Table 9]**

| | | | | | | | | |
|---|---|---|---|---|---|---|---|---|
| 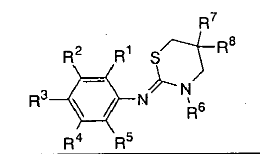 | | | | | | | | |

| | R¹ | R² | R³ | R⁴ | R⁵ | R⁶ | R⁷ | R⁸ |
|---|---|---|---|---|---|---|---|---|
| I-166 | H | H | OPh | H | H | CSSMe | Me | Me |
| I-167 | H | H | Pr | H | H | CSSMe | Me | Me |
| I-168 | H | H | Bu^{*t*} | H | H | CSSMe | Me | Me |
| I-169 | H | CF₃ | H | H | H | CSSMe | Me | Me |
| I-170 | H | H | CF₃ | H | H | CSSMe | Me | Me |
| I-171 | Pr^{*i*} | H | NHAc | H | H | CSSMe | Me | Me |
| I-172 | Pr^{*i*} | H | H | H | NHAc | CSSMe | Me | Me |
| I-173 | H | COOMe | H | H | OMe | CSSMe | Me | Me |
| I-174 | Morpholino | H | H | H | H | CSSMe | Me | Me |
| I-175 | H | Morpholino | H | H | H | CSSMe | Me | Me |
| I-176 | Pr^{*i*} | H | H | COO Et | H | CSSMe | Me | Me |
| I-177 | H | H | Piperidino | H | H | CSSMe | Me | Me |
| I-178 | Pyrrolidino | H | H | H | H | CSSMe | Me | Me |
| I-179 | H | SMe | H | H | H | CSSMe | Me | Me |
| I-180 | H | H | SMe | H | H | CSSMe | Me | Me |
| I-181 | OCF₃ | H | H | H | H | CSSMe | Me | Me |
| I-182 | H | OCF₃ | H | H | H | CSSMe | Me | Me |
| I-183 | H | H | OCF₃ | H | H | CSSMe | Me | Me |
| I-184 | H | H | 3-Pyridyl | H | H | CSSMe | Me | Me |
| I-185 | H | 3-Pyridyl | H | H | H | CSSMe | Me | Me |
| I-186 | 3-Pyridyl | H | H | H | H | CSSMe | Me | Me |
| I-187 | OPh | H | H | H | H | CSSMe | Me | Me |
| I-188 | H | OEt | OEt | H | H | COOMe | Me | Me |
| I-189 | OMe | H | H | H | H | COOMe | Me | Me |
| I-190 | H | H | Et | H | H | COOMe | Me | Me |
| I-191 | H | H | Pr^{*i*} | H | H | COOMe | Me | Me |
| I-192 | OMe | H | H | H | H | COSMe | Me | Me |
| I-193 | H | H | Et | H | H | COSMe | Me | Me |
| I-194 | H | H | Pr^{*i*} | H | H | COSMe | Me | Me |
| I-195 | H | H | OEt | H | H | COSMe | Me | Me |
| I-196 | H | OMe | OEt | H | H | COSMe | Me | Me |
| I-197 | H | Piperidino | H | H | H | CSSMe | Me | Me |
| I-198 | H | H | NEt₂ | H | H | CSSMe | Me | Me |
| I-199 | OMe | H | COOMe | H | H | CSSMe | Me | Me |
| I-200 | H | 2-Oxo pyrrolidino | H | H | H | CSSMe | Me | Me |
| I-201 | H | OPh | H | H | H | CSSMe | Me | Me |
| I-202 | H | H | Ph | H | H | CSSMe | Me | Me |
| I-203 | Ph | H | H | H | H | CSSMe | Me | Me |
| I-204 | H | Ph | H | H | H | CSSMe | Me | Me |
| I-205 | Pr^{*i*} | H | H | H | H | CSOMe | Me | Me |
| I-206 | *Pr*^{*i*} | H | I | H | H | CSSMe | Me | Me |
| I-207 | OMe | H | (Morpholi no)CO | H | H | CSSMe | Me | Me |

**[Table 10]**

| | | | | | | | | |
|---|---|---|---|---|---|---|---|---|
| 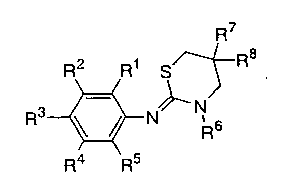 | | | | | | | | |

| | R¹ | R² | R³ | R⁴ | R⁵ | R⁶ | R⁷ | R⁸ |
|---|---|---|---|---|---|---|---|---|
| I-208 | H | H | NMe₂ | H | H | CSSMe | Me | Me |
| I-209 | H | NMe₂ | H | H | H | CSSMe | Me | Me |
| I-210 | N(Me)Et | H | H | H | H | CSSMe | Me | Me |
| I-211 | N(Me)Pr | H | H | H | H | CSSMe | Me | Me |
| I-212 | NEt₂ | H | H | H | H | CSSMe | Me | Me |
| I-213 | F | H | H | H | F | CSSMe | Me | Me |
| I-214 | Pr^{*i*} | H | Cl | H | H | CSSMe | Me | Me |
| I-215 | NMe₂ | Me | H | H | H | CSSMe | Me | Me |
| I-216 | NMe₂ | H | Me | H | H | CSSMe | Me | Me |
| I-217 | NMe₂ | H | H | Me | H | CSSMe | Me | Me |
| I-218 | NMe₂ | H | H | Cl | H | CSSMe | Me | Me |
| I-219 | Me | H | H | H | Me | CSSMe | Me | Me |
| I-220 | NMe₂ | H | H | H | H | CSSEt | Me | Me |
| I-221 | H | NMe₂ | H | H | H | CSSEt | Me | Me |
| I-222 | NMe₂ | H | Me | H | H | CSSEt | Me | Me |
| I-223 | H | H | Pr^{*i*} | H | H | CSSEt | Me | Me |
| I-224 | OMe | H | CONHM | He | H | CSSMe | Me | Me |
| I-225 | OCHF₂ | H | H | H | H | CSSMe | Me | Me |
| I-226 | H | OCHF₂ | H | H | H | CSSMe | Me | Me |
| I-227 | H | NEt₂ | H | H | H | CSSMe | Me | Me |
| I-228 | NMe₂ | H | Cl | H | H | CSSMe | Me | Me |
| I-229 | NMe₂ | H | F | H | H | CSSMe | Me | Me |
| I-230 | NMe₂ | H | H | F | H | CSSMe | Me | Me |
| I-231 | NMe₂ | H | Et | H | H | CSSMe | Me | Me |
| I-232 | NMe₂ | H | H | Et | H | CSSMe | Me | Me |
| I-233 | NMe₂ | H | Cl | H | H | CSSEt | Me | Me |
| I-234 | NMe₂ | H | F | H | H | CSSEt | Me | Me |
| I-235 | NMe₂ | H | Et | H | H | CSSEt | Me | Me |
| I-236 | Pr^{*i*} | H | H | H | H | CSSBu^{*s*} | Me | Me |
| I-237 | Pr^{*i*} | H | H | H | H | CSSBu^{*i*} | Me | Me |
| I-239 | Me | NMe₂ | H | H | H | CSSMe | Me | Me |
| I-240 | NMe₂ | OMe | H | H | H | CSSMe | Me | Me |
| I-241 | H | NMe₂ | Me | H | H | CSSMe | Me | Me |
| I-242 | NMe₂ | Cl | H | H | H | CSSMe | Me | Me |
| I-243 | H | NMe₂ | OMe | H | H | CSSMe | Me | Me |
| I-244 | Pr^{*i*} | H | H | H | H | CSSEt | Et | Et |

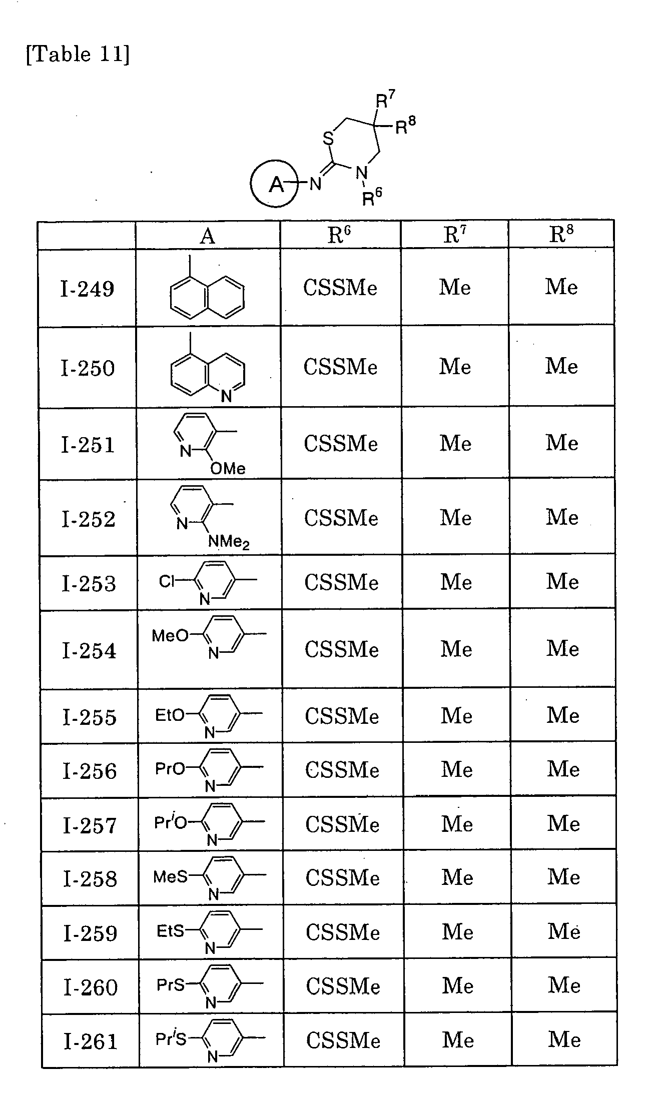

**[Table 12]**

| | | | | | | | | |
|---|---|---|---|---|---|---|---|---|
| 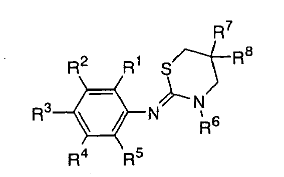 | | | | | | | | |

| | R¹ | R² | R³ | R⁴ | R⁵ | R⁶ | R⁷ | R⁸ |
|---|---|---|---|---|---|---|---|---|
| I-262 | NMe₂ | H | OMe | H | H | CSSMe | Me | Me |
| I-263 | NMe₂ | H | H | OMe | H | CSSMe | Me | Me |
| I-264 | Me | NEt₂ | H | H | H | CSSMe | Me | Me |
| I-265 | H | NEt₂ | Me | H | H | CSSMe | Me | Me |
| I-266 | H | NEt₂ | OMe | H | H | CSSMe | Me | Me |
| I-267 | Bu^{*s*} | H | H | H | H | CSSMe | Et | Et |
| I-268 | Pr^{*i*} | H | H | H | H | CSSMe | Pr | Pr |
| I-269 | Pr^{*i*} | H | H | H | H | CSSMe | -(CH₂)₄- | |
| I-270 | Pr^{*i*} | H | H | H | H | CSSMe | -(CH₂)₅- | |

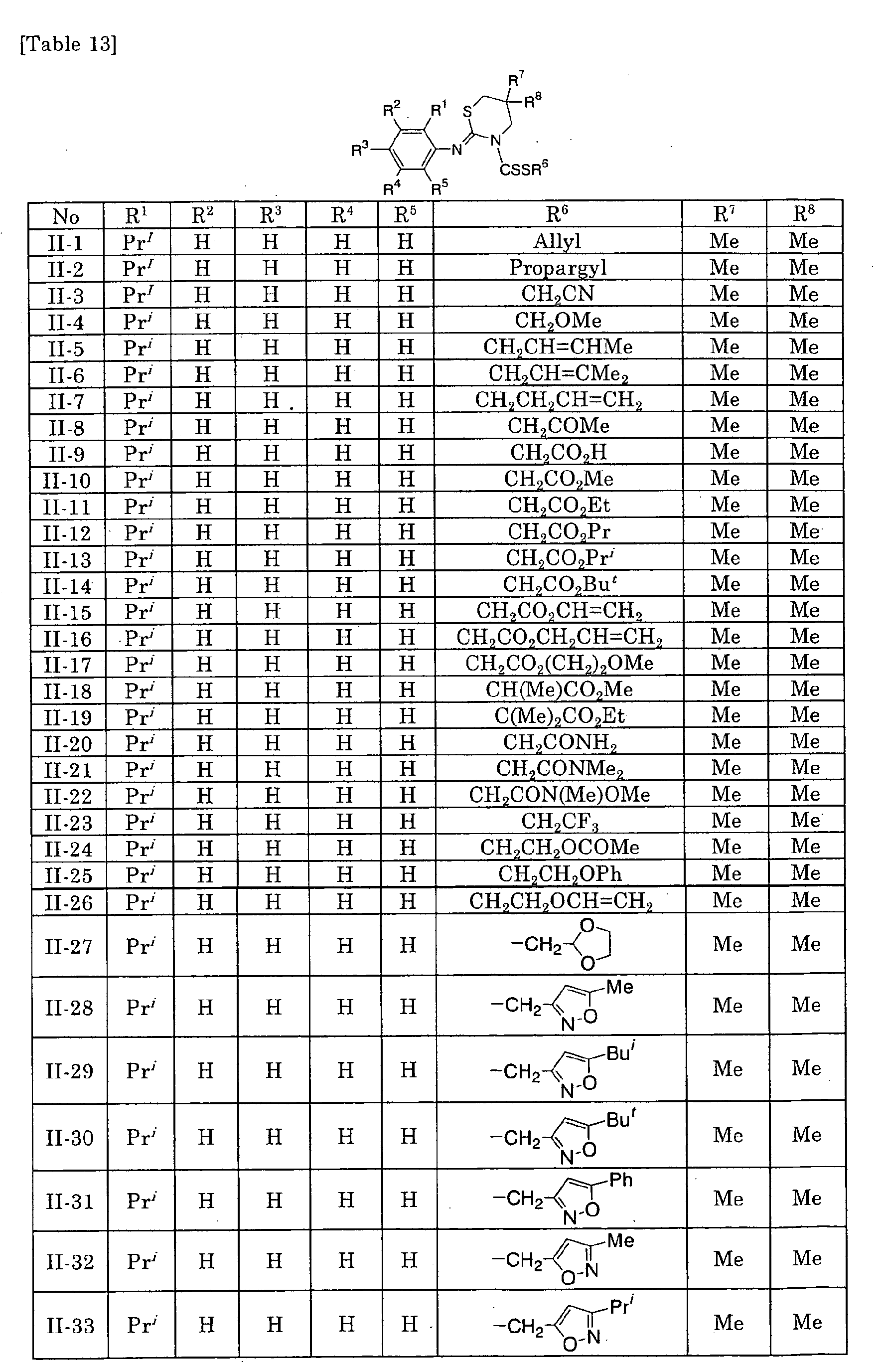

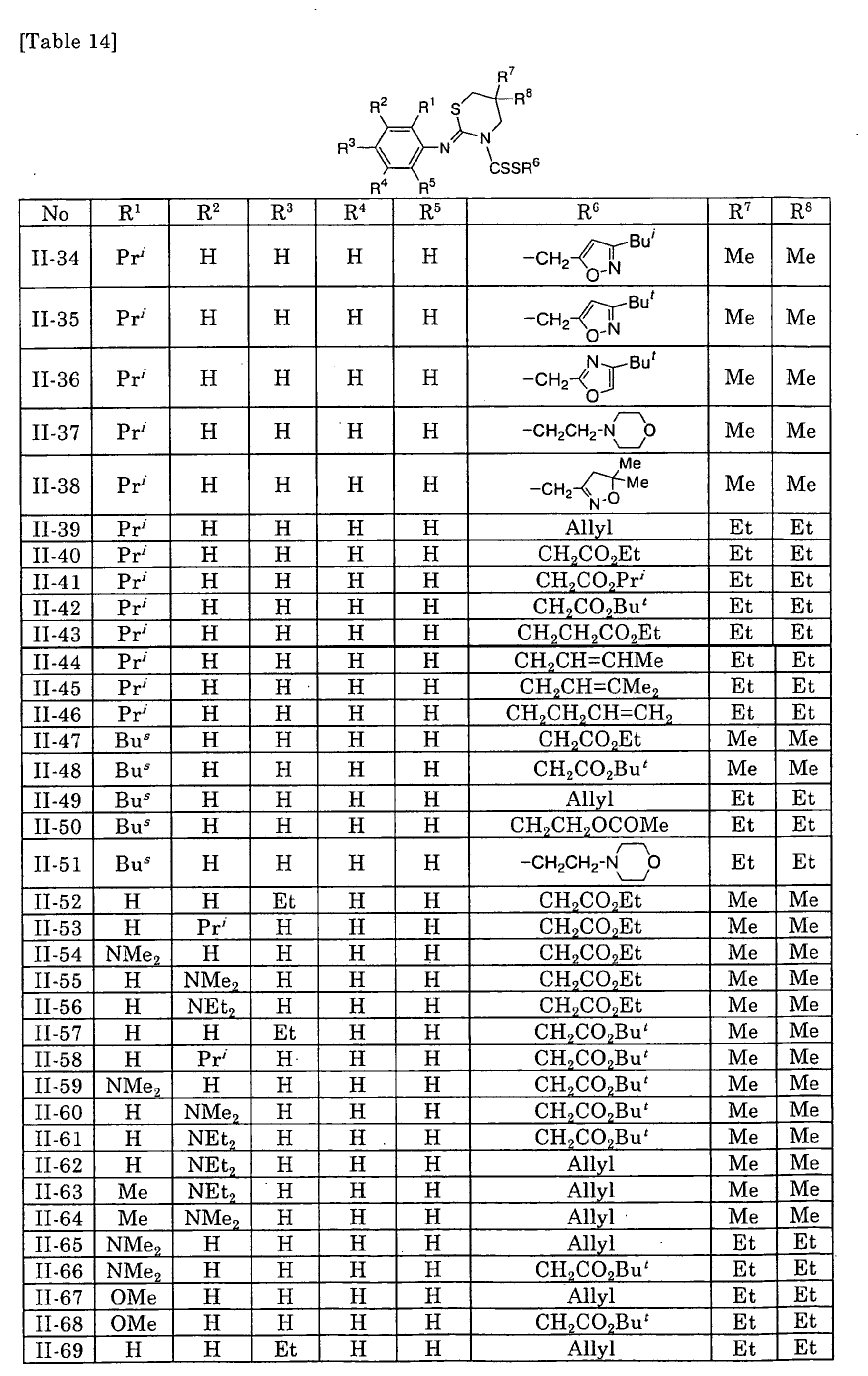

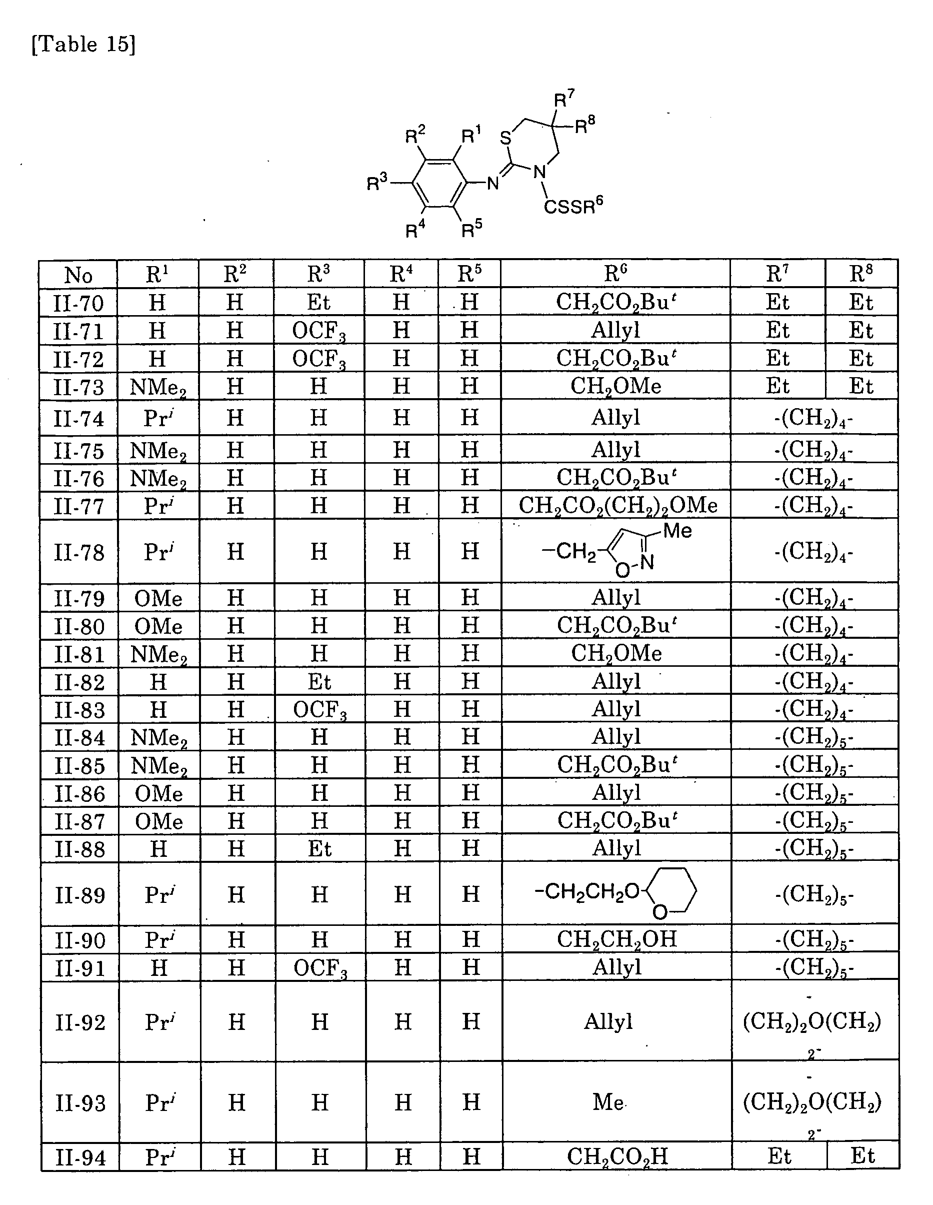

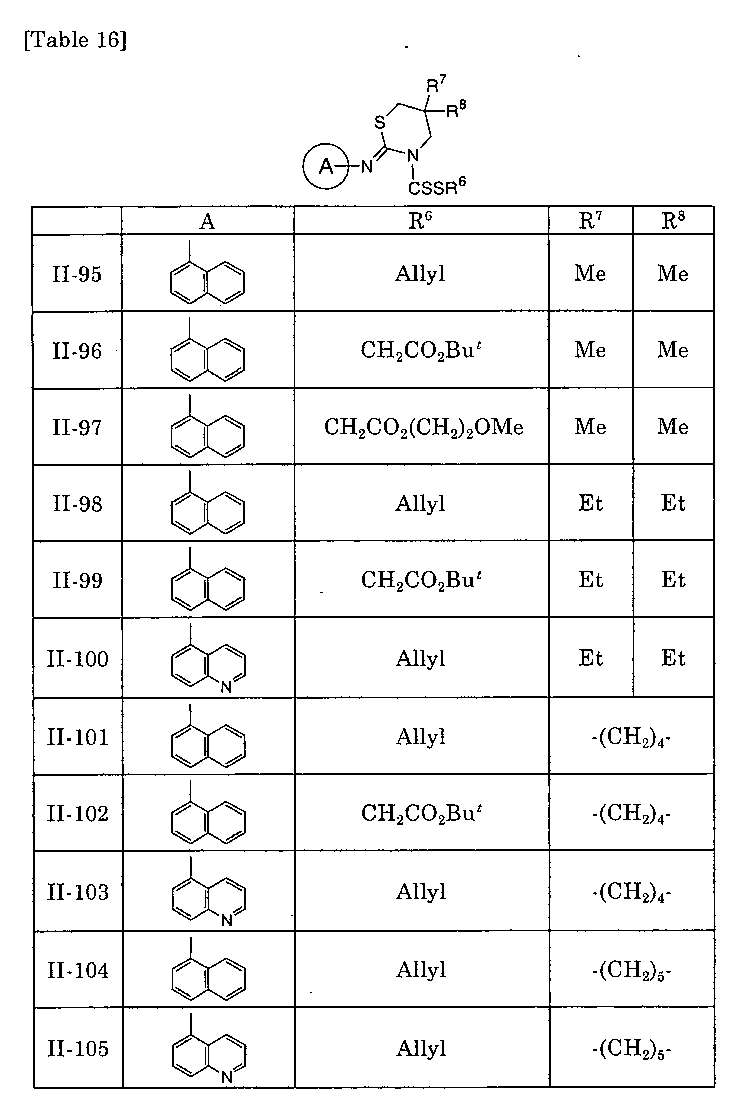

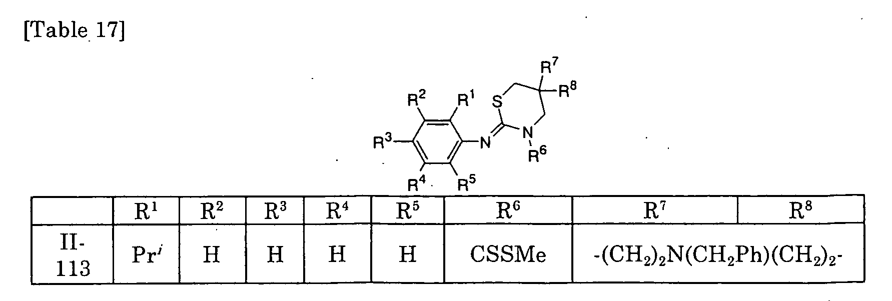

**[Table 18]**

| | | | | | | | | |
|---|---|---|---|---|---|---|---|---|
| 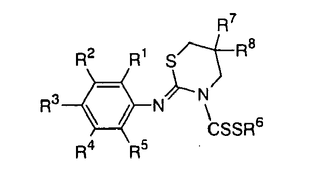 | | | | | | | | |

| | R¹ | R² | R³ | R⁴ | R⁵ | R⁶ | R⁷ | R⁸ |
|---|---|---|---|---|---|---|---|---|
| II-114 | H | SMe | H | H | H | Allyl | Et | Et |
| II-115 | H | SMe | H | H | H | Allyl | -(CH₂)₄- | |
| II-116 | H | SMe | H | H | H | Allyl | -(CH₂)₅- | |
| II-117 | H | H | SMe | H | H | Allyl | -(CH₂)₄- | |
| II-118 | H | H | SMe | H | H | Allyl | -(CH₂)₅- | |
| II-119 | OMe | H | Et | H | H | Allyl | Me | Me |
| II-120 | OMe | H | Pr^{*i*} | H | H | Allyl | Me | Me |
| II-121 | Pr^{*i*} | H | OMe | H | H | Allyl | Me | Me |
| II-122 | Pr^{*i*} | H | OEt | H | H | Allyl | Me | Me |
| II-123 | H | OEt | OEt | H | H | Allyl | Me | Me |
| II-124 | H | OPr | OPr | H | H | Allyl | Me | Me |
| II-125 | H | OMs | OEt | H | H | Allyl | Me | Me |
| II-126 | H | H | (CH₂)₂OEt | H | H | Allyl | Me | Me |
| II-127 | H | OMe | OEt | H | H | Allyl | Et | Et |
| II-128 | H | OEt | OEt | H | H | Allyl | Et | Et |
| II-129 | H | OEt | OPr | H | H | Allyl | Et | Et |
| II-130 | H | OMs | OPr | H | H | Allyl | Et | Et |
| II-131 | H | OPr | OPr | H | H | Allyl | Et | Et |
| II-132 | H | OPr^{*i*} | OPr | H | H | Allyl | Et | Et |
| II-133 | H | H | (CH₂)₂NMe₂ | H | H | Allyl | Me | Me |
| II-134 | Pr^{*i*} | H | H | H | H | CH₂CO₂ Bu^{*t*} | -(CH₂)₅- | |
| II-135 | Pr^{*i*} | H | H | H | H | Me | -(CH₂)₂N(Me)(CH₂)₂- | |
| II-136 | Pr^{*i*} | H | H | H | H | Me | -(CH₂)₂N(Et)(CH₂)₂- | |
| II-137 | F | H | F | H | H | Allyl | Me | Me |
| II-138 | H | Cl | Cl | H | H | Allyl | Me | Me |
| II-139 | Me | H | Cl | H | H | Allyl | Me | Me |
| II-140 | Cl | H | Me | H | H | Allyl | Me | Me |
| II-141 | H | H | (CH₂)₂OMe | H | H | Allyl | Me | Me |
| II-142 | H | H | Pr^{*i*} | H | H | Allyl | -(CH₂)₄- | |
| II-143 | H | H | Pr^{*i*} | H | H | CH₂CO₂ Bu^{*t*} | -(CH₂)₄- | |
| II-144 | H | H | Pr^{*i*} | H | H | Allyl | Et | Et |
| II-145 | H | H | Pr^{*i*} | H | H | CH₂CO₂ Bu^{*t*} | Et | Et |
| II-146 | H | H | Pr^{*i*} | H | H | Allyl | -(CH₂)₅- | |
| II-147 | OMe | H | H | H | H | CH₂CO₂ Bu^{*t*} | Pr | Pr |
| II-148 | OMe | H | H | H | H | CH₂CO₂ Bu^{*t*} | Pr^{*i*} | Pr^{*i*} |
| II-149 | OMe | H | H | H | H | Allyl | Pr | Pr |
| II-150 | Bu^{*s*} | H | H | H | H | Me | -(CH₂)₂N(Me)(CH₂)₂- | |

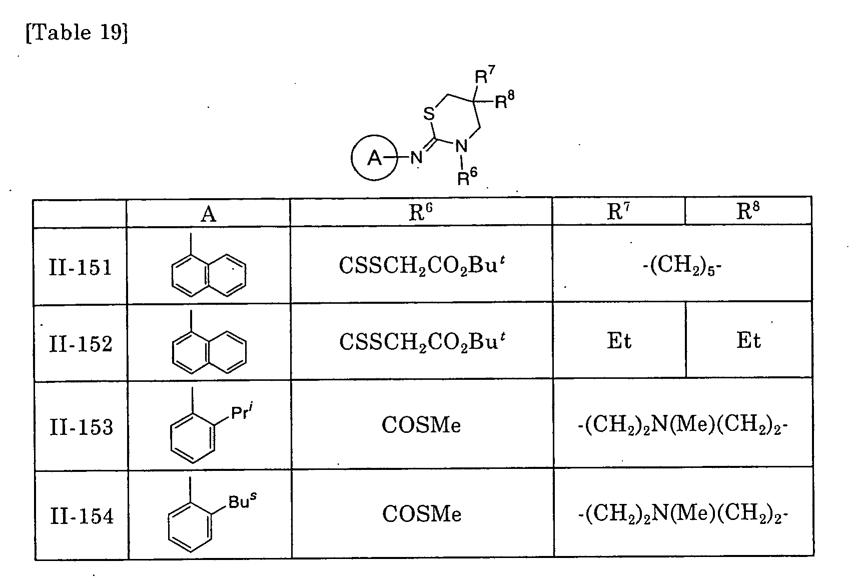

The compounds described in WO 02/053543 are exemplified as the compound represented by the formula (II). Preferable are the compounds described in the following Tables.

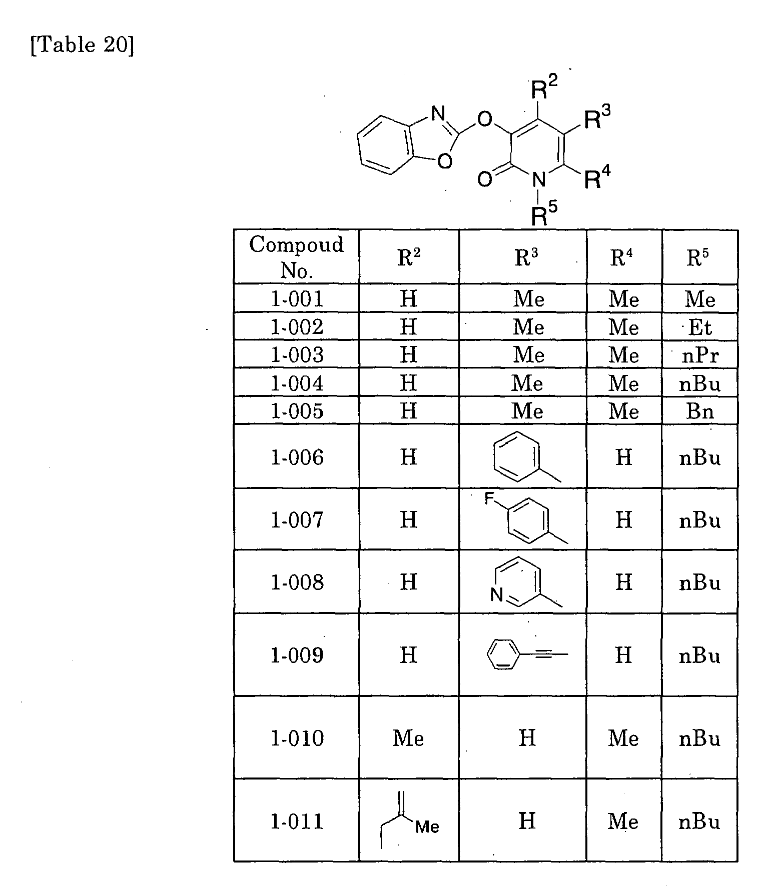

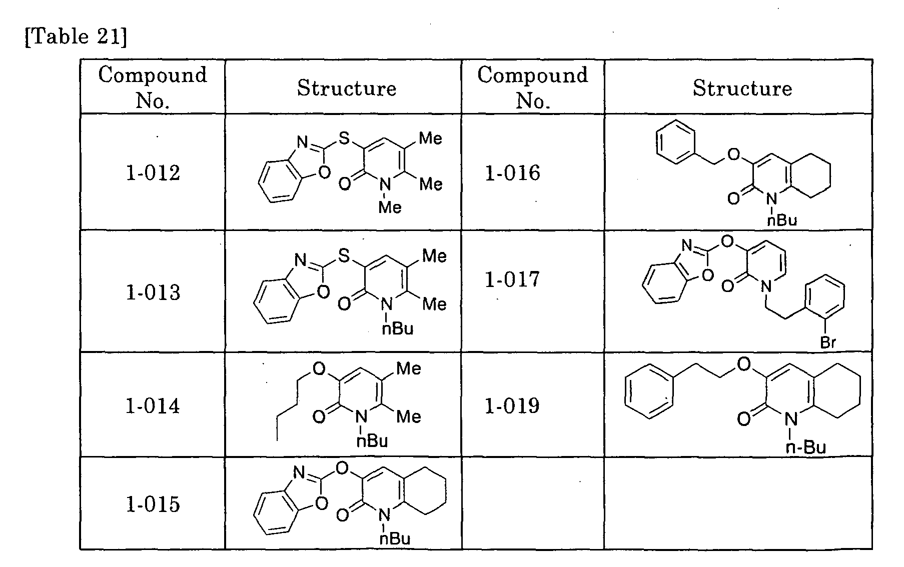

**[Table 22]**

| | | | | | |
|---|---|---|---|---|---|
| 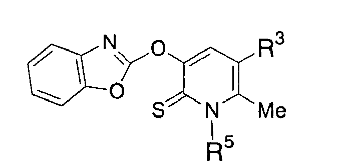 | | | | | |

| Compound No. | R³ | R⁵ | Compound No. | R³ | R⁵ |
|---|---|---|---|---|---|
| 2-001 | Me | Me | 2-008 | Me | Bn |
| 2-002 | Me | Et | 2-009 | Et | Me |
| 2-003 | Me | nPr | 2-010 | Et | Et |
| 2-004 | Me | nBu | 2-011 | Et | nPr |
| 2-005 | Me | iBu | 2-012 | Et | nBu |
| 2-006 | Me | nPent | 2-013 | Et | Bn |
| 2-007 | Me | nHexyl | | | |

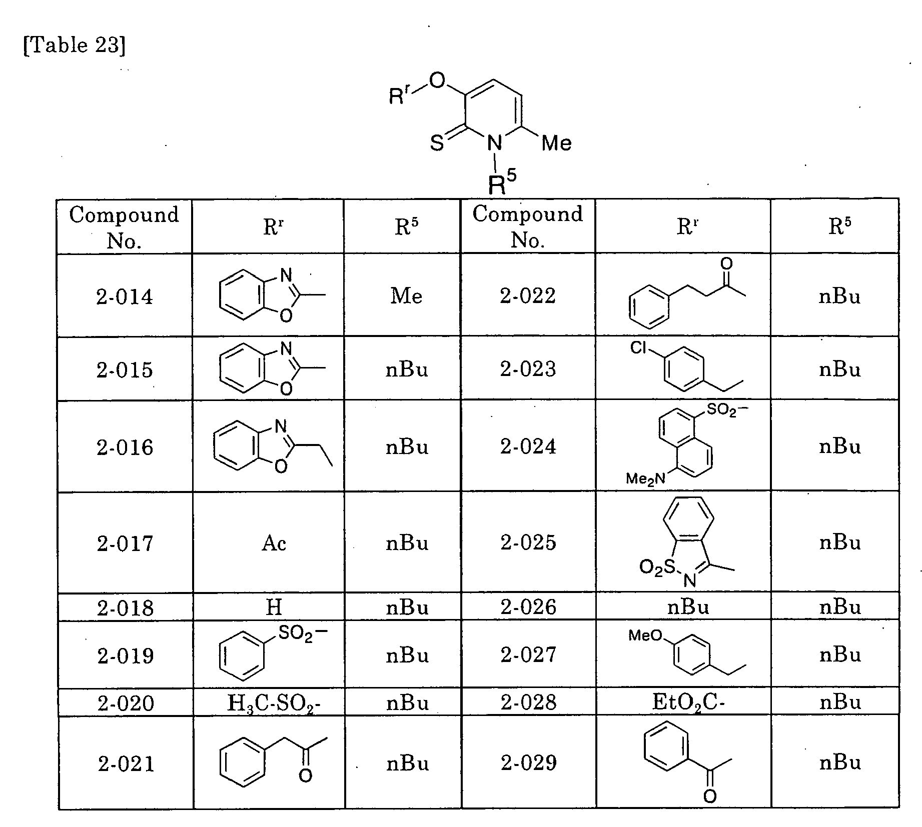

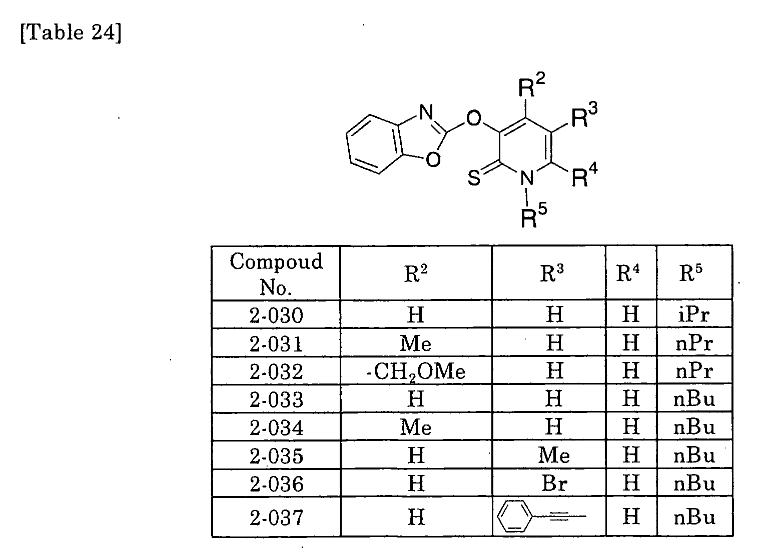

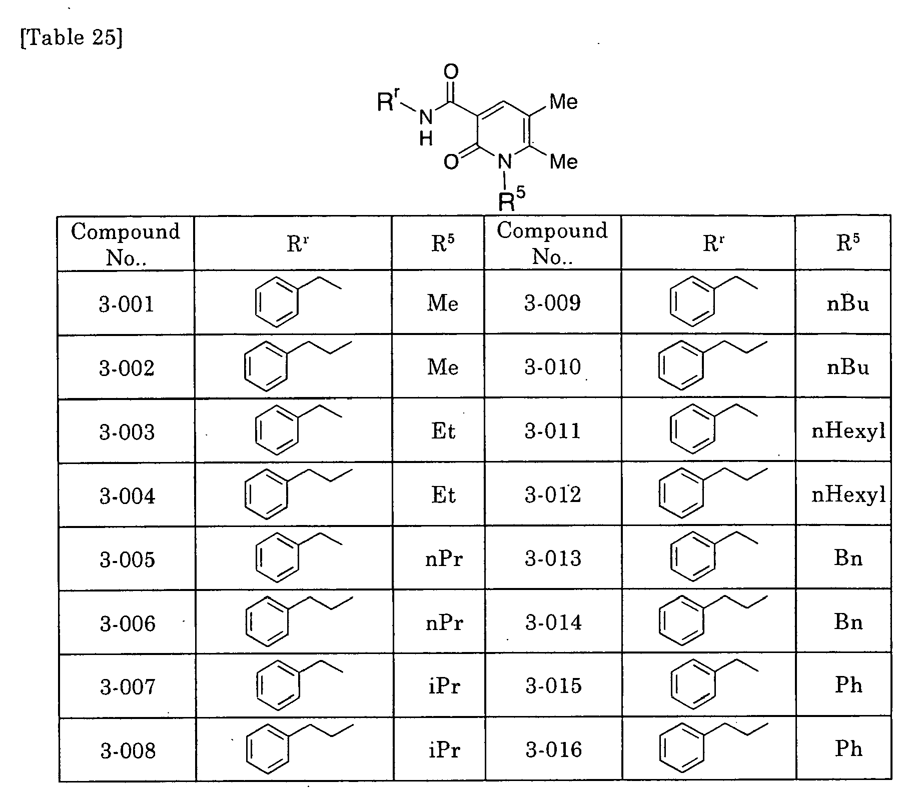

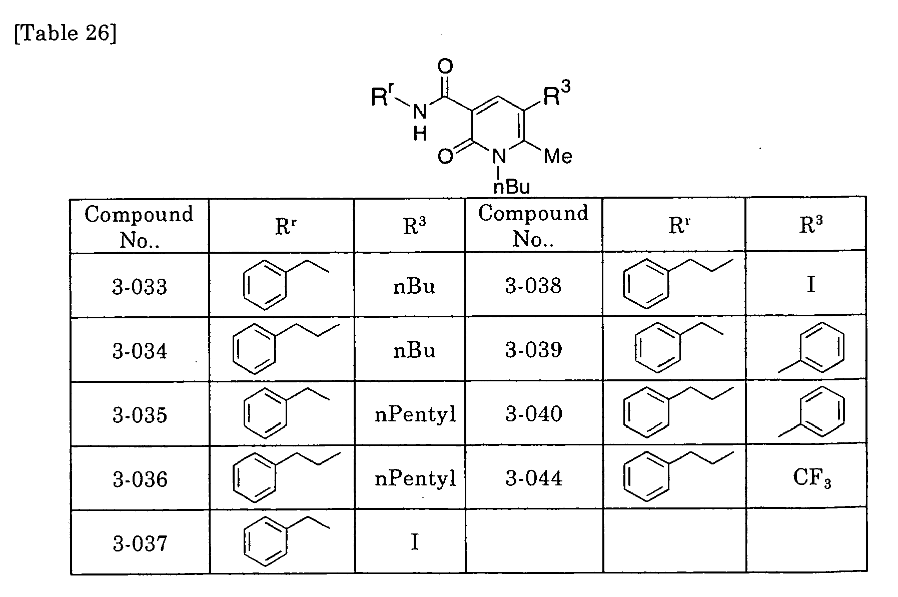

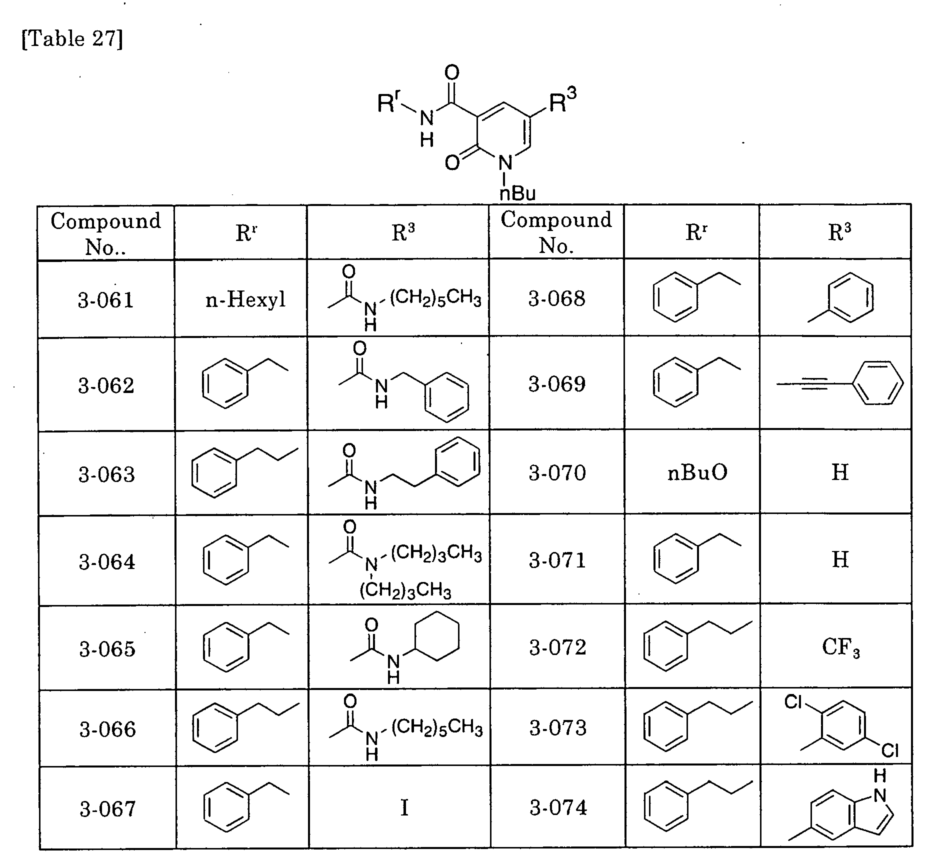

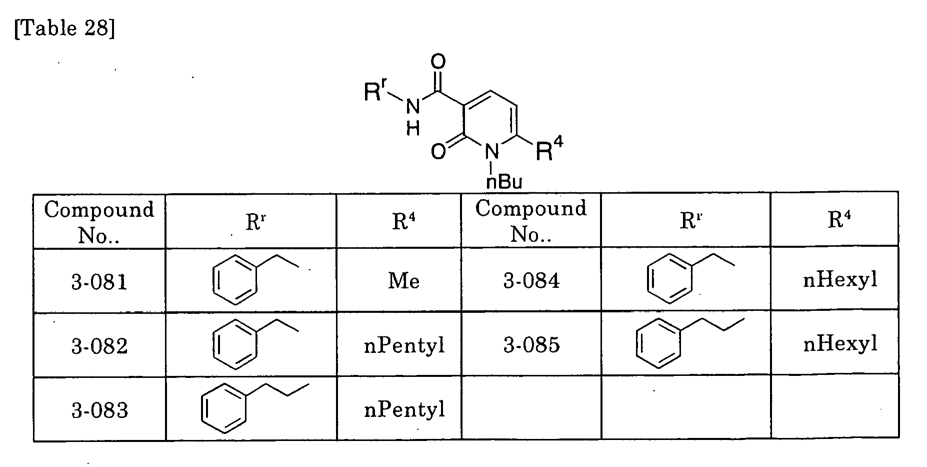

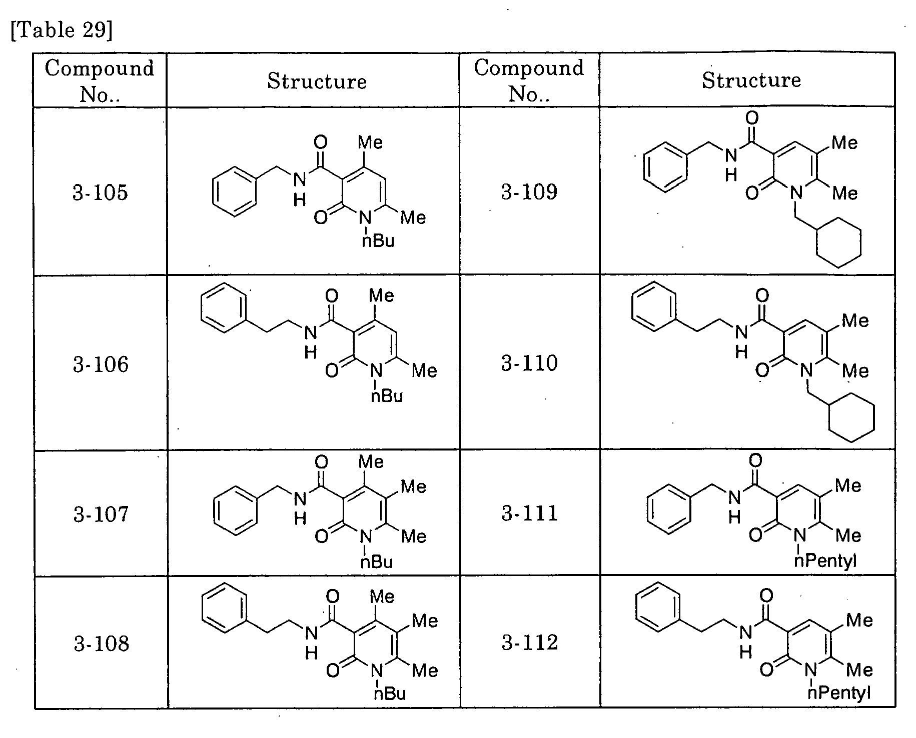

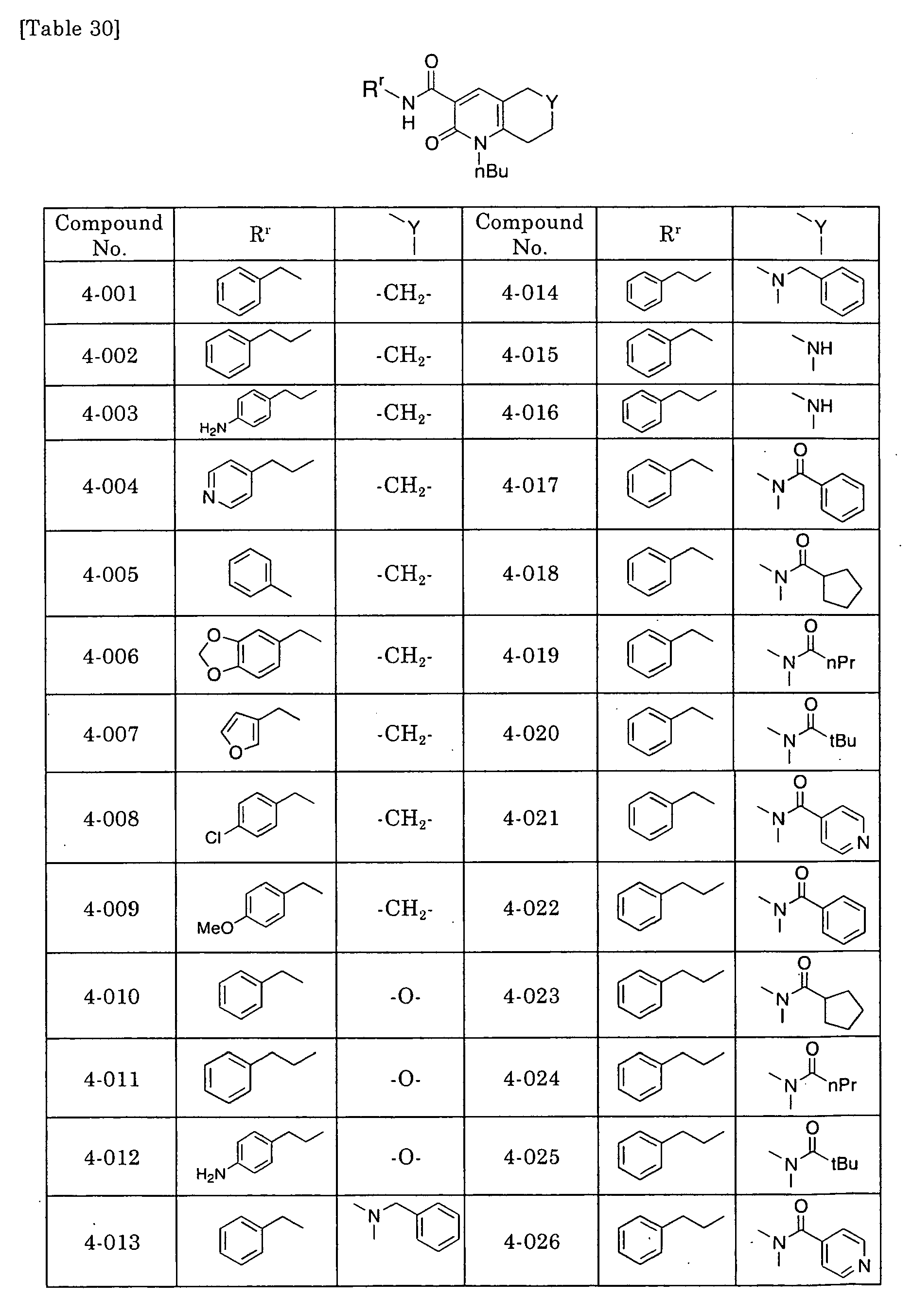

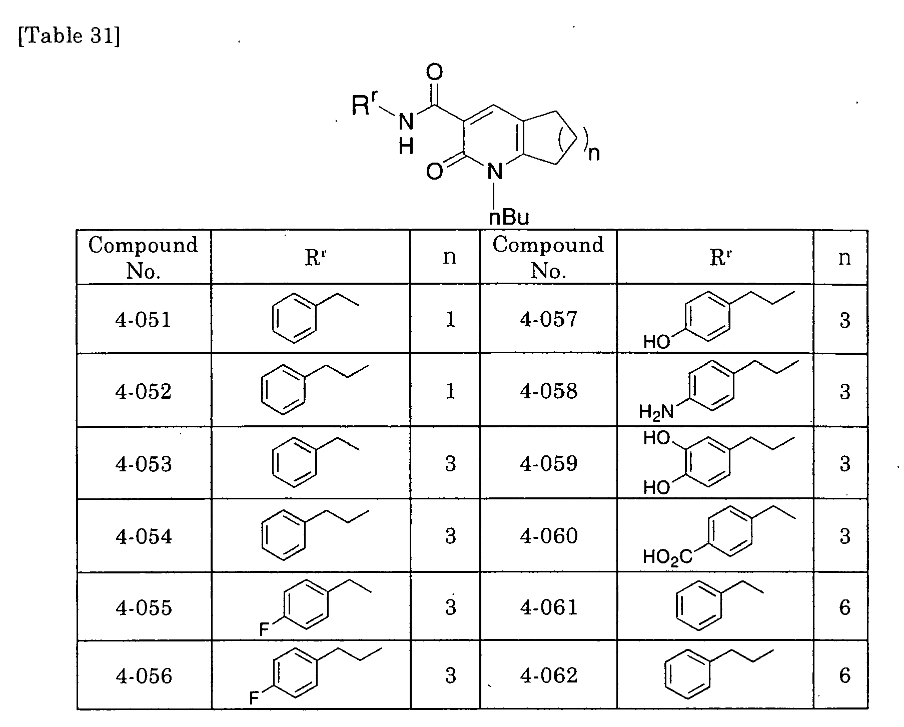

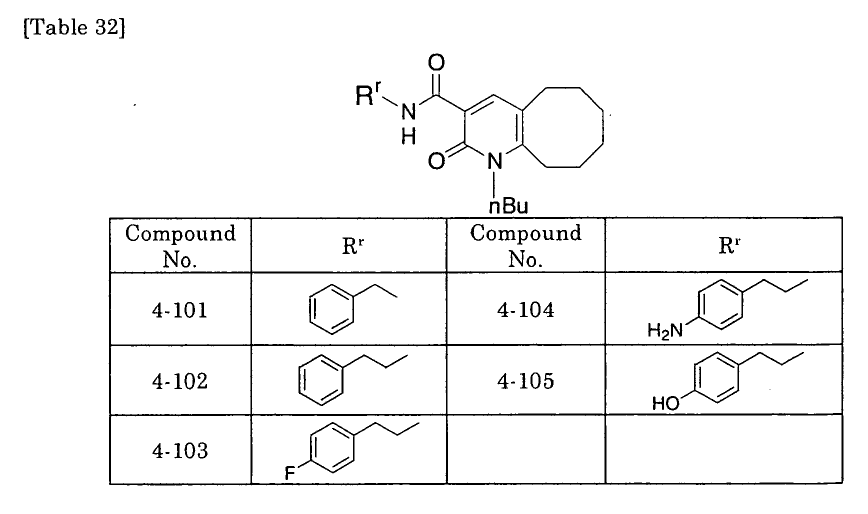

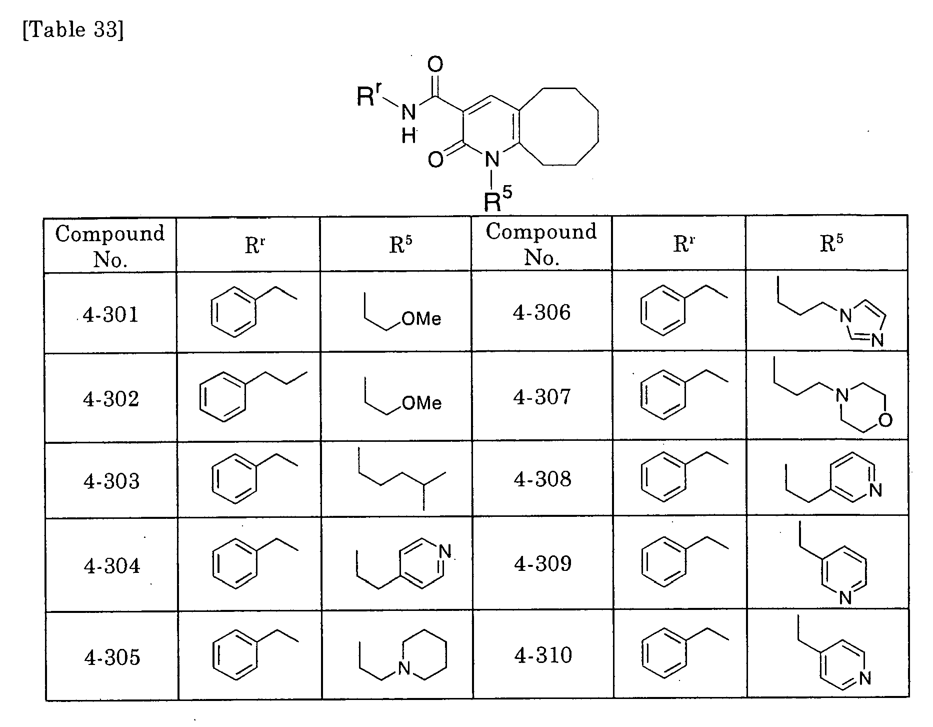

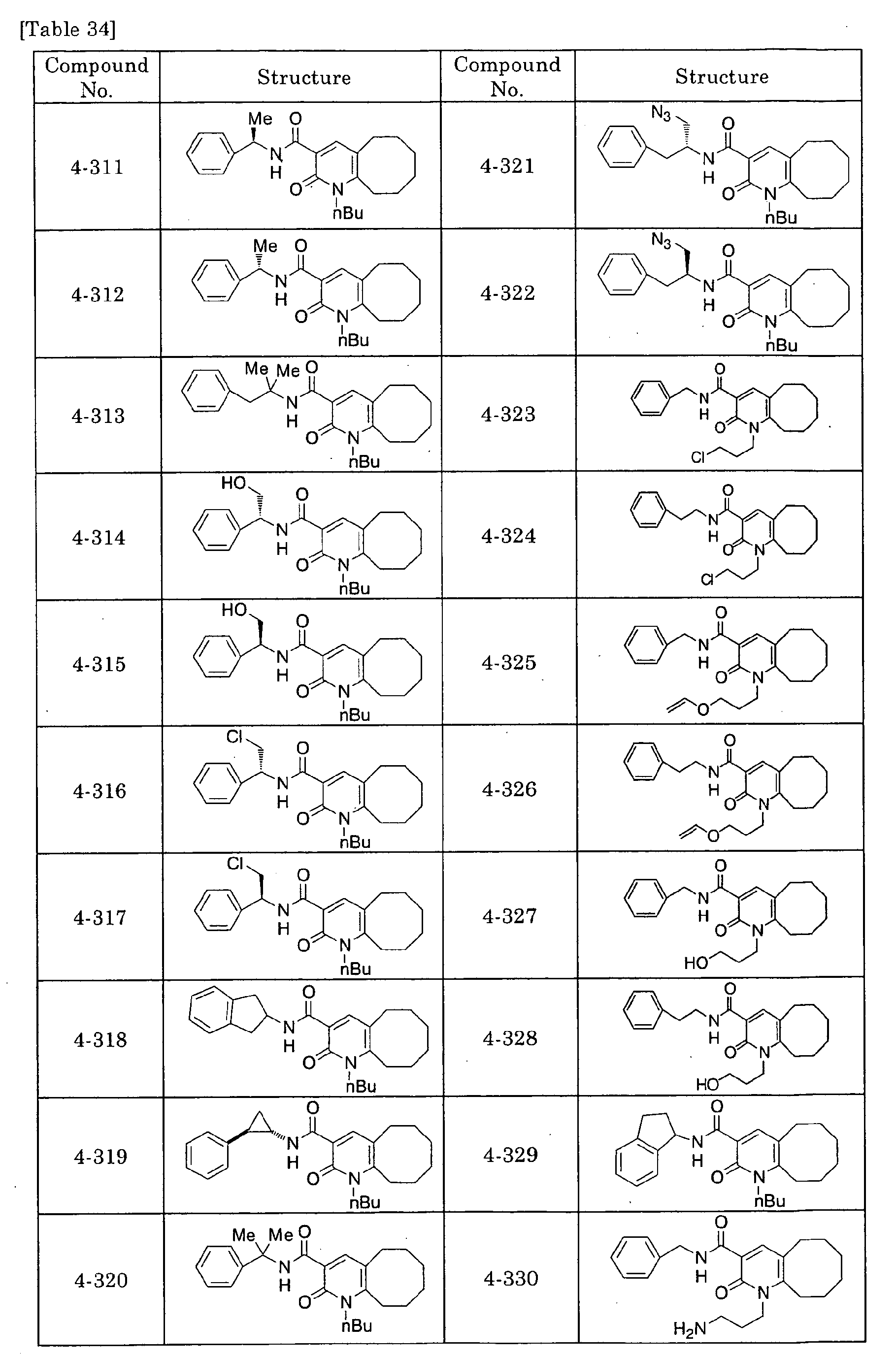

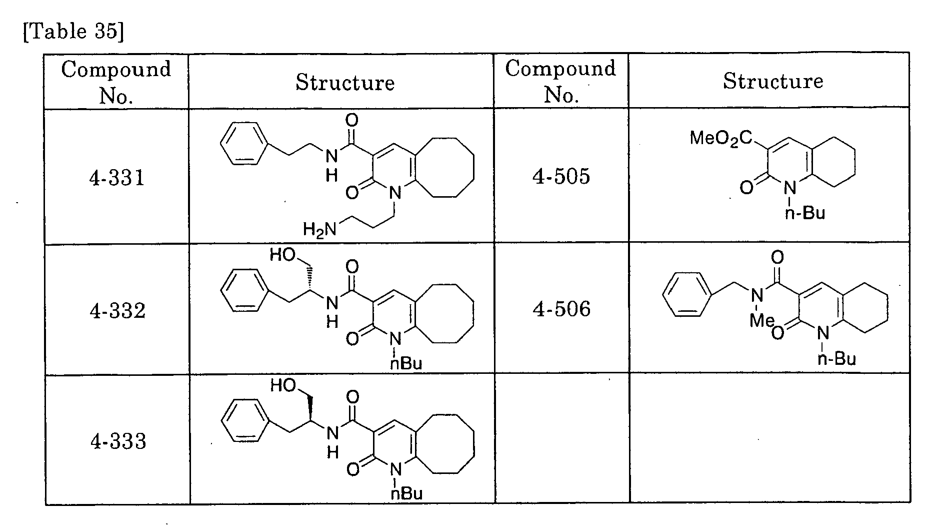

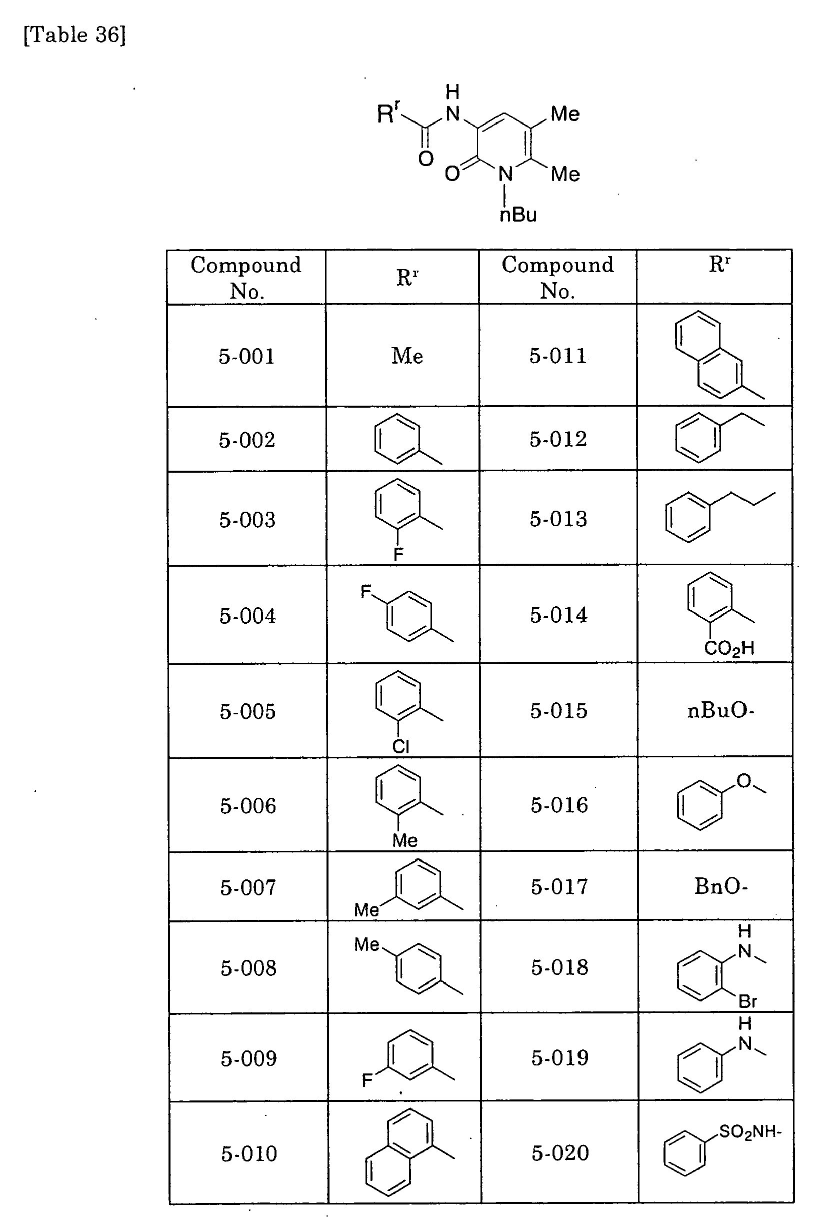

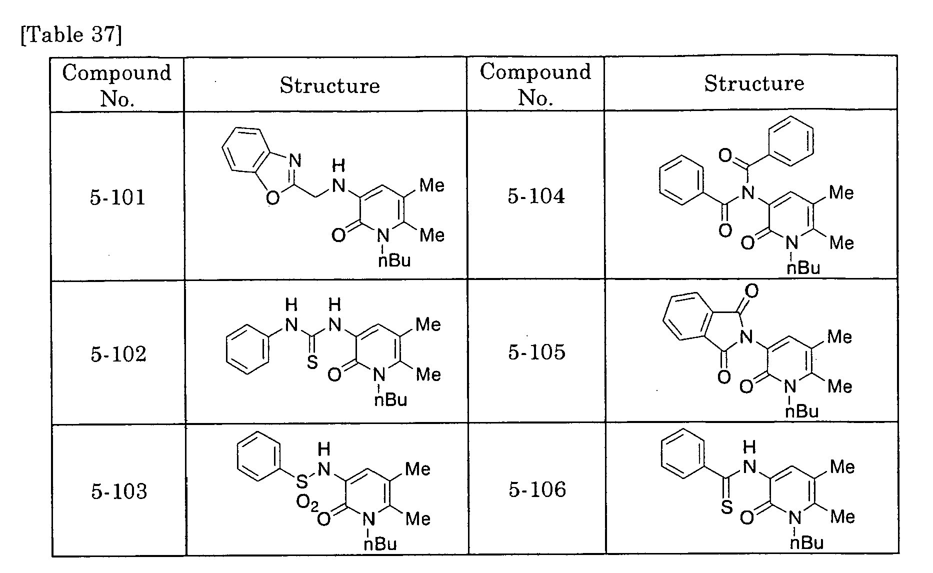

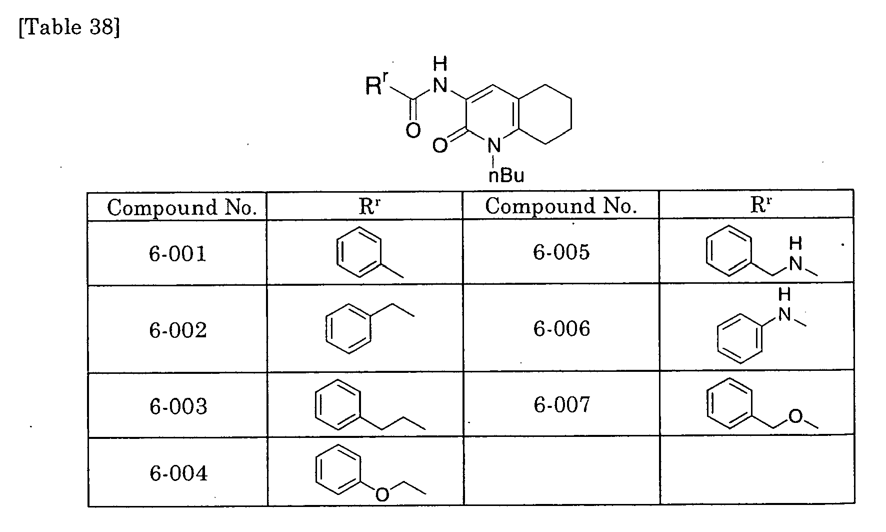

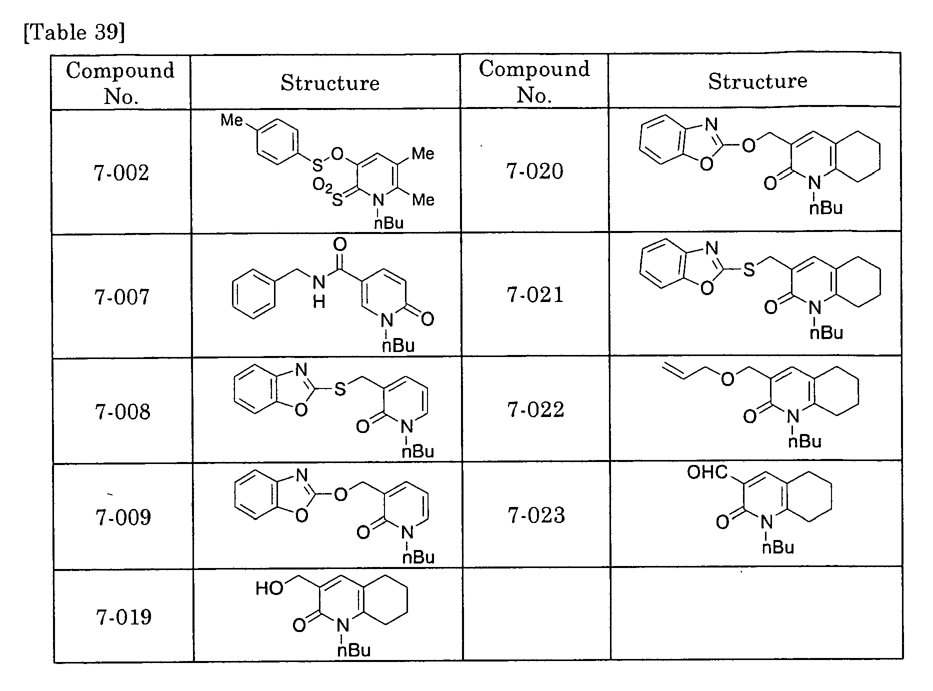

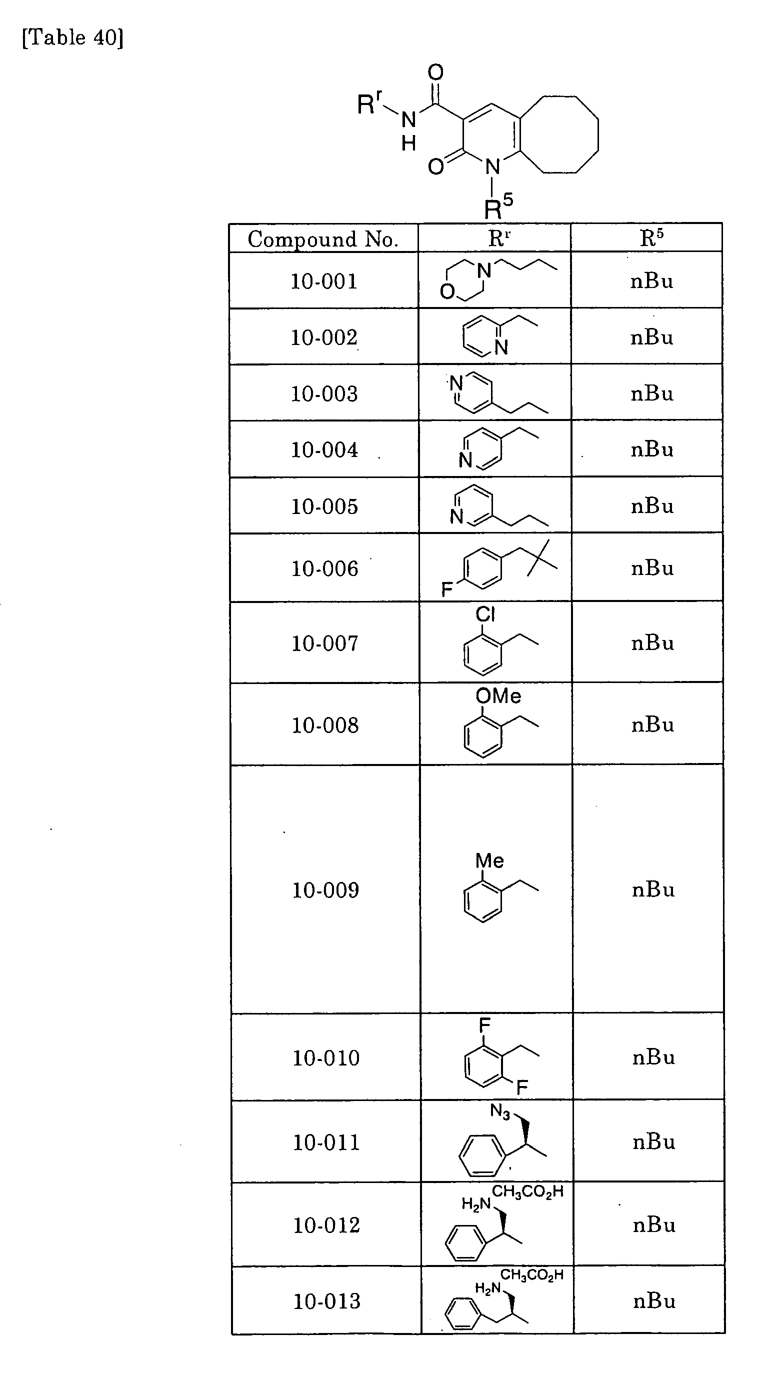

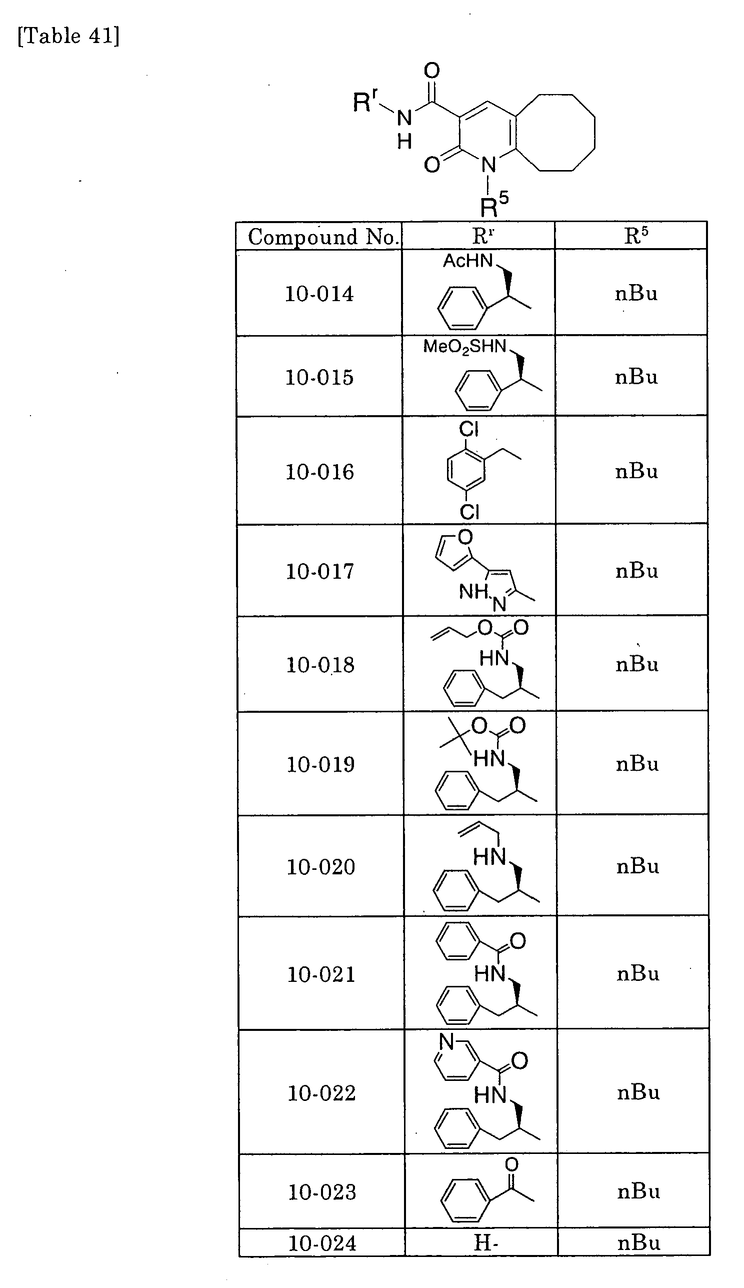

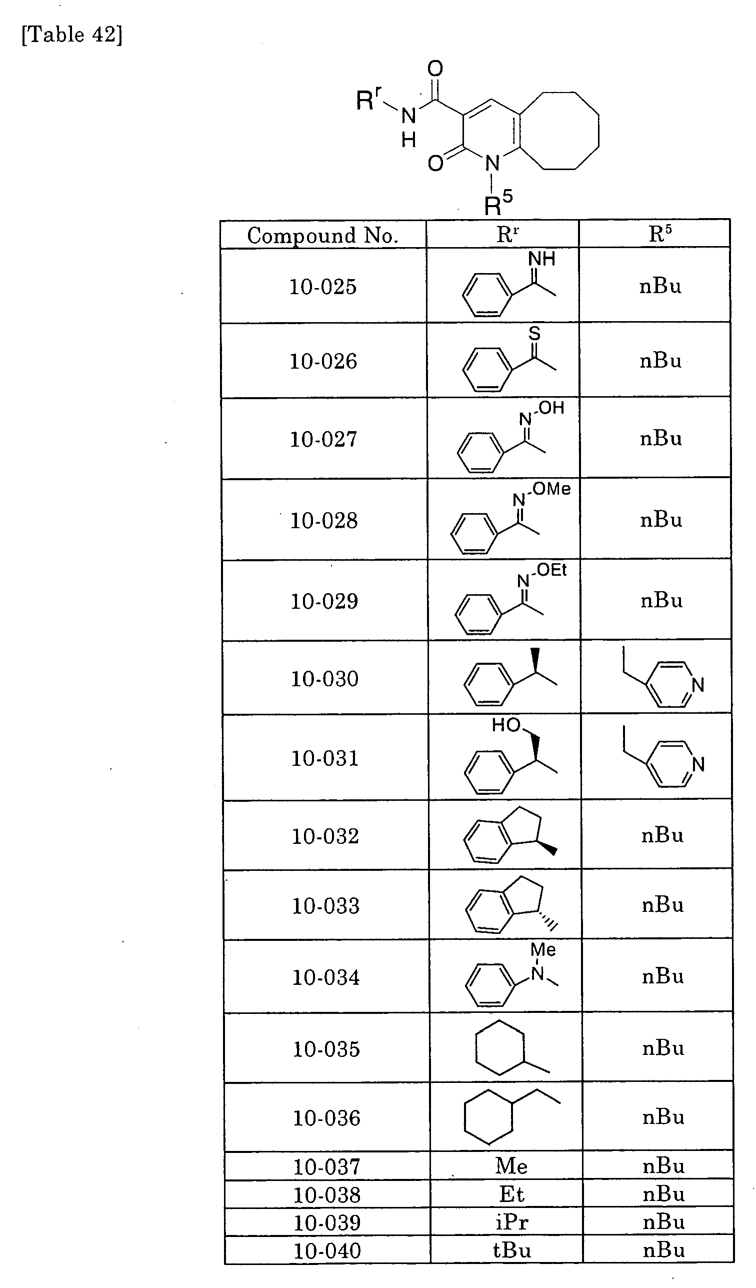

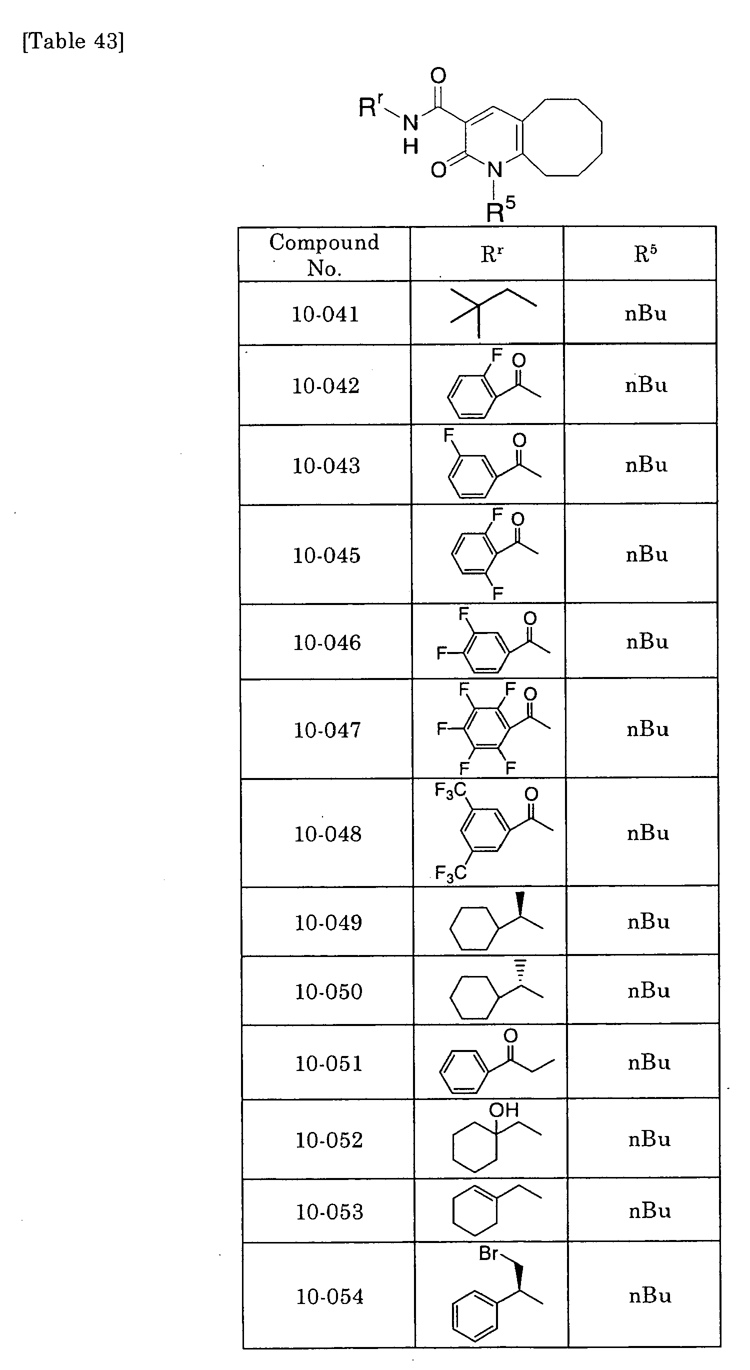

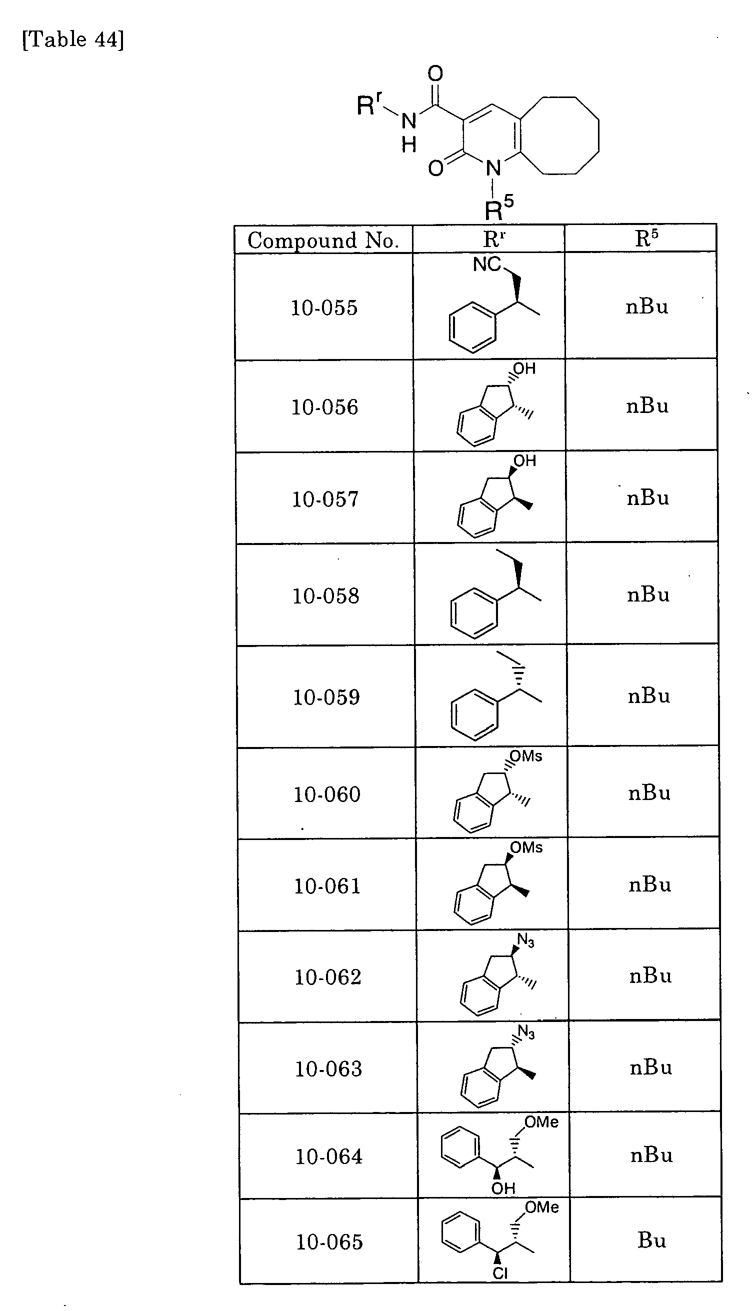

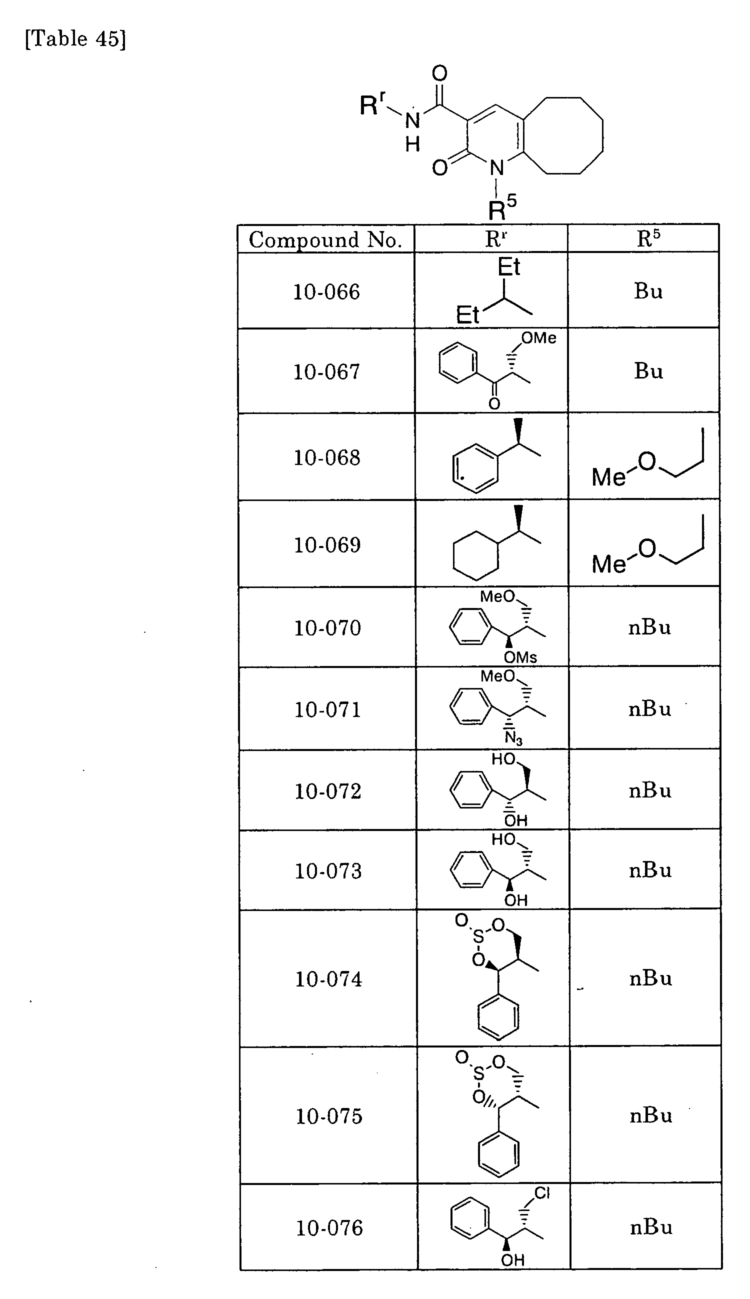

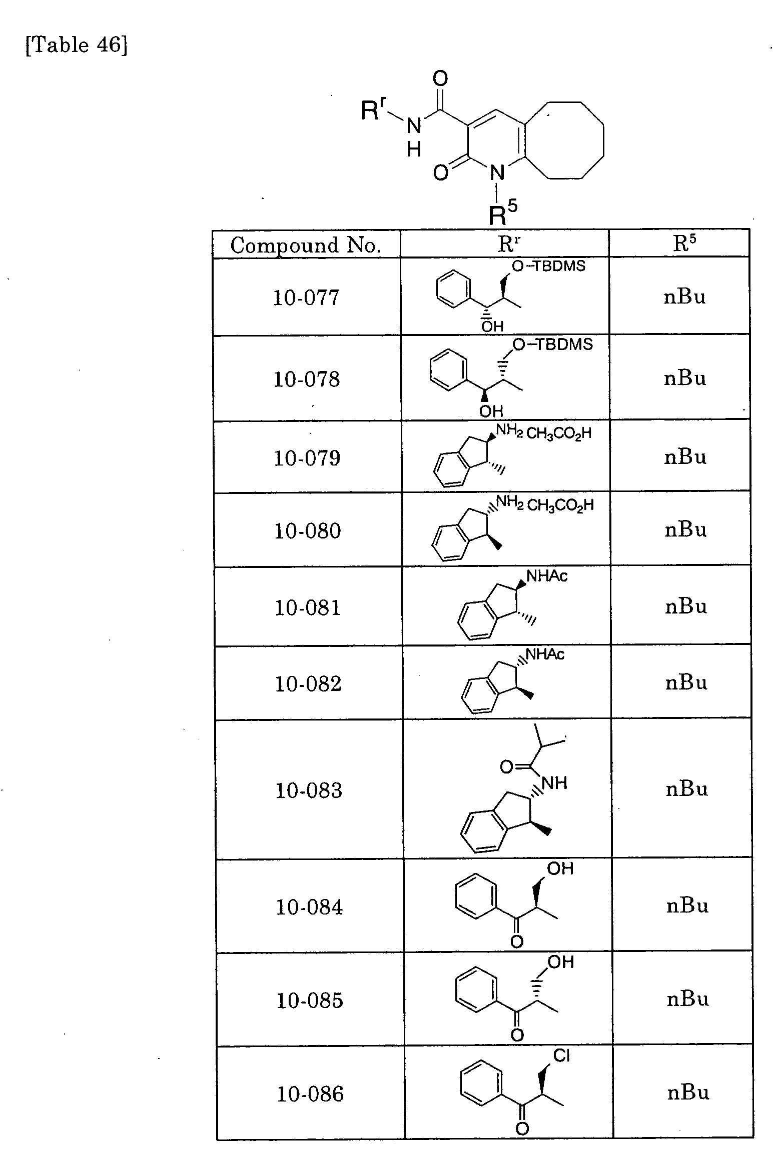

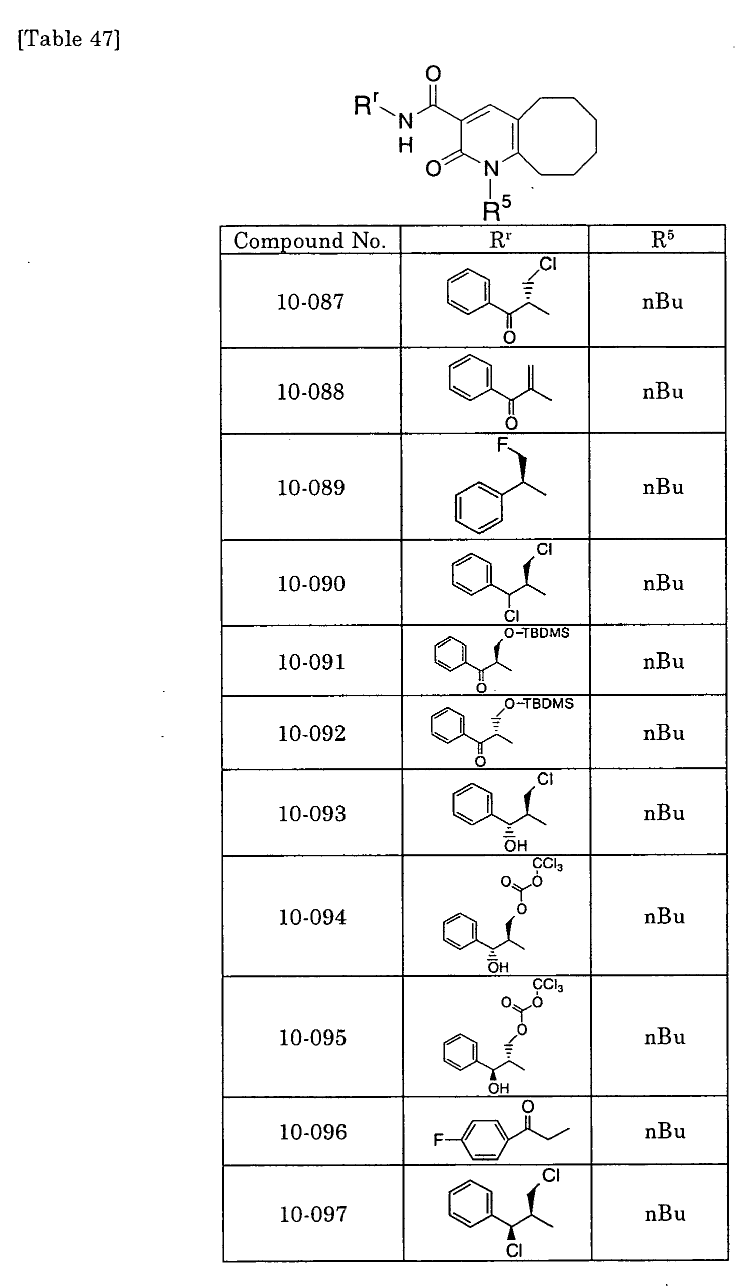

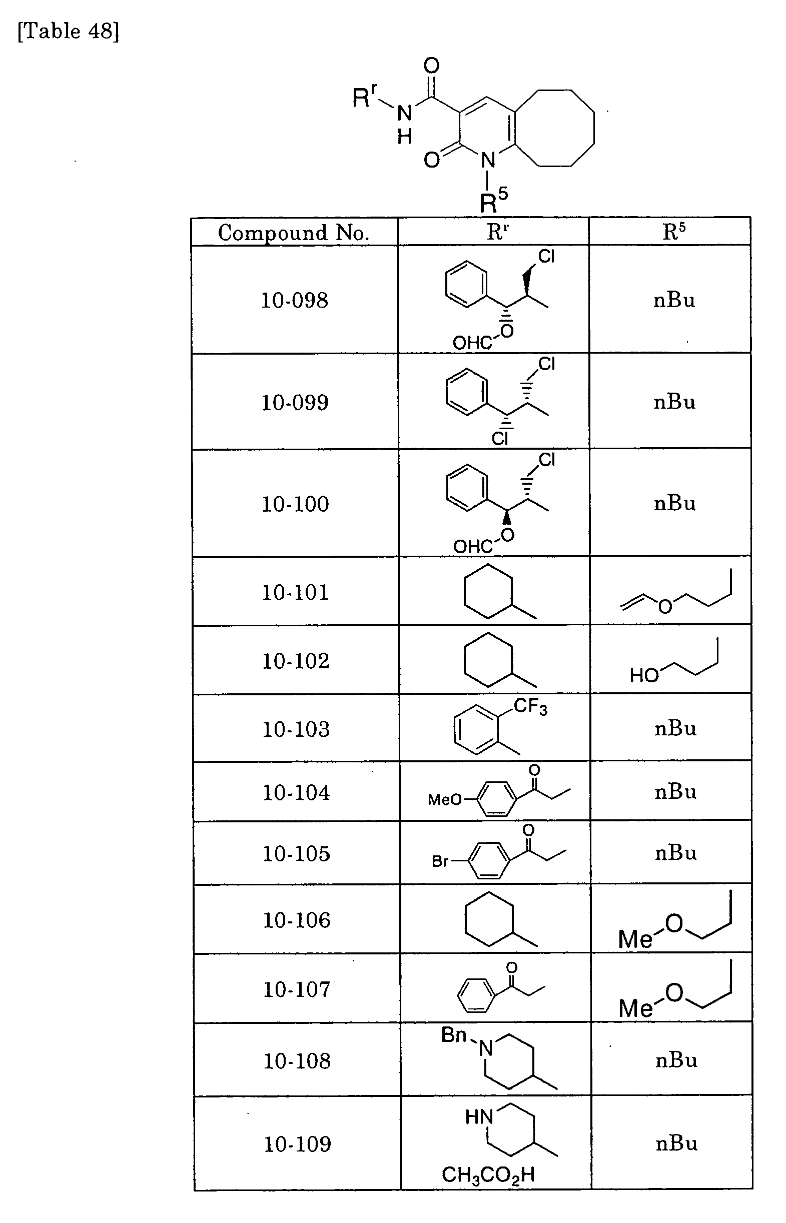

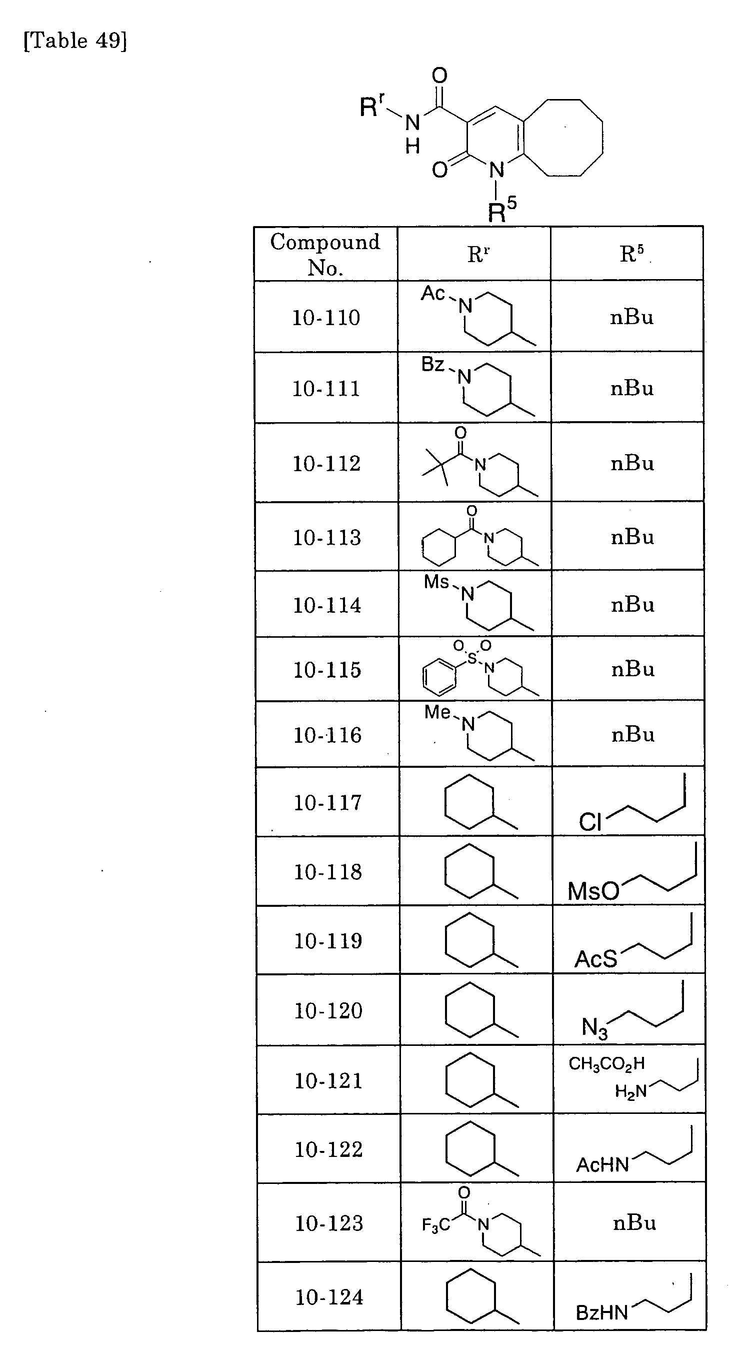

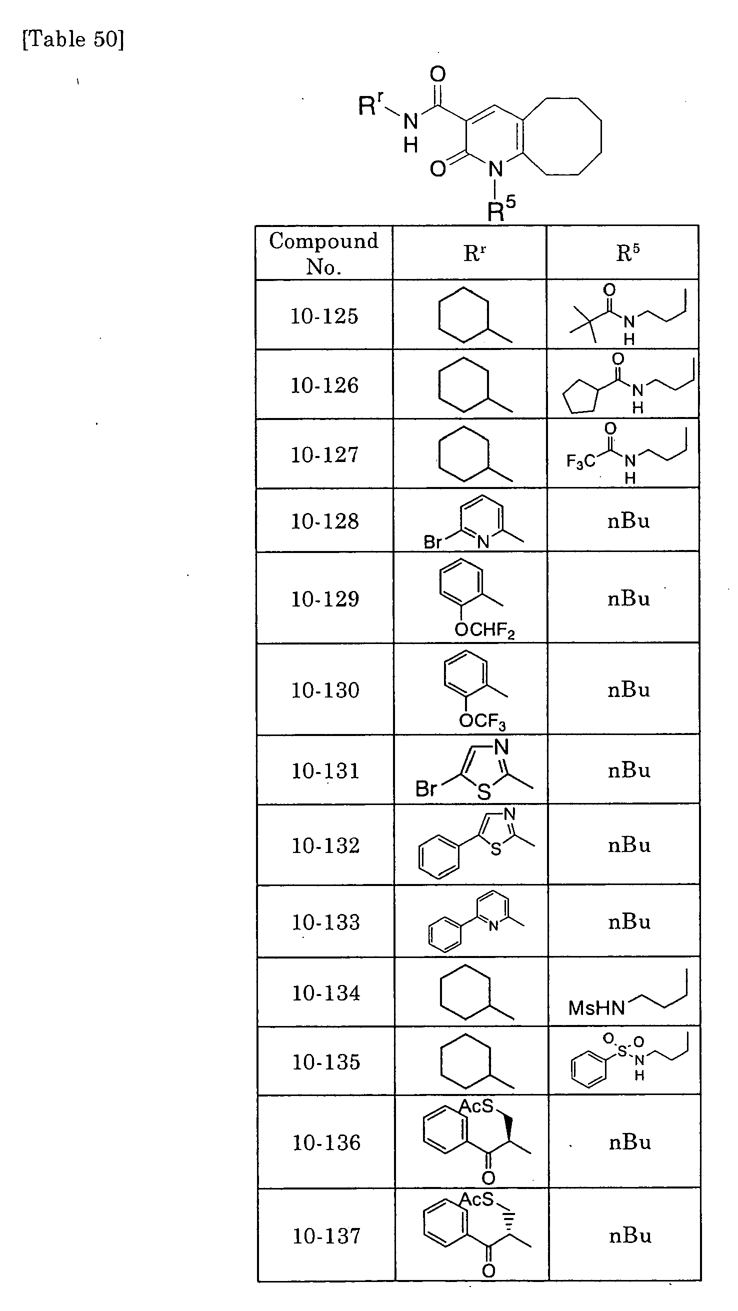

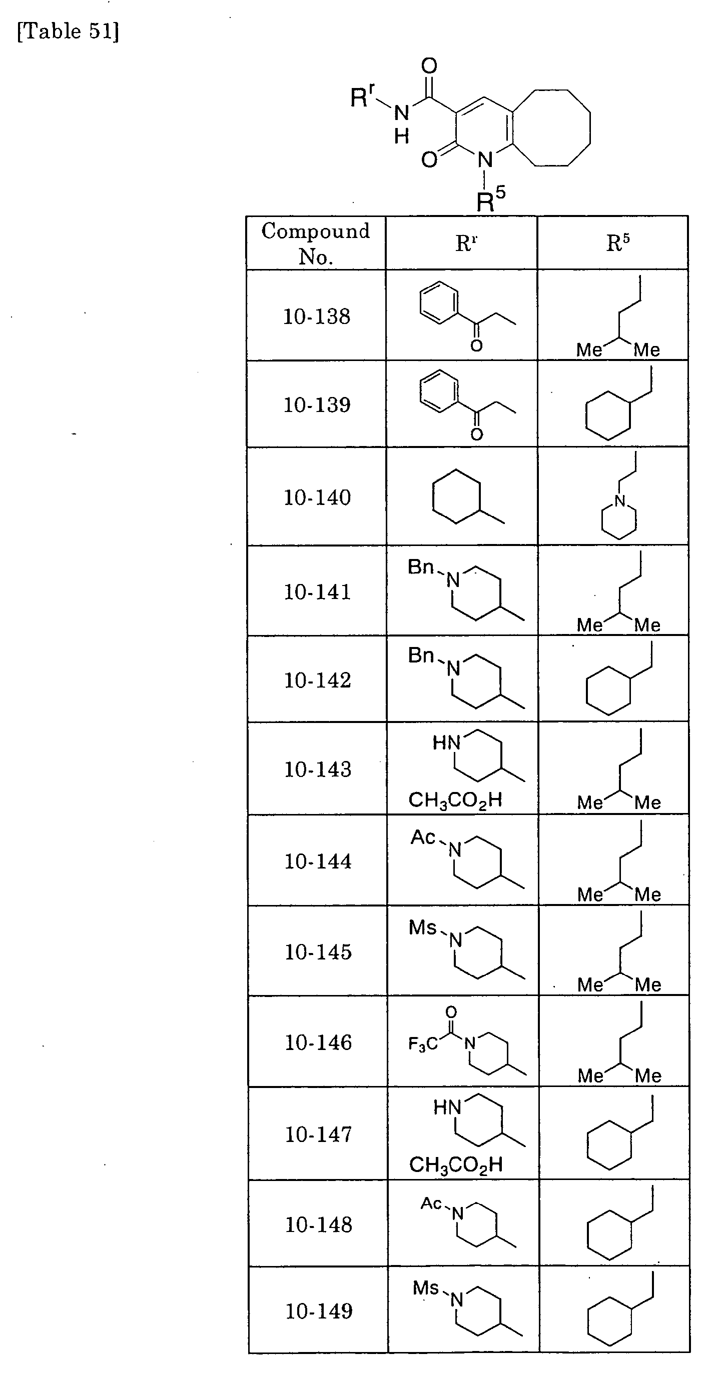

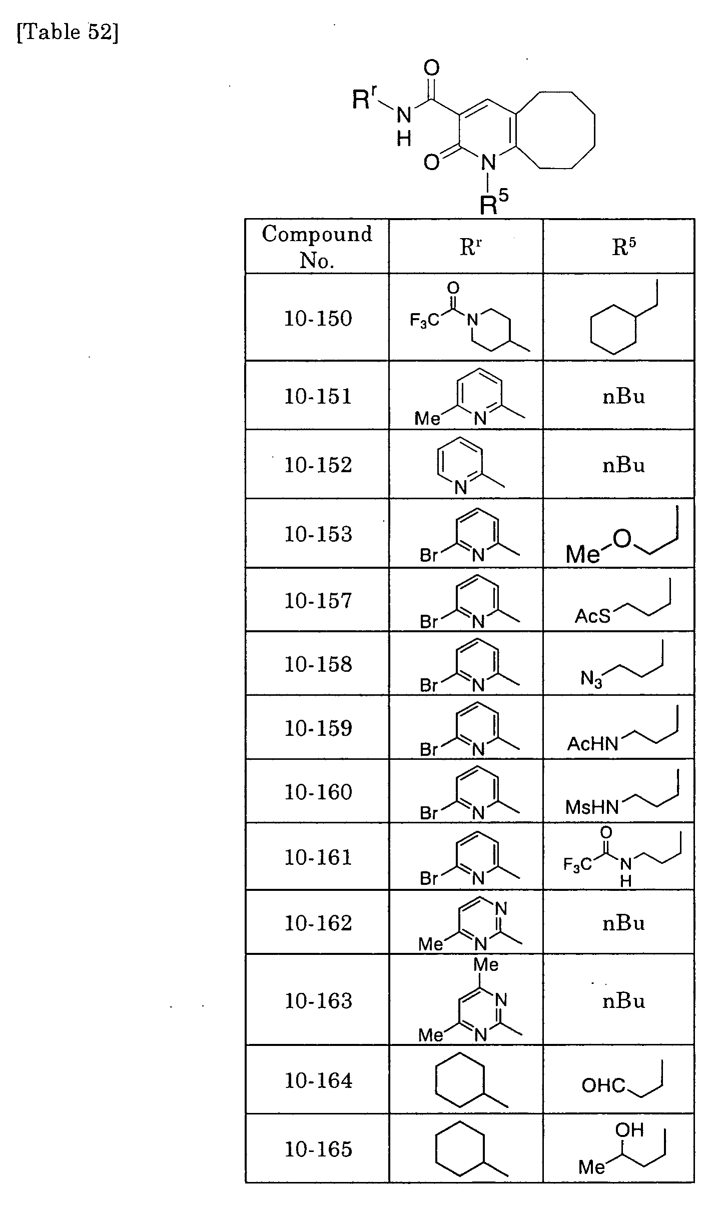

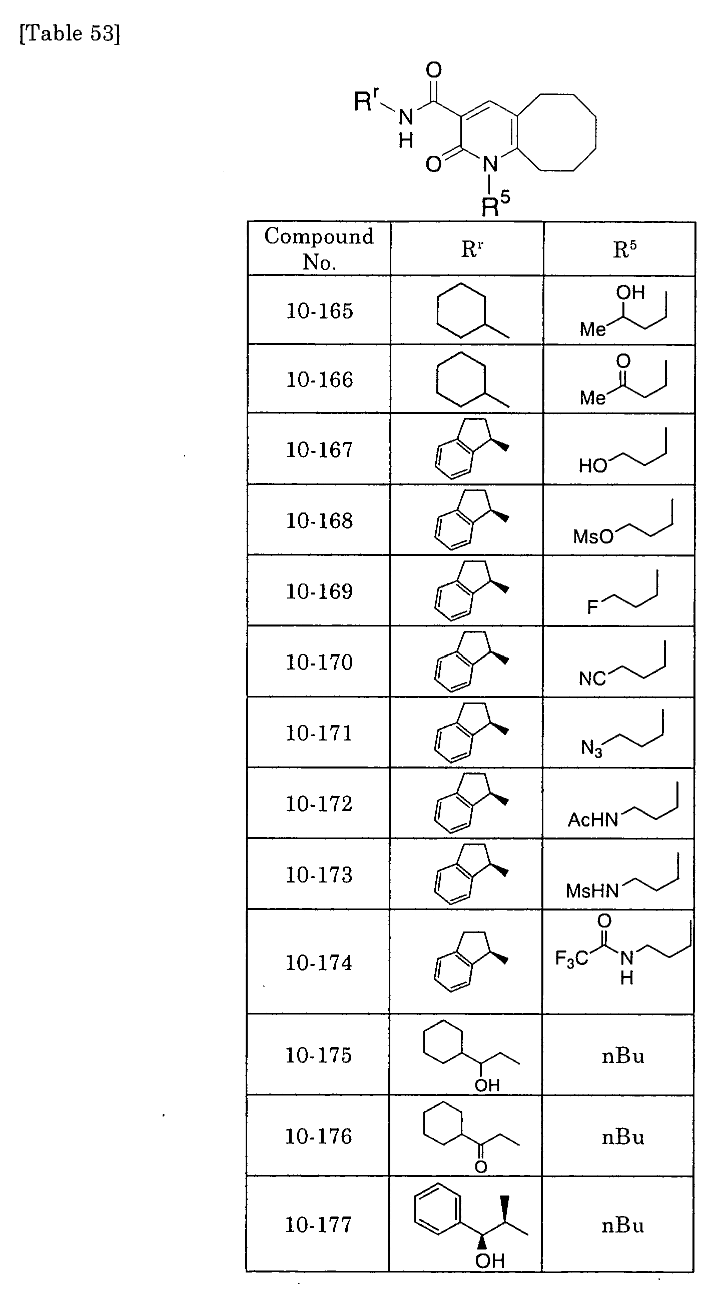

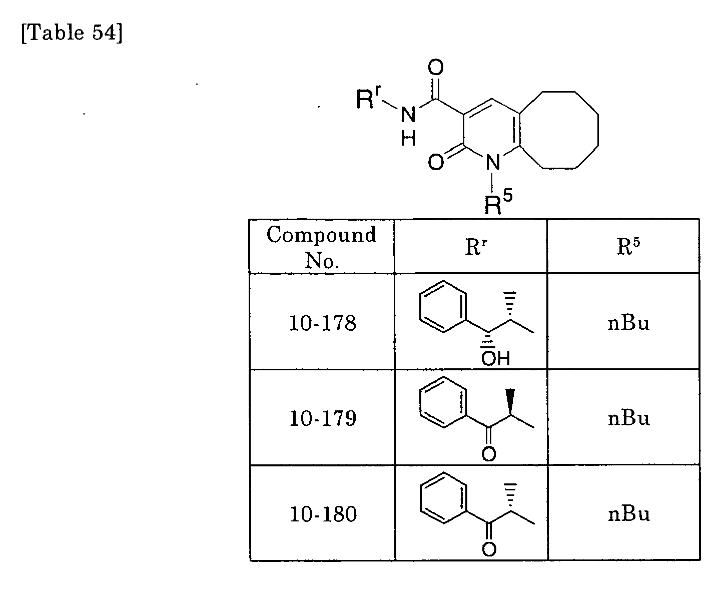

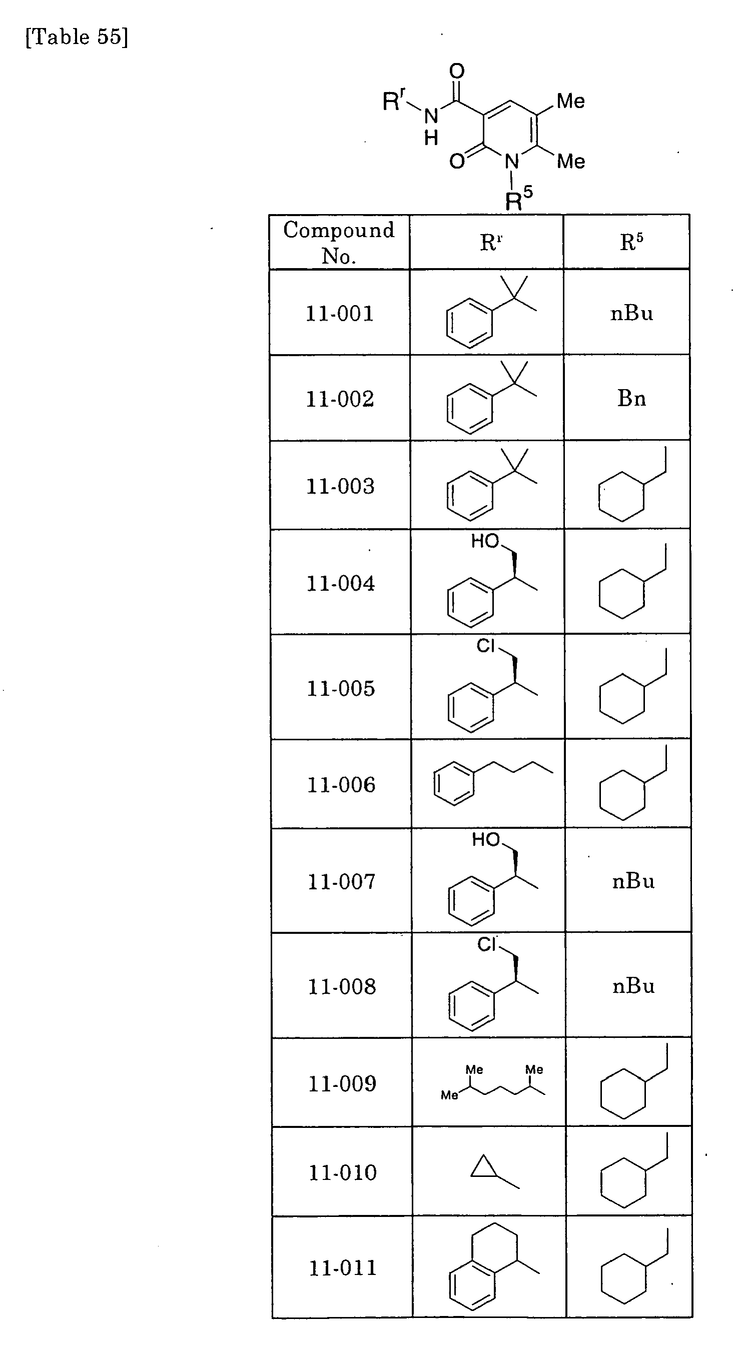

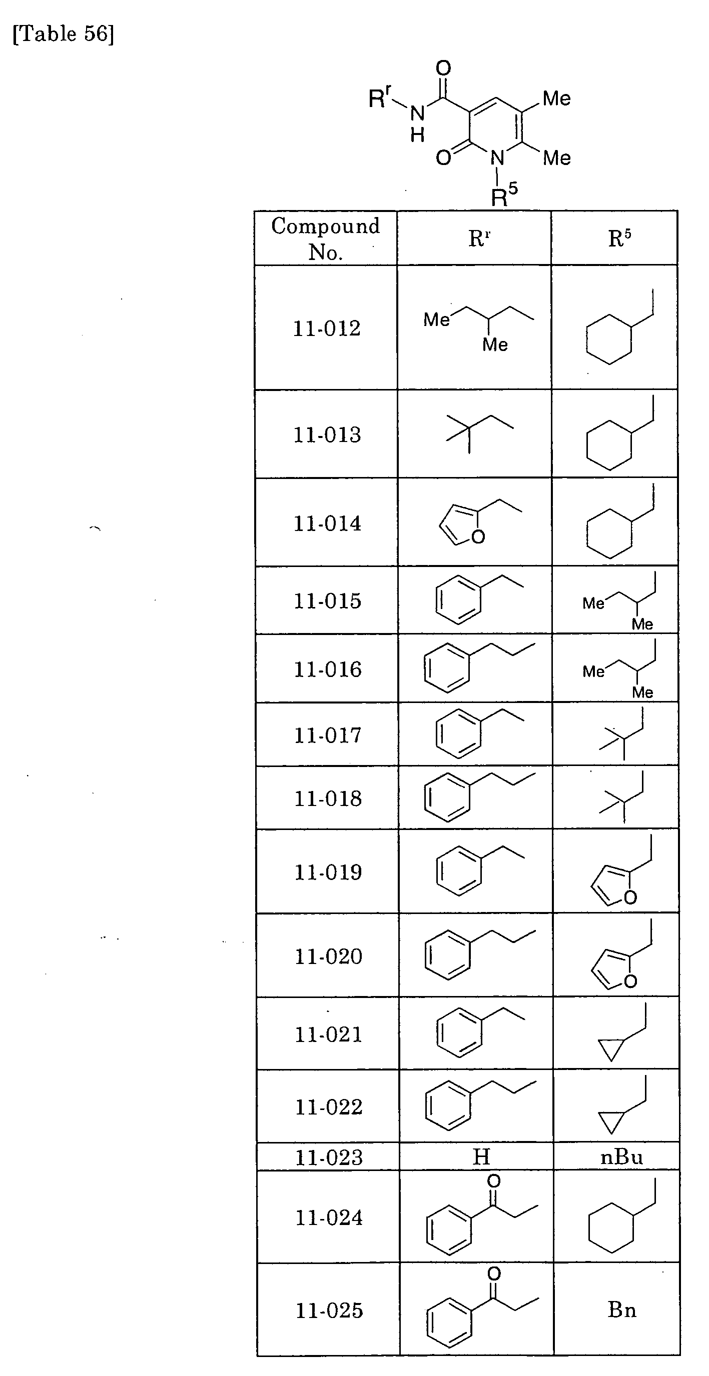

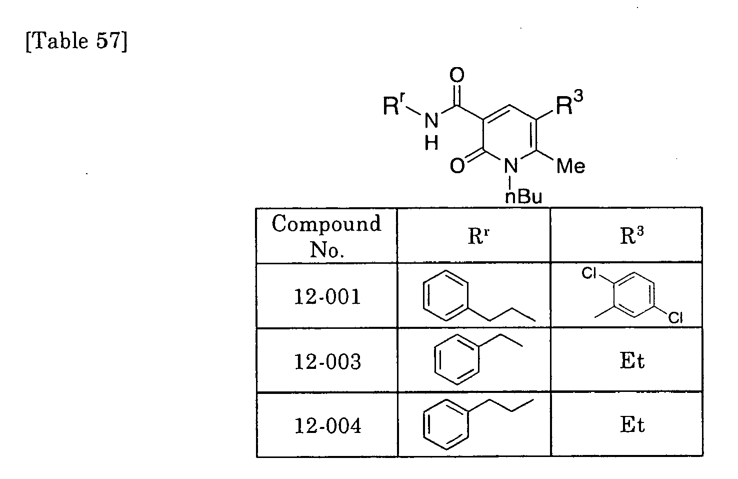

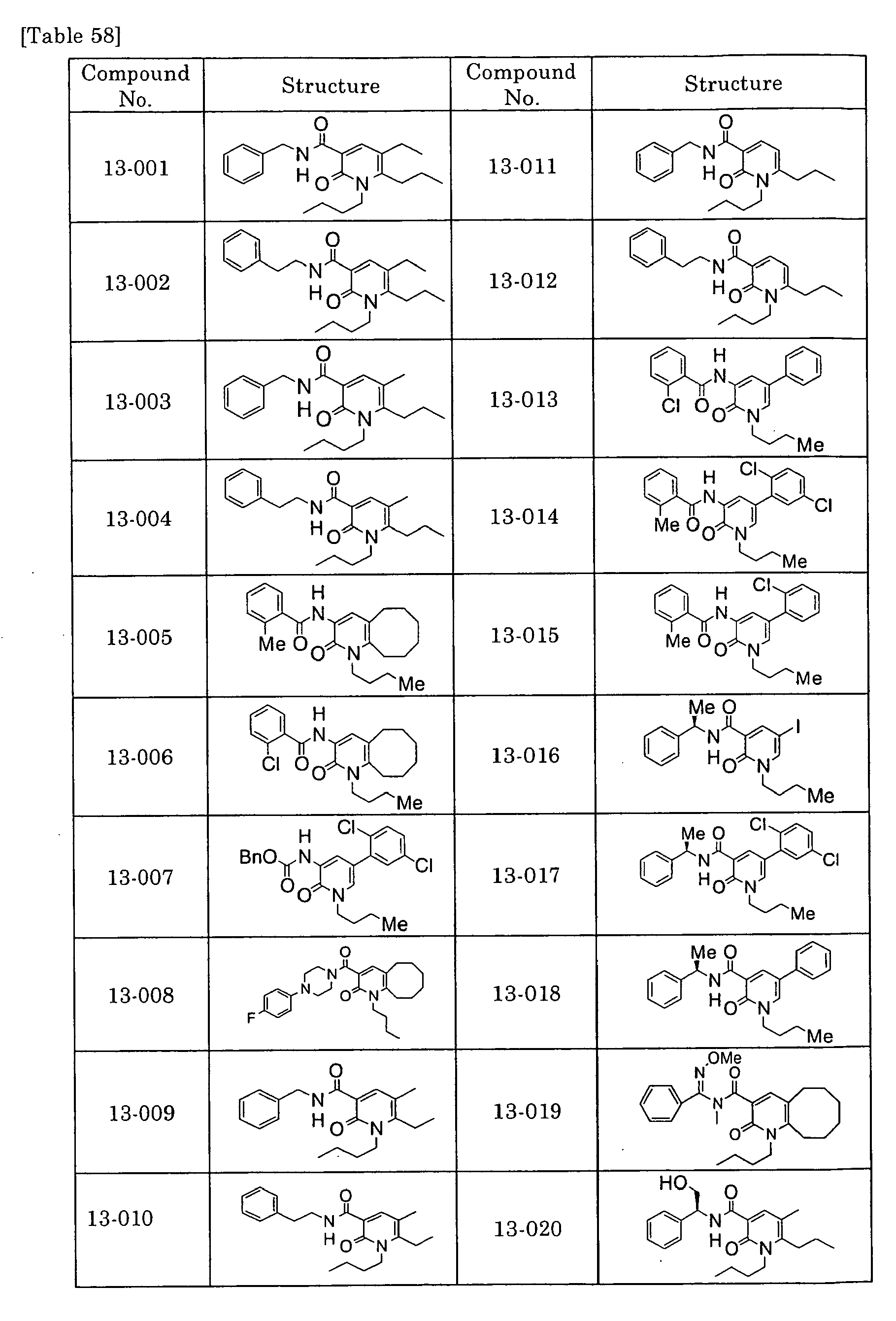

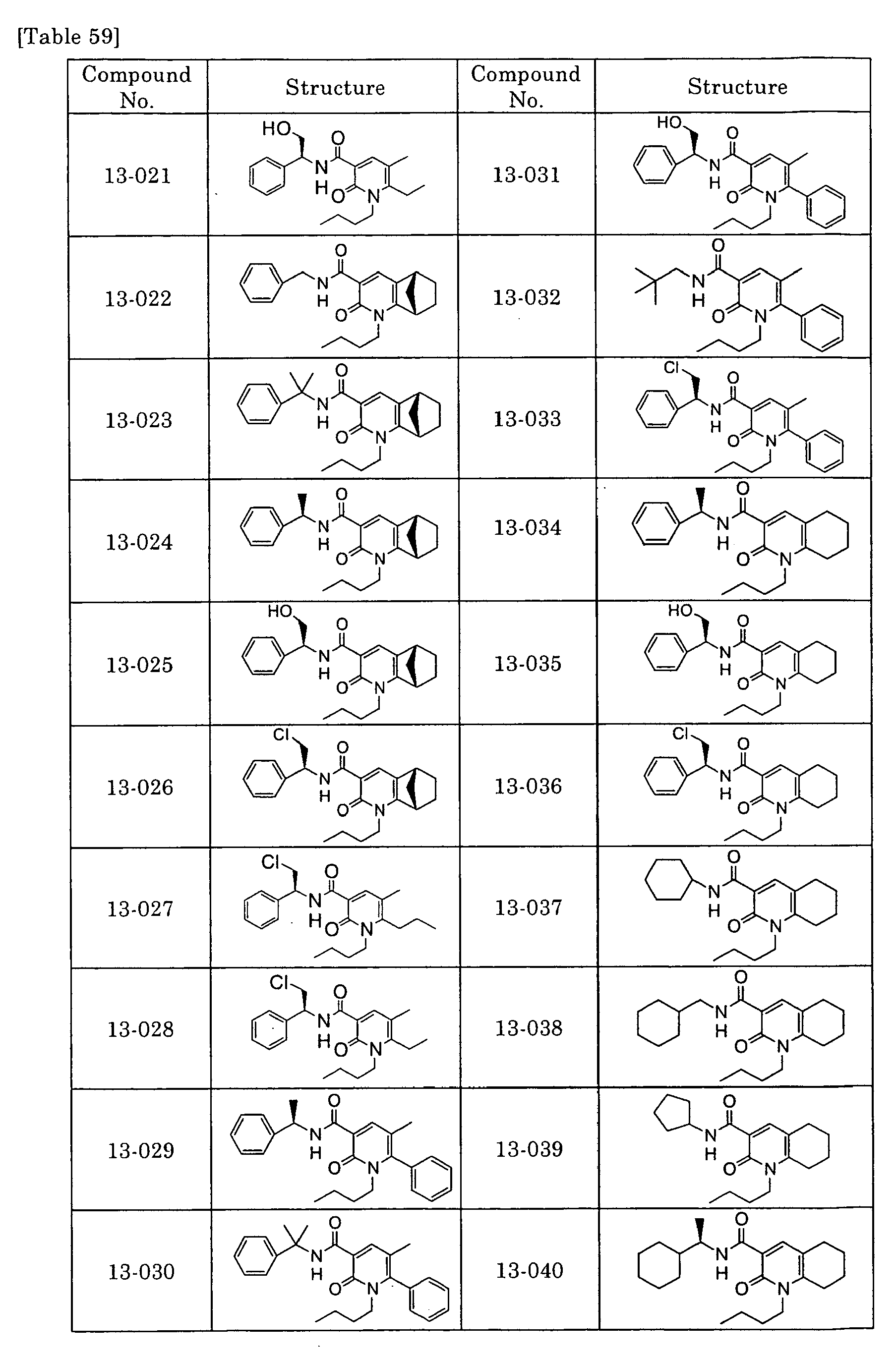

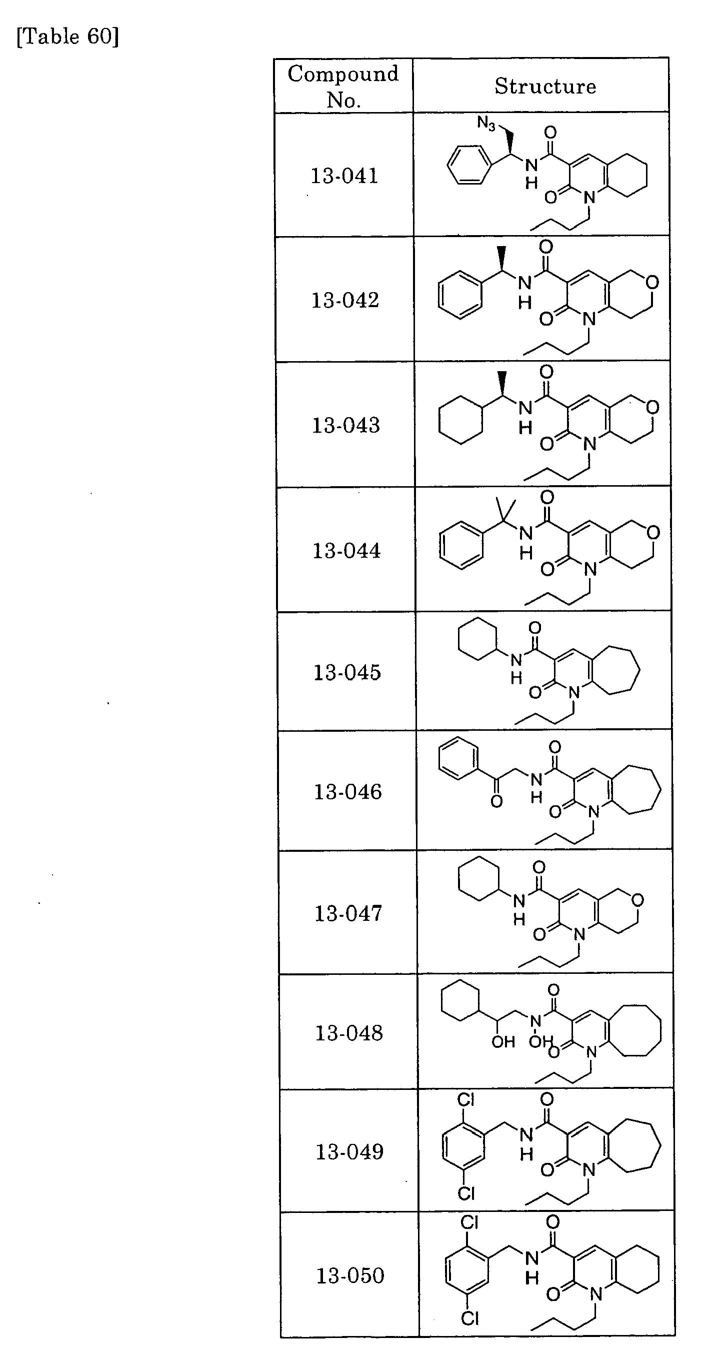

When using a compound of the present invention in treatment, it can be formulated into ordinary formulations for oral and parenteral administration. A pharmaceutical composition containing a compound of the present invention can be in the form for oral and parenteral administration. Specifically, it can be formulated into formulations for oral administration such as tablets, capsules, granules, powders, syrup, and the like; those for parenteral administration such as injectable solution or suspension for intravenous, intramuscular or subcutaneous injection, inhalant, eye drops, nasal drops, suppositories, or percutaneous formulations such as ointment.

When the compound uesed as an active ingredient has a week cannabinoid type 1 receptor agonistic effect and a strong cannabinoid type 2 receptor agonistic effect, all kind of formulations.thereof can be used. Especially, it can be used as oral administration such as tablets, capsules, granules, powders, syrup. When the compound uesed as an active ingredient has a strong cannabinoid type 1 receptor agonistic effect, preferable is a topical administration, especially, preferable are ointment, cream, lotion, and the like.

In preparing the formulations, carriers, excipients, solvents and bases known to one ordinary skilled in the art may be used. Tablets are prepared by compressing or formulating an active ingredient together with auxiliary components. Examples of usable auxiliary components include pharmaceutically acceptable excipients such as binders (e.g., cornstarch), fillers (e.g., lactose, microcrystalline cellulose), disintegrates (e.g., starch sodium glycolate) or lubricants (e.g., magnesium stearate). Tablets may be coated appropriately. In the case of liquid formulations such as syrups, solutions or suspensions, they may contain suspending agents (e.g., methyl cellulose), emulsifiers (e.g., lecithin), preservatives and the like. In the case of injectable formulations, it may be in the form of solution or suspension, or oily or aqueous emulsion, which may contain suspension-stabilizing agent or dispensing agent, and the like. In the case of an inhalant, it is formulated into a liquid formulation applicable to an inhaler. In the case of eye drops, it is formulated into a solution or a suspension.

Although an appropriate dosage of the present compound varies depending on the administration route, age, body weight, sex, or conditions of the patient, and the kind of drug(s) used together, if any, and should be determined by the physician in the end, in the case of oral administration, the daily dosage can generally be between about 0.01 - 100 mg, preferably about 0.01 - 10 mg, more preferably about 0.1 - 10 mg, per kg body weight. In the case of parenteral administration, the daily dosage can generally be between about 0.001 - 100 mg, preferably about 0.001 - 1 mg, more preferably about 0.01 - 1 mg, per kg body weight. The daily dosage can be administered in 1 - 4 divisions.

### Best Mode for Carrying Out the Invention

The compounds represented by the formula (I) can be synthesized by the preparation method described in WO 01/19807 or WO 02/072562. The compounds represented by the formula (II) can be synthesized by the preparation method described in WO 02/053543.

### Example

### Test example

### Experimental Examples 1, 2 and 3: Effect on antigen-induced bronchial hyperresponsiveness, inflammatory cell infiltration and mucus secretion in BN rats (Acute model)

Antigen-induced bronchial hyperresponsiveness in BN rats: Brown Norway (BN) rats (Charles River Japan) were actively sensitized by the intraperitoneal injection of 1 mL mixture containing aluminum hydroxide gel (1 mg) and ovalbumin (0.1 mg, OVA). Ten days later, antigen challenge was performed by the inhalation of an aerosolized 1% OVA solution for 30 min using an ultrasonic nebulizer. ACh was intravenously injected to rats 24 h after antigen challenge under sodium pentobarbital anesthesia (80 mg/kg, i.p.) by increasing doses of ACh every 5 min, then bronchoconstrictor response observed immediately after each ACh injection was measured by the method of Konzett & Rössler with some modifications. Briefly, trachea of rats was incised and a cannula was attached to lung side. An artificial respirator (SN-480-7, Shinano) was connected to the cannula, and then a fixed amount of air (tidal volume: 3 mL, ventilation frequency: 60 times/min) continuously insufflated to maintain respiration. The insufflation pressure overflowed from inhalation tube was monitored by a pressure transducer (TP-400T, Nihon Kohden) and recorded on a recorder (WT-645G, Nihon Kohden) through a carrier amplifier (AP-601G, Nihon Kohden). Test compounds were administered orally once 1 h before antigen challenge. The area under the curve (AUC) calculated from dose-response curve for ACh was compared between vehicle-treated control group and test compound-treated group, and then statistical significance was analyzed concerning inhibitory effect on bronchial hyperresponsiveness.

Compound I-270 exhibited a significant effect (P<0.01) at a dose of 100 mg/kg.

Compound 4-320 exhibited a significant effect (P<0.01) at a dose of 10 mg/kg.

Antigen-induced airway inflammatory cell infiltration in BN rats: After finishing experiment mentioned above, the lungs were washed 3 times with 5 mL of physiological saline through tracheal cannula using injection syringe. Then the cell number in the washing was determined. The preparations for differential cell count were prepared using Cytospin 3 (Shandon). Differential cell counts were performed after May-Grunwald-Giemsa staining, and then statistical significance was analyzed concerning inhibitory effect on airway inflammatory cell infiltration.

Compound 4-320 exhibited a significant effect (P<0.01) at doses of 1 and 10 mg/kg.

Compound 10-051 exhibited a significant effect (P<0.01) at doses of 30 and 100 mg/kg.

Antigen-induced mucus secretion in BN rats: After measurement of bronchial hyperresponsiveness, the lungs were washed 3 times with 5 mL of physiological saline through tracheal cannula using injection syringe, and then the washing was centrifuged. Mucin levels in the supernatants were measured by the method described below: 1) Microtiter plates (Immulon IV) were coated with 1000-fold diluted supernatants diluted with phosphate buffered saline for 2 h at 37°C, and then blocked with Block-Ace. 2) Plates were washed with phosphate buffered saline containing 0.05% Tween 20 (PBST), and then incubated with 150 µL of 5 µg/mL biontinylated jacalin for 1 h at 37°C. 3) Plates were washed with PBST, and then incubated with 150 µL of a 1/500 dilution of streptavidin-conjugated alkaline phosphatase for 30 min at room temperature. 4) After a final wash with PBST, 200 µL of pNPP liquid substrate system was added. 5) Several minutes later, the reaction was stopped by adding 100 µL of 3N NaOH, and then optical densities were measured at 405 nm). Statistical significance was analyzed concerning inhibitory effect on mucus secretion.

Compound 4-320 exhibited a significant effect (P<0.01) at a dose of 10 mg/kg.

### Experimental Examples 4, 5 and 6: Effect on antigen-induced bronchial hyperresponsiveness, inflammatory cell infiltration and mucus secretion in BN rats (Chronic model)

Antigen-induced bronchial hyperresponsiveness in BN rats: BN rats were actively sensitized by the intraperitoneal injection of a mixture containing aluminum hydroxide gel and ovalbumin. Twelve days later, antigen challenge was performed by the inhalation of an aerosolized 1% OVA solution or physiological saline for 30 min using an ultrasonic nebulizer (NE-U12, Omron). To establish chronic bronchial hyerreactivity model, this procedure was repeated 3 times with 1-week intervals. Test compounds were administered orally for 8 days from the day of third antigen challenge. On the day of third antigen challenge, test compounds were administered 1 h before challenge. One hour after last administration of test compounds, forth antigen challenge was performed. Inhibitory effect on bronchial hyperresponsiveness was evaluated 24 h after last antigen challenge by the method described in the section of Experimental Example 1.

Compound I-12 exhibited a significant effect at doses of 30 (P<0.01) and 100 mg/kg (P<0.05).

Compound 4-320 exhibited a significant effect (P<0.01) at a dose of 3 mg/kg.

Antigen-induced airway inflammatory cell infiltration in BN rats: After finishing experiment mentioned above, the lungs were washed 3 times with 5 mL of physiological saline through tracheal cannula using injection syringe. Then the cell number in the washing was determined. The preparations for differential cell count were prepared using Cytospin 3 (Shandon). Differential cell counts were performed after May-Grünwald-Giemsa staining, and then statistical significance was analyzed concerning inhibitory effect on airway inflammatory cell infiltration as in the section of Experimental Example 2.

Compound I-12 exhibited a significant effect (P<0.01) at a dose of 100 mg/kg.

Compound 10-051 exhibited a significant effect (P<0.05) at doses of 3 and 30 mg/kg.

Antigen-induced mucus secretion in BN rats: After measurement of bronchial hyperresponsiveness, the lungs were washed 3 times with 5 mL of physiological saline through tracheal cannula using injection syringe, and then the washing was centrifuged. Mucin levels in the supernatants were measured by the method described below: 1) Microtiter plates (Immulon IV) were coated with 1000-fold diluted supernatants diluted with phosphate buffered saline for 2 h at 37°C, and then blocked with Block-Ace. 2) Plates were washed with phosphate buffered saline containing 0.05% Tween 20 (PBST), and then incubated with 150 µL of 5 µg/mL biontinylated jacalin for 1 h at 37°C. 3) Plates were washed with PBST, and then incubated with 150 µL of a 1/500 dilution of streptavidin-conjugated alkaline phosphatase for 30 min at room temperature. 4) After a final wash with PBST, 200 µL of pNPP liquid substrate system was added. 5) Several minutes later, the reaction was stopped by adding 100 µL of 3N NaOH, and then optical densities were measured at 405 nm). Statistical significance was analyzed concerning inhibitory effect on mucus secretion.

### Experimental Examples 7, 8 and 9: Effect on antigen-induced bronchial hyperresponsiveness, inflammatory cell infiltration and mucus secretion in guinea pigs (Acute model)

Antigen-induced bronchial hyperresponsiveness in guinea pigs: Guinea pigs (Charles River Japan) held in an exposure chamber were actively sensitized by the inhalation of an aerosolized 1% OVA solution for 10 min using an ultrasonic nebulizer (NE-U12, Omron) twice with an interval of 1 week. One week later, antigen challenge was performed by inhalation of an aerosolized 1% OVA generated by an ultrasonic nebulizer for 5 min. Test compounds were administered orally 1 h before antigen challenge. In addition, guinea pigs were treated with diphenhydramine (10 mg/kg, i.p.), an antihistamine, to protect the animals from anaphylactic death 10 min before antigen challenge. ACh was intravenously injected to guinea pigs 24 h after antigen challenge under urethane anesthesia (1.4 g/kg, i.p.) by increasing doses of ACh every 5 min, then bronchoconstrictor response observed immediately after each ACh injection was measured by the method of Konzett & Rössler with some modifications. Briefly, trachea of guinea pigs was incised and a cannula was attached to the lung side. An artificial respirator (SN-480-7, Shinano) was connected to the cannula, and then a fixed amount of air (tidal volume: 4 mL, ventilation frequency: 60 times/min) continuously insufflated to maintain respiration. The insufflation pressure overflowed from inhalation tube was monitored by a pressure transducer (TP-400T, Nihon Kohden) and recorded on a recorder (WT-645G, Nihon Kohden) through a carrier amplifier (AP-601G, Nihon Kohden). The area under the curve (AUC) calculated from dose-response curve for ACh was compared between vehicle-treated control group and test compound-treated group, and then statistical significance was analyzed concerning inhibitory effect on bronchial hyperresponsiveness.

Compound I-12 exhibited a significant effect (P<0.05) at a dose of 10 mg/kg.

Compound 4-320 exhibited a significant effect at doses of 1 (P<0.01) and 10 mg/kg (P<0.05).

Antigen-induced airway inflammatory cell infiltration in guinea pigs: After finishing experiment mentioned above, the lungs are washed 3 times with 10 mL of physiological saline through tracheal cannula using injection syringe. Then the cell number in the washing was determined. The preparations for differential cell count were prepared using Cytospin 3 (Shandon). Differential cell counts were performed after May-Grunwald-Giemsa staining, and then statistical significance was analyzed concerning inhibitory effect on airway inflammatory cell infiltration.

Compound I-12 exhibited a significant effect (P<0.05) at a dose of 10 mg/kg.

Compound I-270 exhibited a significant effect (P<0.05) at a dose of 10 mg/kg.

Compound 4-320 exhibited a significant effect at doses of 1 (P<0.05) and 10 mg/kg (P<0.01).

Compound 10-051 exhibited a significant effect (P<0.05) at a dose of 30 mg/kg.

Antigen-induced mucus secretion in guinea pigs: After measurement of bronchial hyperresponsiveness, the lungs are washed 3 times with 10 mL of physiological saline through tracheal cannula using injection syringe, and then the washing was centrifuged. Mucin levels in the supernatants were measured by the method described below: 1) Microtiter plates (Immulon IV) were coated with 1000-fold diluted supernatants diluted with phosphate buffered saline for 2 h at 37°C, and then blocked with Block-Ace. 2) Plates were washed with phosphate buffered saline containing 0.05% Tween 20 (PBST), and then incubated with 150 µL of 5 µg/mL biontinylated jacalin for 1 h at 37°C. 3) Plates were washed with PBST, and then incubated with 150 µL of a 1/500 dilution of streptavidin-conjugated alkaline phosphatase for 30 min at room temperature. 4) After a final wash with PBST, 200 µL of pNPP liquid substrate system was added. 5) Several minutes later, the reaction was stopped by adding 100 µL of 3N NaOH, and then optical densities were measured at 405 nm). Statistical significance was analyzed concerning inhibitory effect on mucus secretion.

### Experimental Examples 10, 11 and 12: Effect on antigen-induced bronchial hyperresponsiveness, inflammatory cell infiltration and mucus secretion in guinea pigs (Chronic model)

Antigen-induced bronchial hyperresponsiveness in guinea pigs: Guinea pigs held in an exposure chamber were actively sensitized by the inhalation of an aerosolized 1% OVA solution for 10 min using an ultrasonic nebulizer (NE-U12, Omron) twice with an interval of 1 week. One week and 2 weeks later, antigen challenge was performed twice by inhalation of an aerosolized 1% OVA generated by an ultrasonic nebulizer for 5 min. Test compounds were administered orally for 8 days from the day of first antigen challenge. On the day of each antigen challenge, test compounds were administered 1 h before challenge. Guinea pigs were also treated with diphenhydramine (10 mg/kg, i.p.), an antihistamine, to protect the animals from anaphylactic death 10 min before each antigen challenge. Inhibitory effect on bronchial hyperresponsiveness was evaluated 24 h after last antigen challenge by the method described in the section of Experimental Example 7. The area under the curve (AUC) calculated from dose-response curve for ACh was compared between vehicle-treated control group and test compound-treated group, and then statistical significance was analyzed concerning inhibitory effect on bronchial hyperresponsiveness.

Compound 1-12 exhibited a significant effect (P<0.05) at a dose of 30 mg/kg.

Antigen-induced airway inflammatory cell infiltration in guinea pigs: After finishing experiment mentioned above, the lungs are washed 3 times with 10 mL of physiological saline through tracheal cannula using injection syringe. Then the cell number in the washing was determined. The preparations for differential cell count were prepared using Cytospin 3 (Shandon). Differential cell counts were performed after May-Grunwald-Giemsa staining, and then statistical significance was analyzed concerning inhibitory effect on airway inflammatory cell infiltration.

Compound I-12 exhibited a significant effect (P<0.01) at a dose of 30 mg/kg.

Antigen-induced mucus secretion in guinea pigs: After measurement of bronchial hyperresponsiveness, the lungs are washed 3 times with 10 mL of physiological saline through tracheal cannula using injection syringe, and then the washing was centrifuged. Mucin levels in the supernatants were measured by the method described below: 1) Microtiter plates (Immulon IV) were coated with 1000-fold diluted supernatants diluted with phosphate buffered saline for 2 h at 37°C, and then blocked with Block-Ace. 2) Plates were washed with phosphate buffered saline containing 0.05% Tween 20 (PBST), and then incubated with 150 µL of 5 µg/mL biontinylated jacalin for 1 h at 37°C. 3) Plates were washed with PBST, and then incubated with 150 µL of a 1/500 dilution of streptavidin-conjugated alkaline phosphatase for 30 min at room temperature. 4) After a final wash with PBST, 200 µL of pNPP liquid substrate system was added. 5) Several minutes later, the reaction was stopped by adding 100 µL of 3N NaOH, and then optical densities were measured at 405 nm). Statistical significance was analyzed concerning inhibitory effect on mucus secretion.

Compound I-12 exhibited a significant effect (P<0.01) at a dose of 30 mg/kg.

### Experimental Exanmple 13: Bronchodilating effect in guinea pigs

Under urethane anesthesia (1.4 g/kg, i.p.), ACh was intravenously injected to guinea pigs by increasing doses of ACh every 5 min, then bronchoconstrictor response observed immediately after each ACh injection was measured by the method of Konzett & Rössler with some modifications. Briefly, trachea of guinea pigs was incised and a cannula was attached to the lung side. An artificial respirator (SN-480-7, Shinano) was connected to the cannula, and then a fixed amount of air (tidal volume: 4 mL, ventilation frequency: 60 times/min) continuously insufflated to maintain respiration. The insufflation pressure overflowed from inhalation tube was monitored by a pressure transducer (TP-400T, Nihon Kohden) and recorded on a recorder (WT-645G, Nihon Kohden) through a carrier amplifier (AP-601G, Nihon Kohden). Test compounds were administered orally 1 h before ACh injection, then the effect on the dose-response curve of ACh was examined. Statistical significance was analyzed concerning broncohdilating effect in guinea pigs.

Compound 4-320 exhibited a significant effect (P<0.01) at a dose of 10 mg/kg.

### Formulation example

The following formulation examples 1 to 8 are provided to further illustrate formulation example and are not to be construed as limiting the scope of the present invention. The term "an active ingredient" means a compound of the present invention, a tautomer, a prodrug, a pharmaceutical acceptable salt, or a solvate thereof.

### Formulation example 1

Hard gelatin capsule are prepared using the following ingredients.

| | | Dosage (mg/capsule) |
|---|---|---|
| Ingredients | An actve ingredient | 250 |
| | Starch (dry) | 200 |
| | Magnesium stearate | 10 |
| | Total | 460 mg |

### Formulation 2

Tablets are prepared using the following ingredients.

| | | Dosage (mg/tablet) |
|---|---|---|
| Ingredients | An actve ingredient | 250 |
| | Cellulose (microcrystalline) | 400 |
| | Silicon dioxide (fume) | 10 |
| | Stearic acid | 5 |
| | Total | 665 mg |

These ingredients are mixed and condensed to prepare tablets of 665 mg.

### Formulation 3

Aerosol solutions are prepared using the following ingredients.

| | | Weight |
|---|---|---|
| Ingredients | An actve ingredient | 0.25 |
| | Ethanol | 25.75 |
| | Properanto 22 (chlorodifluorometahne) | 74.00 |
| | Total | 100.00 |

An active ingredient and ethanol are mixed, and the mixture is added into a part of properanto 22, cooled at -30 °C, transferred to packing equipment. The amount needed is provided to stainless steel vessel, diluted with residual properanto 22. The bubble unit is insalled to vessel.

### Formulation 4

Tablets containig an active ingredient 60 mg are prepared as folows.

| | | |
|---|---|---|
| Ingredients | An active ingredient | 60 mg |
| | Starch | 45 mg |
| | Microcrystal cellulose | 35 mg |
| | Polyvinylpyrrolidone (10% aqueous solution) | 4 mg |
| | Carboxymethyl starch sodium salt | 4.5 mg |
| | Magnesium stearate | 0.5 mg |
| | Talc | 1 mg |
| | | 150 mg |

An active ingredient, Starch, and cellulose are made pass through a No.45 mesh U.S. sieve and then mixed sufficiently. The resulting mixture is mixed with a polyvinylpyrrolidone aqueous solution, made pass through a No.14 mesh U.S. sieve. The obtained granule is dried at 50 °C, made pass through a No. 18 mesh U.S. sieve. To the granule are added carboxymethyl starch-Na, Magnesium stearate, and talc made pass through a No.60 mesh U.S. sieve, and the mixture was mixed. The mixed powder is compressed by tableting equipment to yield tablets of 150 mg.

### Formulation 5

Capsuls containig an active ingredient 80 mg are prepared as folows.

| | | |
|---|---|---|
| Ingredients | An active ingredient | 80 mg |
| | Starch | 59 mg |
| | Microcrystal cellulose | 59 mg |
| | Magnesium stearate | 2 mg |
| | Total | 200 mg |

An active ingredient, Starch, cellulose, and magnesium stearate are mixed, made pass through a No.45 mesh U.S. sieve, and then packed to hard gelatin capsuls at amount of 200 mg/capsul.

### Formulation 6

Suppository containig an active ingredient 225 mg are prepared as folows.

| | | |
|---|---|---|
| Ingredients | An active ingredient | 225 mg |
| | Saturated fattyacid glyceride | 2000 mg |
| | Total | 2225 mg |

An active ingredient is made pass through a No.60 mesh U.S. sieve, suspended in saturated fattyacid glyceride dissolved by heating at a minimum of necessity. The mixture is cooled in the mould of 2mg.

### Formulation 7

Suspension containig an active ingredient 50 mg are prepared as folows.

| | | |
|---|---|---|
| Ingredients | An active ingredient | 50 mg |
| | Carboxymethylcellulose sodium salt | 50 mg |
| | Syrupus | 1.25 mL |
| | Benzoic acid solution | 0.10 mL |
| | Aroma chemical | q.v. |
| | Pigmentum | q.v. |
| | Water | |
| | Total | 5 mL |

An active ingredient is made pass through a No.60 mesh U.S. sieve, mixed with carboxymethylcellulose sodium salt and to prepare smoothly paste. To the mixture are benzoic acid solution and syrupus which are diluted with a part of water, and the mixture is stirred. To the mixture is residual water to prepare necessary volume.

### Formulation 8

Intravenous formulations are prepared as follows.

| | | |
|---|---|---|
| Ingredients | An active ingredient | 100 mg |
| | Saturated fattyacid glyceride | 1000 ml |

Usually a solution of ingredients above described is administered intravenously to a patient by the speed of 1 ml/min.

### Industrial Applicability

It was found that thiazine derivatives and pyridone derivatives having cannabinoid receptor agonistic acitivity exibit the effect as an inhibitor for inflammatory cell infiltration in the respiratory tract, an inhibitor for hyperirritability in the respiratory tract, a muciparous inhibitor, or a bronchodilator.

## Claims

1. An inhibitor for inflammatory cell infiltration in the respiratory tract, an inhibitor for hyperirritability in the respiratory tract, a muciparous inhibitor, or a bronchodilator which contains as an active ingredient a compound represented by the formula (I): 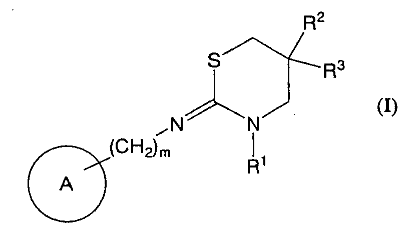 wherein R¹ is the group represented by the formula: -C(=Z)-W-R⁴ wherein Z is an oxygen atom or a sulfur atom; W is an oxygen atom or a sulfur atom; R⁴ is optionally substituted alkyl, optionally substituted alkenyl, or optionally substituted alkynyl;
R² and R³ are independently optionally substituted alkyl or optionally substituted cycloalkyl; or
R² and R³ are taken together to form optionally substituted alkylene which may contain a heteroatom(s);
m is an integer of 0 to 2;
A is optionally substituted aryl or optionally substituted heteroaryl.

2. An inhibitor for inflammatory cell infiltration in the respiratory tract, an inghibitor for hyperirritability in the respiratory tract, a muciparous inhibitor, or a bronchodilator according to claim 1 wherein R¹ is the group represented by the formula: -C(=Z)-W-R⁴ wherein Z is an oxygen atom or a sulfur atom; W is a sulfur atom; R⁴ is optionally substituted alkyl or alkenyl; R² and R³ are independently alkyl; or R² and R³ taken together may form optionally substituted alkylene; m is 0; A is aryl optionally substituted with one or two substitutent(s) selected from the group consisting of alkyl, haloalkyl, hydroxy, alkoxy, haloalkoxy, alkylthio, and haloalkylthio.

3. An inhibitor for inflammatory cell infiltration in the respiratory tract, an inhibitor for hyperirritability in the respiratory tract, a muciparous inhibitor, or a bronchodilator which contains as an active ingredient a compound represented by the formula (II): 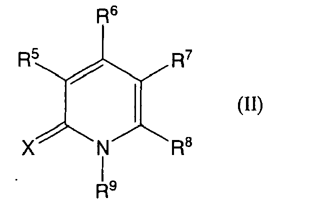 wherein R⁵ is the group represented by the formula: -Y¹-Y²-Y³-R^{a} wherein Y¹ and Y³ are each independently a bond or optionally substituted alkylene; Y² is a bond, -O-, -O-SO₂-, -NR^{b}-, -NR^{b}-C(=O)-, -NR^{b}-SO₂-, -NR^{b}-C(=O)-O-, -NR^{b}-C(=O)-NR^{b}-, -NR^{b}-C(=S)-NR^{b}-, -S-, -C(=O)-O-, or -C(=O)-NR^{b}-; R^{a} is optionally substituted alkyl, optionally substituted alkenyl, optionally substituted alkylnyl, an optionally substituted carbocyclic group, an optionally substituted heterocyclic group, or acyl; R^{b} is each independently a hydrogen atom, optionally substituted alkyl, or acyl;
R⁶ is a hydrogen atom, optionally substituted alkyl, optionally substituted alkenyl, optionally substituted alkylnyl, a halogen atom, or alkoxy;
R⁷ and R⁸ are each independently a hydrogen atom, optionally substituted alkyl, optionally substituted alkenyl, optionally substituted alkylnyl, a halogen atom, optionally substituted phenyl, or optionally substituted carbamoyl; or
R⁷ and R⁸ are taken together with the adjacent carbon atoms to form a 5 to 8 membered ring which may contain a heteroatom(s) and /or an unsaturated bond(s);
R⁹ is a hydrogen atom, optionally substituted alkyl which may contain a heteroatom(s) and /or an unsaturated bond(s), or the group represented by the formula -Y⁶-R^{e} wherein Y⁶ is a bond, optionally substituted alkylene, alkenylene, alkylnylene, -O-, -S-, -SO-, or -SO₂-; R^{e} is an optionally substituted carbocyclic group or an optionally substituted heterocyclic group;
X is an oxygen atom or a sulfur atom;

4. An inhibitor for inflammatory cell infiltration in the respiratory tract, an inhibitor for hyperirritability in the respiratory tract, a muciparous inhibitor, or a bronchodilator according to claim 3 wherein R⁵ is the group represented by the formula: -Y¹-Y²-Y³-R^{a} wherein Y¹ is a bond; Y² is -C(=O)-NH-; Y³ is a bond or optionally substituted alkylene; R^{a} is an optionally substituted carbocyclic group; R⁶ is a hydrogen atom; R⁷ is alkyl, a halogen atom, or optionally substituted phenyl; R⁸ is a hydrogen atom or alkyl; or R⁷ and R⁸ are taken together with the adjacent carbon atoms to form a 8 membered ring which may contain an unsaturated bond(s); R⁹ is optionally substituted C3 or more alkyl which may contain a heteroatom(s) and /or an unsaturated bond(s), or the group represented by the formula -Y⁶-R^{e} wherein Y⁶ is a bond or optionally substituted alkylene; R^{e} is an optionally substituted carbocyclic group.

5. Use of a compounds represented by the formula (I) in claim 1 or (II) in claim 3 for preparation of a pharmaceutical composition for preventing and/or treating an inflammatory cell infiltration in the respiratory tract, a hyperirritability in the respiratory tract, a muciparous, or a bronchoconstrictive action.

6. A method for preventing and/or treating a mammal, including a human, to alleviate the pathological effects of an inflammatory cell infiltration in the respiratory tract, a hyperirritability in the respiratory tract, a muciparous, or a bronchoconstrictive action wherein the method comprises administration to said mammal of a compound represented by the formula (I) in claim 1 or (II) in claim 3, in a pharmaceutically effective amount.
